(19) Europäisches Patentamt
European Patent Office
Office européen des brevets

(11) EP 4 206 196 A1

(12) EUROPEAN PATENT APPLICATION

(43) Date of publication:
05.07.2023 Bulletin 2023/27

(21) Application number: 21383231.4

(22) Date of filing: 29.12.2021

(51) International Patent Classification (IPC):
*C07D 403/14* (2006.01) *C07D 239/48* (2006.01)
*C07D 401/12* (2006.01) *C07D 401/14* (2006.01)
*A61P 17/06* (2006.01) *C07D 403/12* (2006.01)
*C07D 405/14* (2006.01) *C07D 413/14* (2006.01)
*C07F 9/53* (2006.01) *A61K 31/506* (2006.01)

(52) Cooperative Patent Classification (CPC):
**C07D 403/12; A61P 17/06; C07D 239/48; C07D 401/12; C07D 401/14; C07D 403/14; C07D 405/14; C07D 413/14**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Almirall S.A.**
**08022 Barcelona (ES)**

(72) Inventors:
- **PUIG DURAN, Carlos**
  **BARCELONA (ES)**
- **GOMEZ CASTILLO, Elena**
  **BARCELONA (ES)**
- **CATURLA JAVALOYES, Juan Francisco**
  **BARCELONA (ES)**
- **PAGES SANTACANA, Lluis Miquel**
  **BARCELONA (ES)**
- **EASTWOOD, Paul Robert**
  **BARCELONA (ES)**
- **ERRA SOLA, Montserrat**
  **BARCELONA (ES)**

(74) Representative: **Herrero & Asociados, S.L.**
**Cedaceros, 1**
**28014 Madrid (ES)**

(54) **PYRIMIDINE SUBSTITUTED DERIVATIVES AS TYK2 INHIBITORS**

(57) Novel pyrimidine substituted compounds of Formula (I) are disclosed; as well as processes for their preparation, pharmaceutical compositions comprising them and their use in therapy as inhibitors of Tyrosine Kinase 2 (Tyk2).

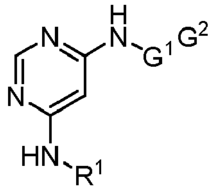

Formula (I)



Processed by Luminess, 75001 PARIS (FR)

## Description

### FIELD OF THE INVENTION

The present invention deals with compounds and methods able to inhibit the TYK2 receptor by binding to its pseudokinase domain and with pharmaceutical formulations of such compounds useful in the treatment of related disorders.

### BACKGROUND OF THE INVENTION

Protein kinases constitute a very relevant and large family of enzymes responsible for several transduction processes within the cells. The main function of such enzymes is to transfer phosphate groups from ATP to specific substrates of very broad nature: carbohydrates, proteins or lipids. Protein kinases direct phosphorylation processes towards threonine, serine, tyrosine and other amino acid residues present in the structure of the proteins. Such phosphorylation results in a functional change of the target protein giving rise to a modification of enzymatic activity, cellular location or modification of its signalling pathway. A huge subfamily of the protein kinases includes tyrosine kinases, those kinases able to phosphorylate tyrosine residues of different proteins. The receptor-associated tyrosine kinases include a tyrosine-kinase domain recruited to a receptor and are involved in a number of signalling cascades (cytokine signalling, growth hormone and others). The Janus kinase (JAK) family belongs to the later family and catalyses the phosphorylation of STAT proteins through a complex structure known as the JAK-STAT signalling pathway. This structure includes the cytokine receptor, the associated JAK proteins and the STAT protein able, once activated by phosphorylation/dimerization, to translocate into the cell nucleus and to induce transcription of target genes. The JAK family members are JAK1, JAK2, JAK3 and TYK2, which are associated to different cytokine receptors and catalyse the phosphorylation of the different STAT family members. Seven different STAT members (STAT1, STAT2, STAT3, STAT4, STAT5A, STAT5B and STAT6) have been described (Shuai, 2006). The binding of cytokines (such as interleukins or interferons) to its corresponding receptors causes the receptors dimerization and further phosphorylation through the JAK proteins. STAT then binds to the phosphorylated receptors and are in turn phosphorylated by JAKs.

Different interactions of JAK isoforms are used by many cytokine-receptor complexes. TYK2 pairs with JAK2 and interact with IL-12 and IL-23 cytokine receptors that are associated with Th1 and Th17 differentiation, respectively (Minegishi, 2006) and with JAK1 for type I interferons. Considering the relevant but narrow spectrum of cytokines that signal through TYK2, inhibitors for this kinase may exert selective inhibitory effects on the mentioned cytokines signaling without broad-based immunosuppression. Interestingly, a single nucleotide polymorphism (P1104A; substitution of a highly conserved proline with an alanine at position 1104 in the kinase domain of the TYK2 protein) has been identified which is protective across different autoimmune diseases including among others psoriasis, rheumatoid arthritis, systemic lupus erythematosus, Crohn's disease and ulcerative colitis. Homozygous individuals show impaired responses to type I IFN, IL-12 and IL-23 and was not associated with increased viral or mycobacterium infections (Dendrou, 2016).

The structure of the JAK family includes several homology regions being the most significant the JH1 or kinase domain, which accommodates the ATP substrate and catalyses the phosphate transferences. There is also a JH2 or pseudokinase domain, structurally similar to the JH1 domain and essential for normal kinase activity, but lacking enzymatic activity. The pseudokinase domain is involved in the regulation of the kinase or catalytic domain and presents lower sequence homology among the different JAK proteins.

The nuclear role of the IL12/IL17 immune axis on the pathogenesis of psoriasis has been well established in recent years with the introduction of biological therapies against different cytokines like TNFa, IL17 or IL23. TYK2 has a key role on the activation of this axis by transduction of the signals of IL12, IL23 and IFNa. The inhibition of TYK2 shows thus a great potential in the management of psoriasis and other autoimmune diseases like Crohn's disease, Rheumatoid Arthritis or Lupus Erythematosus.

Several JAK inhibitors have been introduced as therapeutic agents but most of them lack isoform selectivity and, in addition to TYK2, inhibit other family kinases as JAK1. Such promiscuity could give raise to unwanted side effects that could be potentially avoided by use of TYK2 selective inhibitors.

Given the much lower sequence homology of the pseudokinase domains compared to the catalytic ones, the use of TYK2 JH2 binders shows advantages in terms of selectivity (both isoform and general kinome selectivities).

### SUMMARY OF THE INVENTION

Thus, the present invention is directed to new compounds that possess Tyk2 inhibitory activity. Accordingly, there is provided a pyrimidine substituted derivative, which pyrimidine derivative is a compound of Formula (I), or a pharmaceutically acceptable salt, or a solvate, or a *N*-oxide, or a tautomer, or a stereoisomer, or an isotopically-labelled derivative thereof:

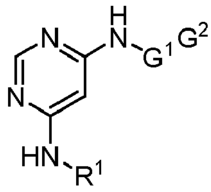

Formula (I)

wherein:

- $G^1$ represents a phenyl group or a 5- to 6-membered heteroaryl group containing one atom of N, wherein the phenyl and heteroaryl groups are unsubstituted or substituted by one or more substituents selected from a linear or branched $C_{1-4}$ alkyl group, a linear or branched $C_{1-3}$ alkenyl, a linear or branched $C_{1-4}$ alkoxy group, a halogen atom, a linear or branched $C_{1-4}$ haloalkyl group, a linear or branched $C_{1-2}$ haloalkoxy group, a CN group, a $-P(O)(CH_3)_2$ group, a linear or branched $C_{1-3}$ hydroxyalkyl group, a linear or branched -($C_{1-4}$ alkyl)-($C_{1-4}$ alkoxy) group, a $-NR^aSO_2R^b$ group, a -(linear or branched $C_{1-3}$ alkyl group)$_{0-1}$-$SO_nCH_3$ group, a $-SO_2NR^cR^d$ group, a $-S(O)Me{=}NR^e$ group and a $-C(O)-R^f$ group;

- $G^2$ is absent or represents a $-(CH_2)$-phenyl group, a $C_{3-6}$ cycloalkyl group, a 5- to 6-membered heteroaryl group containing at least one heteroatom selected from N, O and S, and a 5- to 6-membered heterocyclyl group containing at least one heteroatom selected from N, O and S, wherein the cycloalkyl, heteroaryl and heterocyclyl groups are unsubstituted or substituted by one or more substituents selected from a linear or branched $C_{1-3}$ alkyl group, a linear or branched $C_{1-3}$ haloalkyl, a linear or branched -($C_{1-4}$ alkyl)-($C_{1-4}$ alkoxy) group, a halogen atom, a $-(CH_2)_{0-1}$-4- to 6- membered heterocyclyl group containing at least one heteroatom selected from N, O and S, a $-C(O)-R^g$, and a $-(CH_2)_{0-1}-C_{3-6}$, cycloalkyl group, wherein the cycloalkyl group is unsubstituted or substituted by one or more substituents selected from halogen atom and a hydroxyl group;

- $R^1$ represents a $-C(O)-R^2$ group or a $G^3$ group;

- $R^2$ represents a monocyclic $C_{3-6}$ cycloalkyl group, wherein the $C_{3-6}$ cycloalkyl group is unsubstituted or substituted by one or more halogen atom;

- $G^3$ represents a 5- to 6- membered N-heteroaryl group, wherein the 5- to 6- membered N-heteroaryl group is substituted by one or more substituents selected from a linear or branched $C_{1-4}$ alkyl group, a fluorine atom, a CN group and a 6-membered heterocyclyl group containing at least one atom of O;

- $R^a$ represents a hydrogen atom, a linear or branched $C_{1-4}$ alkyl group or a linear or branched $C_{1-4}$ deuteroalkyl group;

- $R^b$ represents a group consisting of a linear or branched $C_{1-4}$ alkyl group, a linear or branched $C_{1-4}$ haloalkyl group or a $C_{3-6}$ cycloalkyl group;

- $R^c$ and $R^d$ together with the N atom to which they are attached form a 6-membered heterocyclyl group, which optionally contains in addition to the N-atom one additional heteroatom selected from N, O and S;

- $R^e$ represents a linear or branched $C_{1-3}$ alkyl group;

- $R^f$ represents a linear or branched $C_{1-5}$ hydroxyalkyl group, a 5-membered heterocyclyl group containing at least one heteroatom selected from N, O and S, a $-NH_2$ group or a linear or branched $C_{1-3}$ alkylamino group;

- $R^g$ represents a linear or branched $C_{1-4}$ alkoxy group or a heterocyclyl group containing at least one heteroatom selected from N, O and S; and

- n represents 1 or 2.

We have unexpectedly found that the compounds disclosed in the present application are TYK2 selective inhibitors through its binding to the TYK2 pseudokinase domain. This selectivity is present not only across the JAK

isoform family but also across an extended kinase panel. The reason for this selectivity can be related to the interactions of a water unit retained by the molecules with the gatekeeper region of the TYK2 JH2 as shown in the crystal structure of the complex of compound from example 12 and the TYK2 JH2 domain.

The invention further provides synthetic processes and intermediates described herein, which are useful for preparing said pyrimidine derivatives.

The invention is also directed to a pyrimidine derivative of the invention as described herein for use in the treatment of the human or animal body by therapy.

The invention also provides a pharmaceutical composition comprising the pyrimidine derivatives of the invention and a pharmaceutically-acceptable diluent or carrier.

The invention is also directed to the pyrimidine derivatives of the invention as described herein, for use in the treatment of a pathological condition or disease susceptible to amelioration by inhibiton of Tyrosine kinase 2 (Tyk2), in particular wherein the pathological condition or disease is selected from a dermatological disease, an inflammatory or autoimmune-mediated disease and a metabolism/endocrine function disorder. More in particular, wherein the pathological condition or disease is selected from plaque psoriasis, scalp psoriasis, genital psoriasis, pustular psoriasis, psoriatic erythroderma, acrodermatitis continua, pityriasis rubra pilaris, lichen planus, hidradenitis suppurativa, bullous pemphigoid, pyoderma gangrenousm (PASH), ichthyosis, atopic dermatitis, psoriatic arthritis, ankylosing spondylitis, systemic sclerosis, primary biliary cholangitis, asthma and human multiple myeloma.

The invention is also directed to use of the pyrimidine derivatives of the invention as described herein, in the manufacture of a medicament for treatment of a pathological condition or disease susceptible to amelioration by inhibiton of Tyrosine Kinase 2 (Tyk2), in particular wherein the pathological condition or disease is selected from a dermatological disease, an inflammatory or autoimmune-mediated disease and a metabolism/endocrine function disorder. More in particular, wherein the pathological condition or disease is selected from plaque psoriasis, scalp psoriasis, genital psoriasis, pustular psoriasis, psoriatic erythroderma, acrodermatitis continua, pityriasis rubra pilaris, lichen planus, hidradenitis suppurativa, bullous pemphigoid, pyoderma gangrenousm (PASH), ichthyosis, atopic dermatitis, psoriatic arthritis, ankylosing spondylitis, systemic sclerosis, primary biliary cholangitis, asthma and human multiple myeloma.

The invention also provides a method of treatment of a pathological condition or disease susceptible to amelioration by inhibiton of Tyrosine Kinase 2 (Tyk2), in particular wherein the pathological condition or disease is selected from a dermatological disease, an inflammatory or autoimmune-mediated disease and a metabolism/endocrine function disorder. More in particular wherein the pathological condition or disease is selected from plaque psoriasis, scalp psoriasis, genital psoriasis, pustular psoriasis, psoriatic erythroderma, acrodermatitis continua, pityriasis rubra pilaris, lichen planus, hidradenitis suppurativa, bullous pemphigoid, pyoderma gangrenousm (PASH), ichthyosis, atopic dermatitis, psoriatic arthritis, ankylosing spondylitis, systemic sclerosis, primary biliary cholangitis, asthma and human multiple myeloma. The invention also provides a combination product comprising (i) the pyrimidine derivatives of the invention as described herein; and (ii) one or more additional active substances.

The invention also provides a combination product comprising (i) the pyrimidine derivatives of the invention as described herein; and (ii) one or more additional active substances.

## DETAILED DESCRIPTION OF THE INVENTION

When describing pyrimidine substituted derivatives, compositions, combinations and methods of the invention the following terms have the following meaning, unless otherwise indicated.

As used herein, the term $C_{1-4}$ alkyl embraces linear or branched radicals having 1 to 4 carbon atoms. Analogously the term $C_{1-3}$ alkyl embraces unsubstituted linear or branched radicals having 1 to 3 carbon atoms and the term $C_{1-2}$ alkyl embraces unsubstituted linear or branched radicals having 1 to 2 carbon atoms. Examples of such radicals include methyl, ethyl, n-propyl, i-propyl, n-butyl, sec-butyl and t-butyl.

As used herein, the term $C_{1-3}$ alkenyl embraces linear or branched, mono or polyunsaturated radicals having 1 to 3 carbon atoms. Examples of such radicals include vinyl, allyl, 1-propenyl and isopropenyl.

As used herein, the term $C_{1-4}$ deuterioalkyl embraces linear or branched radicals having 1 to 4 carbon atoms. Examples of such radicals include monodeuteriomethyl, dideuteriomethyl, trideuteriomethyl, trideuterioethyl, pentadeuteriopropyl and trideuteriobutyl.

As used herein, the term $C_{3-6}$ cycloalkyl embraces saturated monocyclic carbocyclic radicals having from 3 to 6 carbon atoms. Examples of monocyclic cycloalkyl groups include cyclopropyl, cycobutyl, cyclopentyl and cyclohexyl.

As used herein, the term 5- to 6-membered heteroaryl group is a monocyclic 5- to 6-membered ring system, containing at least one heteroatom selected from O, N and S. Analogously, the term 5-membered heteroaryl group is a monocyclic 5-membered ring system, containing at least one heteroatom selected from O, N and S. Analogously, the term 6-membered heteroaryl group is a monocyclic 6- membered ring system, containing at least one heteroatom selected from O, N and S. Examples of such radicals include pyridyl, pyrazinyl, pyrimidinyl, pyridazinyl, furyl, oxadiazolyl, pyrazolyl, oxazolyl, isoxazolyl, imidazolyl or pyrrolyl. Such heteroaryl radical is typically unsubstituted or substituted by

1, 2 or 3 substituents which may be the same or different.

As used herein, the term 5- to 6-membered N-heteroaryl group is a monocyclic 5- to 6-membered ring system, containing one or more N atoms wherein only N atoms are present as a ring heteroatom. Examples of such radicals include pyridyl, pyrazinyl, pyrimidinyl, pyridazinyl, pyrazolyl, imidazolyl, triazolyl or pyrrolyl. Such N-heteroaryl radical is typically unsubstituted or substituted by 1, 2 or 3 substituents which may be the same or different.

As used herein, the term $C_{1-4}$ alkoxy (or alkyloxy) embraces a linear or branched oxy-containing radicals each having alkyl portions of 1 to 4 carbon atoms. Examples of alkoxy radicals include methoxy, ethoxy, n-propxy, i-propoxy, n-butoxy, sec-butoxy or t-butoxy.

Analogously, the term $C_{1-3}$ alkoxy embraces a linear or branched oxy-containing radicals having alkyl portion of 1 to 3 carbon atoms. Examples of alkoxy radicals include methoxy, ethoxy, n-propxy or i-propoxy.

Analogously, the term $C_{1-2}$ alkoxy embraces a linear or branched oxy-containing radicals having alkyl portion of 1 to 2 carbon atoms. Examples of alkoxy radicals include methoxy, or ethoxy.

As used herein, the term $C_{1-4}$ haloalkyl embraces a linear or branched $C_{1-4}$ alkyl group, which is substituted by one or more, preferably 1, 2 or 3 halogen atoms. Analogously, the term $C_{1-3}$ haloalkyl embraces a linear or branched $C_{1-3}$ alkyl group, which is substituted by one or more, preferably 1, 2 or 3 halogen atoms. Analogously, the term $C_{1-2}$ haloalkyl embraces a linear or branched $C_{1-2}$ alkyl group, which is substituted by one or more, preferably 1, 2 or 3 halogen atoms. Examples of haloalkyl groups include $CCl_3$, $CF_3$, $CHF_2$, $CH_2CF_3$, $CH_2CHF_2$, $CH_2CHFCH_3$, $CH_2CH_2CH_2Cl$ or $CH_2CH_2CH_2CHF_2$.

As used herein, the term $C_{1-2}$ haloalkoxy embraces linear or branched $C_{1-2}$ alkoxyl groups in which one or more hydrogen radicals are replaced by a halo group. Examples of haloalkoxy groups include trifluoromethoxy, bromomethoxy and 1,2-dichloroethoxy.

As used herein, the term $C_{1-5}$ hydroxyalkyl embraces linear or branched alkyl radicals having 1 to 5 carbon atoms, any one of which may be substituted with one or more hydroxyl radicals.

Analogously, the term $C_{1-4}$ hydroxyalkyl embraces linear or branched alkyl radicals having 1 to 4 carbon atoms, any one of which may be substituted with one or more hydroxyl radicals. Analogously, the term $C_{1-3}$ hydroxyakyl embraces linear or branched alkyl radicals having 1 to 3 carbon atoms, any one of which may be substituted with one or more hydroxyl radicals. Examples of such radicals include hydroxymethyl, hydroxyethyl, hydroxypropyl, hydroxybutyl, 2,3-dihydroxypropyl and 1,3-dihydroxypropan-2-yl.

As used herein, the term $C_{1-3}$ alkylamino embraces amino groups which are substituted by one or more alkyl groups having 1 to 3 carbon atoms. Examples of such radicals include methylamine, dimethylamino, ethylmethylamine, ethylamine and propylamine.

As used herein the term -($C_{1-4}$ alkyl)-($C_{1-4}$ alkoxy) embraces linear or branched radicals having 1 to 4 carbon atoms substituted with a $C_{1-4}$ alkoxy group. Analogously, the term -($C_{1-3}$ alkyl)-($C_{1-3}$ alkoxy) embraces linear or branched radicals having 1 to 3 carbon atoms substituted with a $C_{1-3}$ alkoxy group. Examples of such radicals include methyl-methoxy, methyl-ethoxy, ethyl-methoxy, ethyl-ethoxy, propyl-methoxy, methyl-propoxy, propyl-ethoxy, ethyl-propoxy, propyl-propoxy, butyl-methoxy and buthyl-ethoxy.

As used herein, the term 4- to 6- membered heterocyclyl radical embraces typically a monocyclic non-aromatic, saturated or unsaturated $C_{4-6}$ carbocyclic ring system in which one or more, for example, 1, 2, 3 or 4 of the carbon atoms preferably 1 or 2 of the carbon atoms are replaced by a heteroatom selected from N, O and S. Examples of such radicals include piperidinyl, pyrrolidinyl, pyrrolinyl, piperazinyl, oxetanyl, morpholinyl, thiomorpholinyl, pyrrolyl, pyrazolinyl, pirazolidinyl, triazolyl, pyrazolyl, tetrazolyl, imidazolidinyl, 4,5-dihydro-oxazolyl, 1,3-dioxol-2-one, tetrahydrofuranyl, 3-aza-tetrahydrofuranyl, tetrahydrothiophenyl, tetrahydropyranyl, tetrahydrothiopyranyl, dihydropyranyl and 1,4-azathianyl.

Analogously, the term 5- to 6- membered heterocyclic radical embraces typically a monocyclic non-aromatic, saturated or unsaturated $C_{5-6}$ carbocylic ring system in which one or more, for example, 1, 2, 3 or 4 of the carbon atoms preferably 1 or 2 of the carbon atoms are replaced by a heteroatom selected from N, O and S. Examples of 5- to 6-membered heterocyclyl radicals include piperidinyl, pyrrolidinyl, pyrrolinyl, piperazinyl, morpholinyl, thiomorpholinyl, pyrrolyl, pyrazolinyl, pirazolidinyl, triazolyl, pyrazolyl, tetrazolyl, imidazolidinyl, 4,5-dihydro-oxazolyl, 1,3-dioxol-2-one, tetrahydrofuranyl, 3-aza-tetrahydrofuranyl, tetrahydrothiophenyl, tetrahydropyranyl, tetrahydrothiopyranyl and 1,4-azathianyl.

Analogously, the term 5- membered heterocyclic radical embraces typically a monocyclic non-aromatic, saturated or unsaturated $C_5$ carbocylic ring system in which one or more, for example, 1, 2, 3 or 4 of the carbon atoms preferably 1 or 2 of the carbon atoms are replaced by a heteroatom selected from N, O and S. Examples of 5- membered heterocyclyl radicals include pyrrolidinyl, pyrrolinyl, pyrrolyl, pyrazolinyl, pirazolidinyl, triazolyl, pyrazolyl, tetrazolyl, imidazolidinyl, 4,5-dihydro-oxazolyl, 1,3-dioxol-2-one, tetrahydrofuranyl and 3-aza-tetrahydrofuranyl.

Analogously, the term 6- membered heterocyclic radical embraces typically a monocyclic non-aromatic, saturated or unsaturated $C_6$ carbocylic ring system in which one or more, for example, 1, 2, 3 or 4 of the carbon atoms preferably 1 or 2 of the carbon atoms are replaced by a heteroatom selected from N, O and S. Examples of 6- membered heterocyclyl radicals include piperidinyl, piperazinyl, morpholinyl, thiomorpholinyl, tetrahydrothiophenyl, tetrahydropyranyl, tetrahydrothiopyranyl and 1,4-azathianyl.

As used herein, the term halogen atom embraces fluorine, chlorine, bromine and iodine. A halogen atom is typically a fluorine, chlorine or bromine atom. The term halo when used as a prefix has the same meaning.

As used herein, some of the atoms, radicals, moieties, chains and cycles present in the general structures of the invention are "unsubstituted or substituted". This means that these atoms, radicals, moieties, chains and cycles can be either unsubstituted or substituted in any position by one or more, for example 1, 2, 3 or 4, substituents, whereby the hydrogen atoms bound to the unsubstituted atoms, radicals, moieties, chains and cycles are replaced by chemically acceptable atoms, radicals, moieties, chains and cycles.

Compounds containing one or more chiral centers may be used in enantiomerically or diastereomerically pure form, in the form of a racemic mixtures and in the form of mixtures enriched in one or more stereoisomer. The scope of the invention as described and claimed encompasses the racemic forms of the compounds as well as the individual enantiomers, diastereomers and stereoisomer-enriched mixtures.

Conventional techniques for the preparation/isolation of individual enantiomers include chiral synthesis from a suitable optically pure precursor or resolution of the racemate using, for example, chiral high pressure liquid chromatography (HPLC). Alternatively, the racemate (or a racemic precursor) may be reacted with a suitable optically active compound, for example, an alcohol, or, in the case where the compound contains an acidic or basic moiety, an acid or base such as tartaric acid or 1-phenethylamine. The resulting diastereomeric mixture may be separated by chromatography and/or fractional crystallization and one or both of the diastereoisomers converted to the corresponding pure enantiomer(s) by means well known to one skilled in the art. Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% isopropanol, typically from 2 to 20% and from 0 to 5% of an alkylamine, typically 0.1% diethylamine. Concentration of the eluate affords the enriched mixture. Stereoisomer conglomerates may be separated by conventional techniques known to those skilled in the art. See, e.g. "Stereochemistry of Organic Compounds" by Ernest L. Eliel (Wiley, New York, 1994).

The term "therapeutically effective amount" refers to an amount sufficient to effect treatment when administered to a patient in need of treatment.

The term "treatment" as used herein refers to the treatment of a disease or medical condition in a human patient which includes:

(a) preventing the disease or medical condition from occurring, i.e., prophylactic treatment of a patient;

(b) ameliorating the disease or medical condition, i.e., causing regression of the disease or medical condition in a patient;

(c) suppressing the disease or medical condition, i.e., slowing the development of the disease or medical condition in a patient; or

(d) alleviating the symptoms of the disease or medical condition in a patient.

The phrase "pathological condition or disease susceptible to amelioration by inhibiton of Tyk2 includes all disease states and/or conditions that are acknowledged now, or that are found in the future, to be associated with an increased Tyk2 activity. Such disease states include, but are not limited to, dermatological diseases, inflammatory or autoimmune-mediated diseases and a metabolism/endocrine function disorders.

As used herein, the term "pharmaceutically acceptable salt" refers to a salt prepared from a base or acid which is acceptable for administration to a patient, such as a mammal. Such salts can be derived from pharmaceutically-acceptable inorganic or organic bases and from pharmaceutically-acceptable inorganic or organic acids.

As used herein, a N-oxide is formed from the tertiary basic amines or imines present in the molecule, using a convenient oxidising agent.

The pyrimidine substituted derivatives of the invention may exist in both unsolvated and solvated forms. The term solvate is used herein to describe a molecular complex comprising a compound of the invention and an amount of one or more pharmaceutically acceptable solvent molecules. The term hydrate is employed when said solvent is water. Examples of solvate forms include, but are not limited to, compounds of the invention in association with water, acetone, dichloromethane, 2-propanol, ethanol, methanol, dimethylsulfoxide (DMSO), ethyl acetate, acetic acid, ethanolamine, or mixtures thereof.

The invention also includes isotopically-labelled pyrimidine substituted derivatives of the invention, wherein one or more atoms is replaced by an atom having the same atomic number, but an atomic mass or mass number different from the atomic mass or mass number usually found in nature. Examples of isotopes suitable for inclusion in the compounds of the invention include isotopes of hydrogen, such as $^{2}H$ and $^{3}H$, carbon, such as $^{11}C$, $^{13}C$ and $^{14}C$, chlorine,

such as $^{36}Cl$, fluorine, such as $^{18}F$, iodine, such as $^{123}I$ and $^{125}I$, nitrogen, such as $^{13}N$ and $^{15}N$, oxygen, such as $^{15}O$, $^{17}O$ and $^{18}O$, phosphorus, such as $^{32}P$, and sulfur, such as $^{35}S$. Preferred isotopically-labelled compounds include deuterated derivatives of the compounds of the invention. As used herein, the term deuterated derivative embraces compounds of the invention where in a particular position at least one hydrogen atom is replaced by deuterium. Deuterium (D or $^{2}H$) is a stable isotope of hydrogen which is present at a natural abundance of 0.015 molar %.

Isotopically-labelled pyrimidine substituted derivatives of the invention can generally be prepared by conventional techniques known to those skilled in the art or by processes analogous to those described herein, using an appropriate isotopically-labelled reagent in place of the non-labelled reagent otherwise employed.

As used in the present invention, the term tautomer means two or more forms or isomers of an organic compound that readily could be interconverted into each other via a common chemical reaction called tautomerization. This reaction commonly results in the formal migration of a hydrogen atom or proton, accompanied by a switch of a single bond and adjacent double bond. The concept of tautomerizations is called tautomerism. Because of the rapid interconversion, tautomers are generally considered to be the same chemical compound. In solutions in which tautomerization is possible, a chemical equilibrium of the tautomers will be reached. The exact ratio of the tautomers depends on several factors, including temperature, solvent and pH.

As used herein, the term deuterated derivatives embrace compounds of the invention where in a particular position at least one hydrogen atom is replaced by deuterium. Deuterium (D or $^{2}H$) is a stable isotope of hydrogen which is present at a natural abundance of 0.015% molar.

Hydrogen deuterium exchange (deuterium incorporation)- is a chemical reaction in which a covalently bonded hydrogen atom is replaced by a deuterium atom. Said exchange (incorporation) reaction can be total or partial.

Prodrugs of the pyrimidine substituted derivatives described herein are also within the scope of the invention. Thus certain derivatives of the pyrimidine substituted derivatives of the present invention, which derivatives may have little or no pharmacological activity themselves, when administered into or onto the body may be converted into compounds of the present invention having the desired activity, for example, by hydrolytic cleavage. Such derivatives are referred to as 'prodrugs'. Further information on the use of prodrugs may be found in Prodrugs as Novel Delivery Systems, Vol. 14, ACS Symposium Series (T. Higuchi and W. Stella) and Bioreversible Carriers in Drug Design, Pergamon Press, 1987 (ed. E. B. Roche, American Pharmaceutical Association).

Prodrugs in accordance with the invention can, for example, be produced by replacing appropriate functionalities present in the compounds of the present invention with certain moieties known to those skilled in the art as 'pro-moieties' as described, for example, in Design of Prodrugs by H. Bundgaard (Elsevier, 1985).

In the case of pyrimidine substituted derivatives that are solids, it is understood by those skilled in the art that the compounds and salts may exist in different crystalline or polymorphic forms, or in an amorphous form, all of which are intented to be within the scope of the present invention.

Typically, $G^1$ is selected from the group consisting of a phenyl group and a pyridine group, wherein the phenyl and pyridine groups are unsubstituted or substituted by one or more substituents selected from a linear or branched $C_{1-2}$ alkyl group, a linear or branched $C_{1-2}$ alkoxy group, a halogen atom, a linear or branched $C_{1-2}$ haloalkyl group, a CN group, a-$P(O)(CH_3)_2$ group, a -$NR^aSO_2R^b$ group, a linear or branched $C_{1-3}$ hydroxyalkyl group, a -(linear or branched $C_{1-2}$ alkyl group)$_{0-1}$-$SO_nCH_3$ group, a -$S(O)CH_3$=$NCH_3$ and a -C(O)-$R^f$ group.

Preferably, $G^1$ is selected from the group consisting of a phenyl group and a pyridine group, wherein the phenyl and pyridine groups are unsubstituted or substituted by one or more substituents selected from a linear or branched $C_{1-2}$ alkoxy group, a -$NR^aSO_2R^b$ group, a $SO_2CH_3$ group, a -$S(O)CH_3$=$NCH_3$ and a -$P(O)(CH_3)_2$ group.

More preferably, $G^1$ represents a phenyl group, wherein the phenyl is unsubstituted or substituted by one or more substituents selected from a methoxy groups and a $NCH_3SO_2CH_3$ groups.

Typically, $G^2$ is absent or is selected from the group consisting of a 5-membered heteroaryl group containing at least one heteroatom selected from N or O and a 5- to 6-membered heterocyclyl group containing at least one heteroatom selected from N or O, wherein the heteroaryl and heterocyclyl groups are unsubstituted or substituted by one or more substituents selected from a linear or branched $C_{1-2}$ alkyl group or a linear or branched ($C_{1-3}$ alkyl) -($C_{1-3}$ alkoxy) group, a linear or branched $C_{1-2}$ haloalkyl group and a $C(O)R^g$ group.

Preferably, $G^2$ represents a 5-membered heteroaryl group containing at least two atoms of N, wherein the heteroaryl is unsubstituted or substituted by one or more methyl group and a linear or branched $C_{1-2}$ haloalkyl group.

Typically, $R^1$ represents a -C(O)-$R^2$.

Typically, $R^2$ represents a cyclopropyl group, which is unsubstituted or substituted by one or more fluorine atoms.

Preferably, R2 represents a cyclopropyl group, which is substituted by a fluorine atom.

Typically, $R^1$ represents a $G^3$ group.

Typically, $G^3$ group represents a pyrimidinyl group, wherein the pyrimidinyl group is substituted by one or more substituents selected from a methyl group and a fluorine atom.

Typically, $R^a$ represents a methyl group.

Typically, $R^b$ represents a methyl group or a trifluoromethyl group.

In one embodiment,

- $G^1$ is selected from the group consisting of a phenyl group and a 6-membered heteroaryl group containing one atom of N,
  wherein the phenyl and heteroaryl group are unsubstituted or substituted by one or more substituents selected from a linear or branched $C_{1-2}$ alkoxy group, a $-NR^aSO_2R^b$ group, a $SO_2CH_3$ group, a $-S(O)CH_3=NCH_3$ or a $-P(O)(CH_3)_2$ group;
- $G^2$ is selected from the group consisting of a 5-membered heteroaryl group containing at least one heteroatom selected from N or O and a 5- to 6-membered heterocyclyl group containing at least one heteroatom selected from N or O,
  wherein the heteroaryl group is unsubstituted or substituted by one or more substituents selected from a linear or branched $C_{1-2}$ alkyl group or a linear or branched ($C_{1-3}$ alkyl) -($C_{1-3}$ alkoxy) group;
- $R^1$ represents a $-C(O)-R^2$;
- $R^2$ represents a cyclopropyl group, which is unsubstituted or substituted by one or more fluorine atoms;
- $R^a$ represents a methyl group; and
- $R^b$ represents a methyl group or a trifluoromethyl group.

In another embodiment,

- $G^1$ is selected from the group consisting of a phenyl group and a 6-membered heteroaryl group containing one atom of N, wherein the phenyl and heteroaryl group are unsubstituted or substituted by one or more substituents selected from a linear or branched $C_{1-2}$ alkyl group, a linear or branched $C_{1-2}$ alkoxy group, a halogen atom, a-$NR^aSO_2R^b$ group, a $SO_2CH_3$ group, a $-S(O)CH_3=NCH_3$ or a $-P(O)(CH_3)_2$ group;
- $G^2$ is selected from the group consisting of a 5-membered heteroaryl group containing at least one heteroatom selected from N or O and a 5- to 6-membered heterocyclyl group containing at least one heteroatom selected from N or O,
  wherein the heteroaryl group is unsubstituted or substituted by one or more substituents selected from a linear or branched $C_{1-2}$ alkyl group, a linear or branched $C_{1-2}$ haloalkyl group or a linear or branched ($C_{1-3}$ alkyl) -($C_{1-3}$ alkoxy) group;
- $R^1$ represents a $G^3$ group;
- $G^3$ group represents a pyrimidinyl group, wherein the pyrimidinyl group is substituted by one or more substituents selected from methyl group and a fluorine atom.

In a preferred embodiment:

- $G^1$ represents a phenyl group, wherein the phenyl is unsubstituted or substituted by one or more substituents selected from a methoxy group or a $NCH_3SO_2CH_3$ group; and
- $G^2$ represents a 5-membered heteroaryl group containing at least two atoms of N, wherein the heteroaryl is unsubstituted or substituted by one or more methyl groups and a linear or branched $C_{1-2}$ haloalkyl groups.

In a more preferred embodiment:

- $G^1$ represents a phenyl group or a pyridine group, wherein the phenyl and pyridine groups are unsubstituted or substituted by one or more substituents selected from a methyl group, an allyl group, a methoxy group, an ethoxy group, a $-(CH_2)OCH_3$ group, a fluorine group, a bromine atom, a trifluoromethyl group, a trifluoromethoxy group, a $-(CH_2)_{0-2}SO_2CH_3$ group, a $-NR^aSO_2R^b$ group, a -CN group, a $-S(O)Me=NCH_3$ group, a $C(CH_3)_2OH$ group, a $-SO_2$-N-pyperidyl group, a $-PO(CH_3)_2$ group or a $-C(O)R^f$ group;
- $G^2$ is absent or selected from the group consisting of a pyrazolyl group, a pyrimidinyl group, an imidazolyl group, a triazolyl group, an oxadiazolyl group, a tetrazolyl group, a cyclopropyl group, a piperidinyl group, a $CH_2$-phenyl group, a pyrrolidinyl group, a tetrahydrofuranyl group, a tetrahydropyranyl group, a dihydropyranyl group, a pyridazinyl group and an oxazolyl group,
  wherein the pyrazolyl, thea pyrimidinyl, the imidazolyl, the triazolyl, the oxadiazolyl, the tetrazolyl, the cyclopropyl, the piperidinyl, the $CH_2$-phenyl, the pyrrolidinyl, the tetrahydrofuranyl, the tetrahydropyranyl, the dihydropyranyl, the pyridazinyl and the oxazolyl groups are unsubstituted or substituted by one or more substituents selected from a methyl group, an ethyl group, a $-CF_3$ group, a $-CH_2OCH_3$ group, a $-(CH_2)_2OCH_3$ group, a fluorine atom, a $-CH_2CF_3$ group, a $-(CH_2)$-tetrahydropyranyl group, a-$(CH_2)$-morpholinyl group, a-C(O)OtBu group, a-$(CH_2)$-tetrahydrofuranyl group, a-$(CH_2)_{0-1}$-oxetanyl group, a difluorosubstituted $-CH_2$-cyclobutyl group, a difluorosubstituted cyclopropyl group, a $CHF_2$ group and an hydroxyl substituted cyclobutyl group;

- $R^1$ represents a -C(O)-$R^2$ group or a $G^3$ group;
- $R^2$ represents a cyclopropyl group, wherein the cyclopropyl group is unsubstituted or substituted by a fluorine atom;
- $G^3$ represents a pyridyl group, a pyrimidinyl group, a pyridazinyl group, a triazolyl group or a pyrazolyl group, wherein the pyridyl, the pyrimidinyl, the pyridazinyl, the triazolyl and the pyrazolyl groups are substituted by one or more substituents selected from a methyl group, a tetrahydropyranyl group, a CN group and a fluorine atom;
- $R^a$ represents a hydrogen atom, a methyl group or a trideuteromethyl group;
- $R^b$ represents a methyl group or trifluoromethyl group; and
- $R^f$ represents a pyrrolidinyl group or a -$N(CH_3)_2$ group.

Particular individual compounds of the invention include:

N-[6-[2-methoxy-3-(1-methyl-1,2,4-triazol-3-yl)anilino]pyrimidin-4 yl]cyclopropanecarboxamide,

N-[6-(2-methoxyanilino) pyrimidin-4-yl] cyclopropanecarboxamide,

N-[6-(2-methoxy-3-methyl-anilino)pyrimidin-4-yl]cyclopropanecarboxamide,

N6-(2,6-dimethylpyrimidin-4-yl)-N4-[2-methoxy-3-(1-methyl-1,2,4-triazol-3-yl)phenyl]pyrimidine-4,6-diamine,

N6-(2,6-dimethylpyrimidin-4-yl)-N4-(3-methoxyphenyl)pyrimidine-4,6-diamine,

N6-(2,6-dimethylpyrimidin-4-yl)-N4-(2-methoxy-3-methyl-phenyl)pyrimidine-4,6-diamine,

N6-(2,6-dimethylpyrimidin-4-yl)-N4-(3-fluoro-2-methoxy-phenyl) pyrimidine-4,6-diamine,

N4-(2,6-dimethylpyrimidin-4-yl)-N6-(3-methoxy-6-methyl-2-pyridyl)pyrimidine-4,6-diamine ,

N4-(2-methoxy-3-methyl-phenyl)-N6-(1-tetrahydropyran-4-ylpyrazol-4-yl)pyrimidine-4,6-diamine,

N6-(2,6-dimethylpyrimidin-4-yl)-N4-[2-(trifluoromethoxy)phenyl] pyrimidine-4,6-diamine,

N6-(2,6-dimethylpyrimidin-4-yl)-N4-(2-methylsulfonylphenyl)pyrimidine-4,6-diamine,

N-[2-[[6-[(2,6-dimethylpyrimidin-4-yl)amino]pyrimidin-4-yl]amino]phenyl]-N-methyl-methanesulfonamide,

N-methyl-N-[2-[[6-[(1-methyl-1,2,4-triazol-3-yl)amino]pyrimidin-4-yl]amino]phenyl]methanesulfonamide,

N-[6-[2-[methyl(methylsulfonyl)amino]anilino]pyrimidin-4-yl]cyclopropanecarboxamide,

N4-(2,6-dimethylpyrimidin-4-yl)-N6-[2-methoxy-3-(1-methylpyrazol-4-yl)phenyl]pyrimidine-4,6-diamine,

3-[[6-[(2,6-dimethylpyrimidin-4-yl)amino]pyrimidin-4-yl]amino]-2-methoxy-benzonitrile,

N4-(2,4-dimethoxyphenyl)-N6-(2,6-dimethylpyrimidin-4-yl)pyrimidine-4,6-diamine,

N6-(2,6-dimethylpyrimidin-4-yl)-N4-[2-methoxy-3-(trifluoromethyl) phenyl] pyrimidine-4,6-diamine,

N4-(3,4-difluoro-2-methoxy-phenyl)-N6-(2,6-dimethylpyrimidin-4-yl)pyrimidine-4,6-diamine,

N4-(2,3-dimethoxyphenyl)-N6-(2,6-dimethylpyrimidin-4-yl)pyrimidine-4,6-diamine,

N4-(2,6-dimethylpyrimidin-4-yl)-N6-(4-fluoro-2-methoxy-phenyl)pyrimidine-4,6-diamine,

N6-(2,6-dimethylpyrimidin-4-yl)-N4-[2-methoxy-4-(trifluoromethyl)phenyl]pyrimidine-4,6-diamine,

N6-(2,6-dimethylpyrimidin-4-yl)-N4-(2-methoxyphenyl)pyrimidine-4,6-diamine,

N-[2-[[6-[(2,6-dimethylpyrimidin-4-yl)amino]pyrimidin-4-yl]amino]-5-(3-methylimidazol-4-yl)phenyl]-N-methyl-methanesulfonamide,

N-[5-(1,5-dimethylpyrazol-4-yl)-2-[[6-[(2,6-dimethylpyrimidin-4-yl)amino]pyrimidin-4-yl]amino]phenyl]-N-methyl-methanesulfonamide,

N4-(2,6-dimethylpyrimidin-4-yl)-N6-[4-(3-methylimidazol-4-yl)-2-methylsulfonyl-phenyl]pyrimidine-4,6-diamine,

6-[[6-[(2,6-dimethylpyrimidin-4-yl)amino]pyrimidin-4-yl]amino]-5-methoxy-pyridine-3-carbonitrile,

4-N-(2-dimethylphosphorylphenyl)-6-N-(2,6-dimethylpyrimidin-4-yl)pyrimidine-4,6-diamine,

N6-(2,6-dimethylpyrimidin-4-yl)-N4-[2-methoxy-3-[1-methyl-5-(trifluoromethyl)-1,2,4-triazol-3-yl]phenyl]pyrimidine-4,6-diamine,

N4-(2,6-dimethylpyrimidin-4-yl)-N6-[2-methoxy-3-[5-(methoxymethyl)-1,2,4-oxadiazol-3-yl]phenyl]pyrimidine-4,6-diamine,

N4-(2,6-dimethylpyrimidin-4-yl)-N6-[3-(5-fluoropyrimidin-2-yl)-2-methoxy-phenyl]pyrimidine-4,6-diamine,

N-[2-[[6-[(6-cyano-2-pyridyl)amino]pyrimidin-4-yl]amino]phenyl]-N-methyl-methanesulfonamide,

N6-[2-methoxy-3-(1-methyl-1,2,4-triazol-3-yl)phenyl]-N4-(6-methylpyrimidin-4-yl)pyrimidine-4,6-diamine,

N-[2-[[6-[(2,6-dimethylpyrimidin-4-yl)amino]pyrimidin-4-yl]amino]phenyl]-N-(trideuteriomethyl)methanesulfonamide,

N-[3-[[6-[(2,6-dimethylpyrimidin-4-yl)amino]pyrimidin-4-yl]amino]pyridin-4-yl]-N-methylmethanesulfonamide,

N6-(2,6-dimethylpyrimidin-4-yl)-N4-[2-methoxy-3-[2-methyl-5-(trifluoromethyl)pyrazol-3-yl]phenyl]pyrimidine-4,6-diamine,

N4-(2,6-dimethylpyrimidin-4-yl)-N6-[2-methoxy-3-(5-methyl-1,2,4-oxadiazol-3-yl)phenyl]pyrimidine-4,6-diamine,

N6-(2,6-dimethylpyrimidin-4-yl)-N4-[2-methoxy-3-[5-methyl-1-(2,2,2-trifluoroethyl)-1,2,4-triazol-3-yl]phenyl]pyrimidine-4,6-diamine,

N6-(2,6-dimethylpyrimidin-4-yl)-N4-[3-(1,5-dimethyl-1,2,4-triazol-3-yl)-2-methoxy-phenyl]pyrimidine-4,6-diamine,

N6-(2,6-dimethylpyrimidin-4-yl)-N4-[3-(2,5-dimethyl-1,2,4-triazol-3-yl)-2-methoxy-phenyl]pyrimidine-4,6-diamine,

N4-(2,6-dimethylpyrimidin-4-yl)-N6-[2-methoxy-3-(2-methyltetrazol-5-yl)phenyl]pyrimidine-4,6-diamine,

N6-(2,6-dimethylpyrimidin-4-yl)-N4-(2-methoxy-3-pyridazin-4-yl-phenyl)pyrimidine-4,6-diamine,

N4-(2,6-dimethylpyrimidin-4-yl)-N6-[2-methoxy-3-[1-(oxetan-3-yl)-1,2,4-triazol-3-yl]phenyl]pyrimidine-4,6-diamine,

N6-(2,6-dimethylpyrimidin-4-yl)-N4-[2-methoxy-3-[1-(2-methoxyethyl)-1,2,4-triazol-3-yl]phenyl]pyrimidine-4,6-diamine,

N4-(2,6-dimethylpyrimidin-4-yl)-N6-[2-ethoxy-3-(1-methyl-1,2,4-triazol-3-yl)phenyl]pyrimidine-4,6-diamine,

N4-(2,6-dimethylpyrimidin-4-yl)-N6-[3-(1-ethyl-1,2,4-triazol-3-yl)-2-methoxyphenyl]pyrimidine-4,6-diamine,

N4-(2,6-dimethylpyrimidin-4-yl)-N6-[2-methoxy-3-[1-(tetrahydropyran-2-ylmethyl)-1,2,4-triazol-3-yl]phenyl]pyrimidine-4,6-diamine,

N4-(2,6-dimethylpyrimidin-4-yl)-N6-[2-methoxy-3-[5-methyl-2-(oxetan-3-yl)-1,2,4-triazol-3-yl]phenyl]pyrimidine-4,6-diamine,

N4-(2,6-dimethylpyrimidin-4-yl)-N6-[2-methoxy-3-[5-methyl-1-(oxetan-3-yl)-1,2,4-triazol-3-yl]phenyl]pyrimidine-4,6-diamine,

N6-(3-bromo-2-methoxy-phenyl)-N4-(2,6-dimethylpyrimidin-4-yl)pyrimidine-4,6-diamine,

N6-(2,6-dimethylpyrimidin-4-yl)-N4-[2-methoxy-3-[1-(oxetan-3-ylmethyl)-1,2,4-triazol-3-yl]phenyl]pyrimidine-4,6-diamine,

N4-(2,6-dimethylpyrimidin-4-yl)-N6-[2-methoxy-3-[1-(tetrahydropyran-4-ylmethyl)-1,2,4-triazol-3-yl]phenyl]pyrimidine-4,6-diamine,

N6-[3-[1-[(3,3-difluorocyclobutyl)methyl]-1,2,4-triazol-3-yl]-2-methoxy-phenyl]-N4-(2,6-dimethylpyrimidin-4-yl)pyrimidine-4,6-diamine,

N4-[3-[1-(difluoromethyl)-1,2,4-triazol-3-yl]-2-methoxy-phenyl]-N6-(2,6-dimethylpyrimidin-4-yl)pyrimidine-4,6-diamine,

N6-[3-[1-(2,2-difluorocyclopropyl)-1,2,4-triazol-3-yl]-2-methoxy-phenyl]-N4-(2,6-dimethylpyrimidin-4-yl)pyrimidine-4,6-diamine,

N6-(2,6-dimethylpyrimidin-4-yl)-N4-(2-methoxy-3-tetrahydropyran-2-yl-phenyl)pyrimidine-4,6-diamine,

N4-(2,6-dimethylpyrimidin-4-yl)-N6-[2-methoxy-3-[1-(tetrahydropyran-3-ylmethyl)-1,2,4-triazol-3-yl]phenyl]pyrimidine-4,6-diamine,

3-[3-[3-[[6-[(2,6-dimethylpyrimidin-4-yl)amino]pyrimidin-4-yl]amino]-2-methoxy-phenyl]-1,2,4-triazol-1-yl]cyclobutanol,

N4-(2,6-dimethylpyrimidin-4-yl)-N6-[2-methoxy-3-[1-(tetrahydrofuran-2-ylmethyl)-1,2,4-triazol-3-yl]phenyl]pyrimidine-4,6-diamine,

N6-(2,6-dimethylpyrimidin-4-yl)-N4-[2-methoxy-3-[1-(tetrahydrofuran-3-ylmethyl)-1,2,4-triazol-3-yl]phenyl]pyrimidine-4,6-diamine,

N-(2-((6-((2,6-dimethylpyrimidin-4-yl)amino)pyrimidin-4-yl)amino)-4-methylpyridin-3-yl)-N-methylmethanesulfonamide,

N6-(2,6-dimethylpyrimidin-4-yl)-N4-[2-methoxy-3-[5-(morpholinomethyl)-1,2,4-oxadiazol-3-yl]phenyl]pyrimidine-4,6-diamine,

6-N-(2,6-dimethylpyrimidin-4-yl)-4-N-[2-methoxy-4-methyl-3-(1-methyl-1,2,4-triazol-3-yl)phenyl]pyrimidine-4,6-diamine,

N4-(2,6-dimethylpyrimidin-4-yl)-N6-[4-fluoro-2-methoxy-3-(1-methyl-1,2,4-triazol-3-yl)phenyl]pyrimidine-4,6-diamine,

N4-(5-fluoro-2-pyridyl)-N6-[2-methoxy-3-(1-methyl-1,2,4-triazol-3-yl)phenyl]pyrimidine-4,6-diamine,

N4-(2,6-dimethylpyrimidin-4-yl)-N6-[2-(methylsulfonylmethyl)phenyl]pyrimidine-4,6-diamine,

N-[5-bromo-2-[[6-[(2,6-dimethylpyrimidin-4-yl)amino]pyrimidin-4-yl]amino]phenyl]-N-methyl-methanesulfonamide,

N-[2-[[6-[(2,6-dimethylpyrimidin-4-yl)amino]pyrimidin-4-yl]amino]-5-methyl-phenyl]-N-methyl-methanesulfonamide,

N-[5-cyclopropyl-2-[[6-[(2,6-dimethylpyrimidin-4-yl)amino]pyrimidin-4-yl]amino]phenyl]-N-methyl-methanesulfonamide,

N-[2-[[6-[(2,6-dimethylpyrimidin-4-yl)amino]pyrimidin-4-yl]amino]phenyl]-1,1,1-trifluoro-N-methyl-methanesulfonamide,

N4-(2,6-dimethylpyrimidin-4-yl)-N6-[2-(1-methylsulfonylethyl)phenyl]pyrimidine-4,6-diamine,

N6-(2,6-dimethylpyrimidin-4-yl)-N4-[2-(1-piperidylsulfonyl)phenyl]pyrimidine-4,6-diamine,

N-[2-[[6-[(2,6-dimethylpyrimidin-4-yl)amino]pyrimidin-4-yl]amino]-4-fluorophenyl]-N-methylmethanesulfonamide,

N-[2-[[6-[(2,6-dimethylpyrimidin-4-yl)amino]pyrimidin-4-yl]amino]-6-fluorophenyl]-N-methylmethanesulfonamide,

4-[[6-[(2,6-dimethylpyrimidin-4-yl)amino]pyrimidin-4-yl]amino]-N,N-dimethyl-3-[methyl(methylsulfonyl)amino]benzamide,

[4-[[6-[(2,6-dimethylpyrimidin-4-yl)amino]pyrimidin-4-yl]amino]-3-methoxy-phenyl]-pyrrolidin-1-yl-methanone,

N6-(2,6-dimethylpyrimidin-4-yl)-N4-[2-(1-methyl-1,2,4-triazol-3-yl)phenyl]pyrimidine-4,6-diamine,

N6-[4-(1,5-dimethylpyrazol-4-yl)-2-methoxy-phenyl]-N4-(2,6-dimethylpyrimidin-4-yl)pyrimidine-4,6-diamine,

N4-(2,6-dimethylpyrimidin-4-yl)-N6-[4-(5-fluoropyrimidin-2-yl)-2-methoxy-phenyl]pyrimidine-4,6-diamine,

N6-(2,6-dimethylpyrimidin-4-yl)-N4-[2-methoxy-4-[1-methyl-5-(trifluoromethyl)-1,2,4-triazol-3-yl]phenyl]pyrimidine-4,6-diamine,

N6-(2,6-dimethylpyrimidin-4-yl)-N4-[2-methoxy-3-methyl-4-(1-methyl-1,2,4-triazol-3-yl)phenyl]pyrimidine-4,6-diamine,

N4-[4-(1,5-dimethylpyrazol-4-yl)-2-methoxy-3-methyl-phenyl]-N6-(2,6-dimethylpyrimidin-4-yl)pyrimidine-4,6-diamine,

N6-(2,6-dimethylpyrimidin-4-yl)-N4-[2-methoxy-4-(1-methyl-1,2,4-triazol-3-yl)phenyl]pyrimidine-4,6-diamine,

N4-[4-(1,5-dimethylpyrazol-4-yl)-2-methylsulfonyl-phenyl]-N6-(2,6-dimethylpyrimidin-4-yl)pyrimidine-4,6-diamine,

N-[2-[[6-[(2,6-dimethylpyrimidin-4-yl)amino]pyrimidin-4-yl]amino]-5-[2-methyl-5-(trifluoromethyl)pyrazol-3-yl]-3-pyridyl]-N-methyl-methanesulfonamide,

N-[2-[[6-[(2,6-dimethylpyrimidin-4-yl)amino]pyrimidin-4-yl]amino]-5-(1,3,5-trimethylpyrazol-4-yl)-3-pyridyl]-N-methyl-methanesulfonamide,

N-[2-[[6-[(2,6-dimethylpyrimidin-4-yl)amino]pyrimidin-4-yl]amino]-5-(5-methyl-1H-pyrazol-4-yl)phenyl]-N-methyl-methanesulfonamide,

N4-(2,6-dimethylpyrimidin-4-yl)-N6-[2-methoxy-4-(1-methylimidazol-4-yl)phenyl]pyrimidine-4,6-diamine,

N4-(2,6-dimethylpyrimidin-4-yl)-N6-[2-methoxy-4-(1-methylimidazol-2-yl)phenyl]pyrimidine-4,6-diamine,

N-[5-(3,4-dihydro-2H-pyran-6-yl)-2-[[6-[(2,6-dimethylpyrimidin-4-yl)amino]pyrimidin-4-yl]amino]phenyl]-N-methyl-methanesulfonamide,

N6-(2,6-dimethylpyrimidin-4-yl)-N4-[2-methoxy-4-(5-methyl-1,2,4-oxadiazol-3-yl)phenyl]pyrimidine-4,6-diamine,

N-[5-(3,6-dihydro-2H-pyran-5-yl)-2-[[6-[(2,6-dimethylpyrimidin-4-yl)amino]pyrimidin-4-yl]amino]phenyl]-N-methyl-methanesulfonamide,

N-[5-(3,6-dihydro-2H-pyran-4-yl)-2-[[6-[(2,6-dimethylpyrimidin-4-yl)amino]pyrimidin-4-yl]amino]phenyl]-N-methyl-methanesulfonamide,

N4-[4-(1,5-dimethylpyrazol-4-yl)-2-fluoro-6-methylsulfonyl-phenyl]-N6-(2,6-dimethylpyrimidin-4-yl)pyrimidine-4,6-diamine,

N-[2-[[6-[(2,6-dimethylpyrimidin-4-yl)amino]pyrimidin-4-yl]amino]-5-(1,5-dimethyl-1,2,4-triazol-3-yl)phenyl]-N-methyl-methanesulfonamide,

N-[5-(1,5-dimethylpyrazol-4-yl)-2-[[6-[(2,6-dimethylpyrimidin-4-yl)amino]pyrimidin-4-yl]amino]phenyl]-N-(trideuteriomethyl)methanesulfonamide,

N-[2-[[6-[(2,6-dimethylpyrimidin-4-yl)amino]pyrimidin-4-yl]amino]-5-(2,5-dimethyl-1,2,4-triazol-3-yl)phenyl]-N-methyl-methanesulfonamide,

N-[2,4-dimethyl-6-[[6-[(6-methylpyrimidin-4-yl)amino]pyrimidin-4-yl]amino]phenyl]-N-methylmethanesulfonamide,

N-[2-[[6-[(5-fluoro-4-methyl-2-pyridyl)amino]pyrimidin-4-yl]amino]phenyl]-N-methyl-methanesulfonamide,

N-[2-[[6-[(5-cyano-2-pyridyl)amino]pyrimidin-4-yl]amino]phenyl]-N-methyl-methanesulfonamide,

N-[5-(1,5-dimethylpyrazol-4-yl)-2-[[6-[(5-fluoro-4-methyl-2-pyridyl)amino]pyrimidin-4-yl]amino]-3-pyridyl]-N-methyl-methanesulfonamide,

4-N-[2-methoxy-5-methyl-3-(1-methyl-1,2,4-triazol-3-yl)phenyl]-6-N-(6-methylpyrimidin-4-yl)pyrimidine-4,6-diamine,

N-[2-[[6-[(2,6-dimethylpyrimidin-4-yl)amino]pyrimidin-4-yl]amino]-5-tetrahydropyran-3-yl-phenyl]-N-methyl-methanesulfonamide,

N-[2-[[6-[(2,6-dimethylpyrimidin-4-yl)amino]pyrimidin-4-yl]amino]-5-tetrahydropyran-4-yl-phenyl]-N-methyl-methanesulfonamide,

N-[2-[[6-[(2,6-dimethylpyrimidin-4-yl)amino]pyrimidin-4-yl]amino]-5-tetrahydropyran-2-yl-phenyl]-N-methyl-methanesulfonamide,

N-[2-[[6-[(2,6-dimethylpyrimidin-4-yl)amino]pyrimidin-4-yl]amino]-5-vinyl-phenyl]-N-methyl-methanesulfonamide,

N-[5-benzyl-2-[[6-[(2,6-dimethylpyrimidin-4-yl)amino]pyrimidin-4-yl]amino]phenyl]-N-methyl-methanesulfonamide,

N-[2-[[6-[(2,6-dimethylpyrimidin-4-yl)amino]pyrimidin-4-yl]amino]-5-pyrrolidin-3-yl-phenyl]-N-methyl-methanesulfonamide,

N-[2-[[6-[(2,6-dimethylpyrimidin-4-yl)amino]pyrimidin-4-yl]amino]-5-tetrahydrofuran-3-yl-phenyl]-N-methyl-methanesulfonamide,

N6-(2,6-dimethylpyrimidin-4-yl)-N4-[2-(N,S-dimethylsulfonimidoyl)phenyl]pyrimidine-4,6-diamine,

N4-[4-(1,5-dimethylpyrazol-4-yl)-2-(N,S-dimethylsulfonimidoyl)phenyl]-N6-(2,6-dimethylpyrimidin-4-yl)pyrimidine-4,6-diamine,

N-[2-[[6-[(2,6-dimethylpyrimidin-4-yl)amino]pyrimidin-4-yl]amino]-5-tetrahydrofuran-2-yl-phenyl]-N-methyl-methanesulfonamide,

N-[2-[[6-[(2,6-dimethylpyrimidin-4-yl)amino]pyrimidin-4-yl]amino]-5-(methoxymethyl)phenyl]-N-methyl-methanesulfonamide;

N6-(2,6-dimethylpyrimidin-4-yl)-N4-[2-(N,S-dimethylsulfonimidoyl)-3-methyl-phenyl]pyrimidine-4,6-diamine;

N4-(2,6-dimethylpyrimidin-4-yl)-N6-(2-methoxy-4-tetrahydrofuran-3-yl-phenyl)pyrimidine-4,6-diamine;

N4-[4-cyclopropyl-2-(N,S-dimethylsulfonimidoyl)phenyl]-N6-(2,6-dimethylpyrimidin-4-yl)pyrimidine-4,6-diamine;

N6-(2,6-dimethylpyrimidin-4-yl)-N4-[3-methoxy-4-(1-methyl-1,2,4-triazol-3-yl)-2-pyridyl]pyrimidine-4,6-diamine;

N-[2-[[6-[(2,6-dimethylpyrimidin-4-yl)amino]pyrimidin-4-yl]amino]-3-pyridyl]-N-methyl-methanesulfonamide;

N-[2-[[6-[(2,6-dimethylpyrimidin-4-yl)amino]pyrimidin-4-yl]amino]-3-pyridyl]-N-(trideuteriomethyl)methanesulfonamide;

N-[2-[[6-[(2,6-dimethylpyrimidin-4-yl)amino]pyrimidin-4-yl]amino]-5-[1-methyl-5-(trifluoromethyl)-1,2,4-triazol-3-yl]-3-pyridyl]-N-methyl-methanesulfonamide;

N-[2-[[6-[(2,6-dimethylpyrimidin-4-yl)amino]pyrimidin-4-yl]amino]-5-(1-methyl-1,2,4-triazol-3-yl)-3-pyridyl]-N-methyl-methanesulfonamide;

N-[5-(1,5-dimethylpyrazol-4-yl)-2-[[6-[(2,6-dimethylpyrimidin-4-yl)amino]pyrimidin-4-yl]amino]-3-pyridyl]-N-methyl-methanesulfonamide;

N4-(2,6-dimethylpyrimidin-4-yl)-N6-(3-methylsulfonyl-2-pyridyl)pyrimidine-4,6-diamine;

N-[2-[[6-[(2,6-dimethylpyrimidin-4-yl)amino]pyrimidin-4-yl]amino]-5-(1-methylpyrazol-4-yl)-3-pyridyl]-N-methyl-methanesulfonamide;

N-[2-[[6-[(2,6-dimethylpyrimidin-4-yl)amino]pyrimidin-4-yl]amino]-5-(2-methylpyrazol-3-yl)-3-pyridyl]-N-methyl-methanesulfonamide;

N-[2-[[6-[(2,6-dimethylpyrimidin-4-yl)amino]pyrimidin-4-yl]amino]-5-(1-methylimidazol-2-yl)-3-pyridyl]-N-methyl-methanesulfonamide;

N-[5-(2,5-dimethylpyrazol-3-yl)-2-[[6-[(2,6-dimethylpyrimidin-4-yl)amino]pyrimidin-4-yl]amino]-3-pyridyl]-N-methyl-methanesulfonamide;

N-[2-[[6-[(2,6-dimethylpyrimidin-4-yl)amino]pyrimidin-4-yl]amino]-5-(2-methyl-1,2,4-triazol-3-yl)-3-pyridyl]-N-methyl-methanesulfonamide;

N-[2-[[6-[(2,6-dimethylpyrimidin-4-yl)amino]pyrimidin-4-yl]amino]-5-(1-methylpyrazol-3-yl)-3-pyridyl]-N-methyl-methanesulfonamide;

N4-(2,6-dimethylpyrimidin-4-yl)-N6-(3-methoxy-4-methyl-2-pyridyl)pyrimidine-4,6-diamine;

N-[5-(1,5-dimethylpyrazol-4-yl)-2-[[6-[(2,6-dimethylpyrimidin-4-yl)amino]pyrimidin-4-yl]amino]-3-pyridyl]-N-(trideuteriomethyl)methanesulfonamide;

N-[2-[[6-[(2,6-dimethylpyrimidin-4-yl)amino]pyrimidin-4-yl]amino]-5-(4,5-dimethyl-1,2,4-triazol-3-yl)-3-pyridyl]-N-methyl-methanesulfonamide;

N-[5-(1,5-dimethylpyrazol-4-yl)-2-[[6-[(6-methylpyrimidin-4-yl)amino]pyrimidin-4-yl]amino]-3-pyridyl]-N-methyl-methanesulfonamide;

N-[2-[[6-[(2,6-dimethylpyrimidin-4-yl)amino]pyrimidin-4-yl]amino]-5-(4,5-dimethyl-1,2,4-triazol-3-yl)-3-pyridyl]-N-(trideuteriomethyl)methanesulfonamide;

N4-[5-(1,5-dimethylpyrazol-4-yl)-3-methylsulfonyl-2-pyridyl]-N6-(2,6-dimethylpyrimidin-4-yl)pyrimidine-4,6-diamine;

N-[5-(1,5-dimethylpyrazol-4-yl)-2-[[6-[(5-fluoro-2-pyridyl)amino]pyrimidin-4-yl]amino]-3-pyridyl]-N-methyl-methanesulfonamide;

N-[6-[[5-(1,5-dimethylpyrazol-4-yl)-3-[methyl(methylsulfonyl)amino]-2-pyridyl]amino]pyrimidin-4-yl]cyclopropanecarboxamide;

N-[5-(1,5-dimethylimidazol-2-yl)-2-[[6-[(2,6-dimethylpyrimidin-4-yl)amino]pyrimidin-4-yl]amino]-3-pyridyl]-N-methyl-methanesulfonamide;

N6-[5-(1,5-dimethylpyrazol-4-yl)-3-methoxy-4-methyl-2-pyridyl]-N4-(2,6-dimethylpyrimidin-4-yl)pyrimidine-4,6-diamine;

N-[5-(2,3-dimethylimidazol-4-yl)-2-[[6-[(2,6-dimethylpyrimidin-4-yl)amino]pyrimidin-4-yl]amino]-3-pyridyl]-N-methyl-methanesulfonamide;

N6-(2,6-dimethylpyrimidin-4-yl)-N4-[3-methoxy-4-[5-(trifluoromethyl)-1,2,4-oxadiazol-3-yl]-2-pyridyl]pyrimidine-4,6-diamine;

N-[5-(1,5-dimethylpyrazol-4-yl)-2-[[6-[(2,6-dimethylpyrimidin-4-yl)amino]pyrimidin-4-yl]amino]-3-pyridyl]methanesulfonamide;

N6-(3-dimethylphosphoryl-2-pyridyl)-N4-(2,6-dimethylpyrimidin-4-yl)pyrimidine-4,6-diamine;

N6-[5-(1,5-dimethylpyrazol-4-yl)-3-methoxy-2-pyridyl]-N4-(2,6-dimethylpyrimidin-4-yl)pyrimidine-4,6-diamine;

N-[2-[[6-[(2,6-dimethylpyrimidin-4-yl)amino]pyrimidin-4-yl]amino]-5-(1-methylimidazol-4-yl)-3-pyridyl]-N-methyl-methanesulfonamide;

N4-(2,6-dimethylpyrimidin-4-yl)-N6-[4-(1,5-dimethyl-1,2,4-triazol-3-yl)-3-methoxy-2-pyridyl]pyrimidine-4,6-diamine;

N6-[3-dimethylphosphoryl-5-(1,5-dimethylpyrazol-4-yl)-2-pyridyl]-N4-(2,6-dimethylpyrimidin-4-yl)pyrimidine-4,6-diamine;

N4-(2,6-dimethylpyrimidin-4-yl)-N6-(4-methyl-3-methylsulfonyl-2-pyridyl)pyrimidine-4,6-diamine;

N-[6-[[5-(1,5-dimethylpyrazol-4-yl)-3-methylsulfonyl-2-pyridyl]amino]pyrimidin-4-yl]cyclopropanecarboxamide;

N4-[5-(1,5-dimethylpyrazol-4-yl)-3-methylsulfonyl-2-pyridyl]-N6-(5-fluoro-2-pyridyl)pyrimidine-4,6-diamine;

N6-[5-(1,5-dimethylpyrazol-4-yl)-4-methyl-3-methylsulfonyl-2-pyridyl]-N4-(2,6-dimethylpyrimidin-4-yl)pyrimidine-4,6-diamine;

N-[5-(1,5-dimethyl-1,2,4-triazol-3-yl)-2-[[6-[(6-methylpyrimidin-4-yl)amino]pyrimidin-4-yl]amino]-3-pyridyl]-N-methyl-methanesulfonamide;

N-[5-(2,5-dimethyl-1,2,4-triazol-3-yl)-2-[[6-[(6-methylpyrimidin-4-yl)amino]pyrimidin-4-yl]amino]-3-pyridyl]-N-methyl-methanesulfonamide;

N6-(6-methyl-3-methylsulfonyl-2-pyridyl)-N4-(6-methylpyrimidin-4-yl)pyrimidine-4,6-diamine;

N-[2-[[6-[(2,6-dimethylpyrimidin-4-yl)amino]pyrimidin-4-yl]amino]-5-(oxan-4-yl)pyridin-3-yl]-N-methylmethanesulfonamide;

N-[5-(1,5-dimethylpyrazol-4-yl)-2-[[6-[(6-methylpyridazin-3-yl)amino]pyrimidin-4-yl]amino]-3-pyridyl]-N-methyl-methanesulfonamide;

4-N-(2,6-dimethylpyrimidin-4-yl)-6-N-[3-methoxy-4-(5-methyl-1,2,4-oxadiazol-3-yl)pyridin-2-yl]pyrimidine-4,6-diamine;

(1R,2R)-N-[6-[[5-(1,5-dimethylpyrazol-4-yl)-3-[methyl(methylsulfonyl)amino]-2-pyridyl]amino]pyrimidin-4-yl]-2-fluoro-cyclopropanecarboxamide;

N-[6-[[3-dimethylphosphoryl-5-(1,5-dimethylpyrazol-4-yl)-2-pyridyl]amino]pyrimidin-4-yl]cyclopropanecarboxamide;

N-methyl-N-[6-methyl-5-(1-methylpyrazol-4-yl)-2-[[6-[(6-methylpyrimidin-4-yl)amino]pyrimidin-4-yl]amino]-3-pyridyl]methanesulfonamide;

N-[5-(1,5-dimethylpyrazol-4-yl)-6-methyl-2-[[6-[(6-methylpyrimidin-4-yl)amino]pyrimidin-4-yl]amino]-3-pyridyl]-N-methyl-methanesulfonamide;

N4-(2,6-dimethylpyrimidin-4-yl)-N6-[3-(N,S-dimethylsulfonimidoyl)-2-pyridyl]pyrimidine-4,6-diamine;

N4-[3-methoxy-6-methyl-5-(1-methylpyrazol-4-yl)-2-pyridyl]-N6-(6-methylpyrimidin-4-yl)pyrimidine-4,6-diamine;

N4-(2,6-dimethylpyrimidin-4-yl)-N6-(3-methoxy-5-tetrahydropyran-2-yl-2-pyridyl)pyrimidine-4,6-diamine;

N-[2-[[6-[(2,6-dimethylpyrimidin-4-yl)amino]pyrimidin-4-yl]amino]-5-tetrahydropyran-2-yl-3-pyridyl]-N-methyl-methanesulfonamide;

N6-[5-(1,5-dimethylpyrazol-4-yl)-3-(N,S-dimethylsulfonimidoyl)-2-pyridyl]-N4-(2,6-dimethylpyrimidin-4-yl)pyrimidine-4,6-diamine;

N6-(2,6-dimethylpyrimidin-4-yl)-N4-[3-methoxy-4-[1-(2-methoxyethyl)-1,2,4-triazol-3-yl]-2-pyridyl]pyrimidine-4,6-diamine;

N-[2-[[6-[(2,6-dimethylpyrimidin-4-yl)amino]pyrimidin-4-yl]amino]-5-(4-methyl-1,3-oxazol-5-yl)pyridin-3-yl]-N-methylmethanesulfonamide;

4-N-(5-cyclopropyl-3-dimethylphosphorylpyridin-2-yl)-6-N-(2,6-dimethylpyrimidin-4-yl)pyrimidine-4,6-diamine;

2-[2-[[6-[(2,6-dimethylpyrimidin-4-yl)amino]pyrimidin-4-yl]amino]pyridin-3-yl]propan-2-ol;

N4-[5-(1,5-dimethylpyrazol-4-yl)-3-methylsulfinyl-2-pyridyl]-N6-(2,6-dimethylpyrimidin-4-yl)pyrimidine-4,6-diamine;

N4-(5-fluoro-6-methyl-2-pyridyl)-N6-[3-methoxy-4-(1-methyl-1,2,4-triazol-3-yl)-2-pyridyl]pyrimidine-4,6-diamine;

6-N-(2,6-dimethylpyrimidin-4-yl)-4-N-[3-methylsulfonyl-5-(oxan-2-yl)pyridin-2-yl]pyrimidine-4,6-diamine;

N6-[5-(1,5-dimethylpyrazol-4-yl)-3-methoxy-6-methyl-2-pyridyl]-N4-(6-methylpyrimidin-4-yl)pyrimidine-4,6-diamine;

or a pharmaceutically acceptable salt, or solvate, or N-oxide, or tautomer, or stereoisomer, or isotopically labelled derivative thereof.

GENERAL SYNTHETIC PROCEDURES

The compounds of the invention can be prepared using the methods and procedures described herein, or using similar methods and procedures. It will be appreciated that where typical or preferred process conditions (i.e., reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Starting compounds are commercially available or may be obtained following the conventional synthetic methods already known in the art.

Additionally, as will be apparent to those skilled in the art, conventional protecting groups may be necessary to prevent certain functional groups from undergoing undesired reactions. The choice of a suitable protecting group for a particular functional group, as well as suitable conditions for protection and deprotection, are well known in the art. For example, numerous protecting groups, and their introduction and removal are described in T. W. Greene and G. M.

Wuts, Protecting Groups in Organic Synthesis, Third Edition, Wiley, New York, 1999, and references cited therein.

Processes for preparing compounds of the invention are provided as further embodiments of the invention and are illustrated by the procedures below.

The methods presented below are not meant to limit the synthetic pathways in order to obtain the compounds of the invention. All examples of the present application are prepared by the methods disclosed herein.

Method A

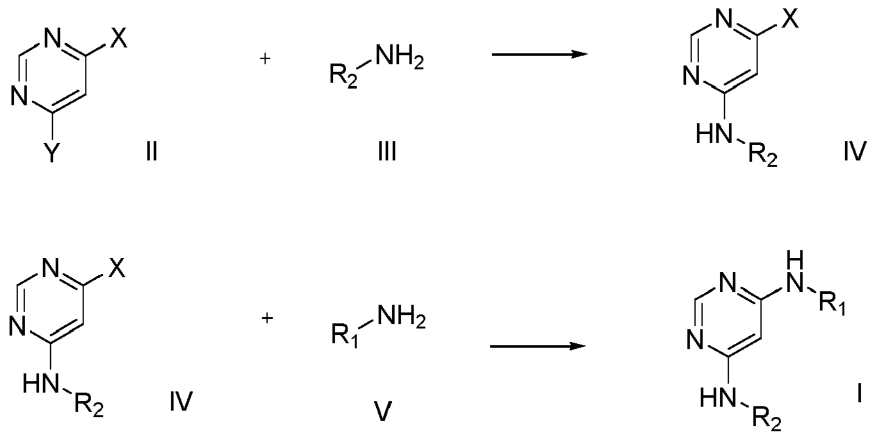

II III IV

IV V I

Scheme 1

Scheme 1 depicts the synthesis of compounds I from haloderivatives IV (X=Cl, Br, I) by reaction with amines V. Intermediates IV may be obtained from halopyrimidines II (X,Y = Cl, Br, I) by reaction with amines or amides III. Both transformations can be carried on either in the presence of derivatives of transition metals like palladium which catalyse the process or also without metal catalyst.

In the case of $R_2$ being a -C(=O)$R_6$ group, intermediates IV can alternatively be prepared from amino halo pyrimidines VI (X=Cl, Br, I) by acylation with derivatives VII according to Scherme 2. This step requires the previous activation of carboxylic acids VII.

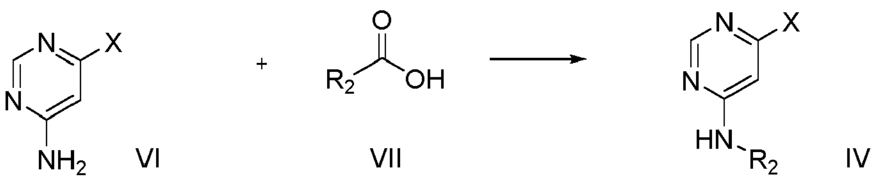

VI VII IV

Scheme 2

Method B

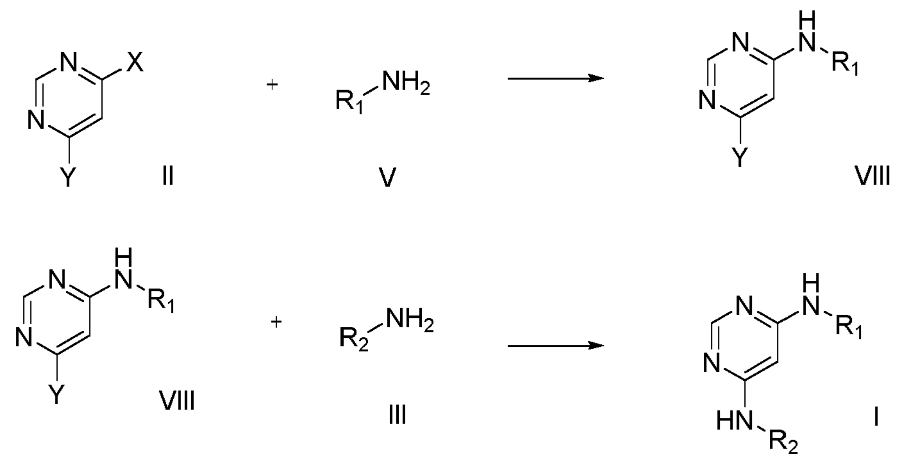

II V VIII

VIII III I

Scheme 3

The overall process shown in Scheme 1 may reverse the synthetic steps as depicted in Scheme 3. According to this method compounds I may be prepared from intermediate haloderivatives VIII (Y=Cl, Br, I) by reaction with amines or amides III.

**EXAMPLES**

The syntheses of the compounds of the invention are illustrated by the following Examples (1 to 176) including Intermediates which do not limit the scope of the invention in any way.

**General**

Reagents, starting materials, and solvents were purchased from commercial suppliers and used as received. Commercial intermediates are referred to in the experimental section by their IUPAC name. Ether refers to diethyl ether, unless otherwise specified. Concentration or evaporation refer to evaporation under vacuum using a Büchi rotatory evaporator.

Reaction products were purified, when necessary, by flash chromatography on silica gel (40-63 μm) with the solvent system indicated. Purifications in reverse phase were made in a Biotage Isolera® automated purification system equipped with a C18 column and using a gradient, unless otherwise stated, of water-acetonitrile/MeOH (1:1) (0.1% v/v ammonium formate both phases) from 0% to 100% acetonitrile/MeOH (1:1) in 40 column volumes. The conditions "formic acid buffer" refer to the use of 0.1% v/v formic acid in both phases. The appropriate fractions were collected and the solvents evaporated under reduced pressure and/or liofilized.

Purifications in reverse phase were also made in a Biotage SP1® automated purification system equipped with a C18 column and using a gradient of, unless otherwise stated, water-acetonitrile/MeOH (1:1) (0.1% v/v ammonium formate both phases) from 0% to 100% acetonitrile/MeOH (1:1) in 80 column volumes. The conditions "formic acid buffer" refer to the use of 0.1% v/v formic acid in both phases. The appropriate fractions were collected and freeze dried.

Preparative HPLC-MS were performed on a Waters instrument equipped with a 2767 injector/collector, a 2525 binary gradient pump, a 2996 PDA detector, a 515 pump as a make-up pump and a ZQ4000 Mass spectrometer detector or on a Agilent 1200 Series coupled to an Agilent 6120 Mass spectrometer detector. Both systems were equipped with a Symmetry Prep C18 (19 x 300 mm, 7 μm) column or a XBridge Prep C18 (19 x 100 mm, 5 μm) column. The mobile phase was formic acid (0.4 mL), ammonia (0.1 mL), methanol (500 mL) and acetonitrile (500 mL) (B) and formic acid (0.5 mL), ammonia (0.125 mL) and water (1000 mL) (A), the specific gradients used are specified in each particular case. The flow rate was 20 ml/min.

The UPLC chromatographic separations were obtained using a Waters Acquity UPLC system coupled to a SQD mass spectrometer detector. The system was equipped with an ACQUITY UPLC BEH C-18 (2.1x50 mm, 1.7 mm) column. The mobile phase was formic acid (0.4 mL), ammonia (0.1 mL), methanol (500 mL) and acetonitrile (500 mL) (B) and formic acid (0.5 mL), ammonia (0.125 mL) and water (1000 mL) (A). A gradient between 0 to 95% of B was used. The run time was 3 or 6 minutes. The injection volume was 0.5 microliter. Chromatograms were processed at 210 nM or 254 nM. Mass spectra of the chromatograms were acquired using positive and negative electrospray ionization.

The HPLC chromatographic separations were obtained using a Waters Alliance HT HPLC system coupled to a ZQ mass spectrometer detector. The system was equipped with an YMC-Pack ODS-AQ C-18 (4-6×50 mm, 3μM)

column. The mobile phase was formic acid (1.0 mL) and acetonitrile (1000 mL) (B), formic acid (1.0 mL) and water (1000 mL) (A). A gradient between 5 to 100% of B was used. The run time was 3.5 minutes. The injection volume was 5 μL. Chromatograms were processed from 210 nM to 400 nM. Mass spectra of the chromatograms were acquired using positive electrospray ionization.

1H Nuclear Magnetic Resonance Spectra were recorded on a Varian Mercury plus operating at a frequency of 400MHz or a Varian VNMRS operating at 600MHz and equipped with a cold probe for the 1H spectra. Samples were dissolved in the specified deuterated solvent. Tetramethylsilane was used as reference.

**Abbreviations**:

$Pd_2(dba)_3$: tris(dibenzylidineacetone)dipalladium(0)

Xantphos: (9,9-dimethyl-9H-xanthene-4,5-diyl)bis(diphenylphosphine)

DIPEA: Diisopropylethylamine

DMF: N,N-dimethylformamide

DMSO: dimethylsulfoxyde

THF: Tetrahydrofuran

AcOEt: Ethyl acetate

DCM: Dichloromethane

IPA: Isopropyl alcohol

HATU: 1-(bis(dimethylamino)methylene)-1H-[1,2,3]triazolo[4,5-b]pyridine-1-ium 3-oxide hexafluorophosphate(V)

**Example 1: N-[6-[2-methoxy-3-(1-methyl-1,2,4-triazol-3-yl)anilino]pyrimidin-4-yl]cyclopropanecarboxamide**

**Step 1-1: Synthesis of 2-methoxy-3-(4,4,5,5-tetramethyl-1,3,2-dioxaborolan-2-yl)aniline**

To a solution of 3-bromo-2-methoxy-aniline (150 mg, 0.74 mmol) in 4 mL of anh. dioxane was added, under Ar bubbling, 4,4,5,5-tetramethyl-2-(4,4,5,5-tetramethyl-1,3,2-dioxaborolan-2-yl)-1,3,2-dioxaborolane (283 mg, 1.11 mmol), potassium acetate (218 mg, 2.22 mmol) and [1,1'-Bis(diphenylphosphino)ferrocene]dichloropalladium(II) (1:1) (30 mg, 0.37 mmol). The mixture was heated at 100 °C overnight. Excess water was added, the mixture extracted with AcOEt (× 3) and the combined organic fractions were washed with water (× 3) and brine; dried over anh. MgSO4 and filtered. The residue was purified via flash chromatography in Hx/AcOEt to have a brown solid (100 mg, 54 %)

HPLC-MS: tr = 2.50 min. (99 %). LRMS (m/z): 250 (M+1)

$^1$H NMR (400 MHz, Chloroform-d) δ 1.35 (s, 12H), 3.84 (s, 3H), 6.93-7.02 (m, 2H), 7.20 (dd, J = 7.1, 2.0 Hz, 1H).

**Step 1-2: Synthesis of 2-methoxy-3-(1-methyl-1,2,4-triazol-3-yl)aniline**

3-bromo-1-methyl-1,2,4-triazole (170mg, 1.04mmol), 2-methoxy-3-(4,4,5,5-tetramethyl-1,3,2-dioxaborolan-2-yl)aniline (Step 1-1; 313 mg, 1.25mmol), Pd(dppf)Cl2 (38mg, 0.051mmol) and 2M potassium phosphate (1.6mL, 3.2mmol) were heated in degassed dioxane (4mL) at 100°C for 2 hr. Water and AcOEt were added and the aqueaos phase extracted with AcOEt (x2). The combined organic phases were dried over MgSO4, filtered and concentrated. The crude was purified by flash chromatography (Biotage Telos12g) using dichloromethane/MeOH (up to 5%) (220nm) to give the title compound (124mg, 58%).

HPLC-MS tr = 1.28min (98%). LRMS (m/z): 205 (M+1)

$^1$H NMR (400 MHz, Chloroform-d) δ 3.79 (s, 3H), 4.00 (s, 3H), 7.02 (t, J = 7.8 Hz, 1H), 7.40 (d, J = 7.5 Hz, 1H), 8.16 (s, 1H).

### Step 1-3: Synthesis of 6-chloro-N-[2-methoxy-3-(1-methyl-1,2,4-triazol-3-yl)phenyl]pyrimidin-4-amine

A mixture of 4,6-dichloropyrimidine (100 mg; 0.67 mmol), 2-methoxy-3-(1-methyl-1,2,4-triazol-3-yl)aniline (Step 4; 136 mg, 0.66 mmol) and DIPEA (234 uL, 1.34 mmol) in 2-propanol (500 uL) was stirred at 50°C overnight. After adding 20 additional mg (0.13 mmol) of 4,6-dichloropyrimidine and 60 additional uL (0.34 mmol) of DIPEA the stirring was prosecuted at 80°C for 5 hr. The mixture is concentrated and directly purified by flash chromatography using DCM/MeOH (0-5%) to give the title compound (66 mg, 29%) as a white solid.

uPLC-MS tr = 1.27min (94%). LRMS (m/z): 317-319 (M+1; Cl)

$^1$H NMR (400 MHz, Chloroform-d) δ 3.77 (s, 3H), 4.02 (s, 3H), 6.80 (s, 1H), 7.31-7.38 (m, 1H), 7.78 (dd, J = 7.9, 1.6 Hz, 1H), 7.99 (d, J = 7.1 Hz, 1H), 8.10 (d, J = 16.5 Hz, 1H), 8.55 (d, J = 0.7 Hz, 1H).

### Step 1-4: Synthesis of N-[6-[2-methoxy-3-(1-methyl-1,2,4-triazol-3-yl)anilino]pyrimidin-4-yl]cyclopropanecarboxamide

A mixture of 6-chloro-N-[2-methoxy-3-(1-methyl-1,2,4-triazol-3-yl)phenyl]pyrimidin-4-amine (Step 1-3; 65 mg; 0.20 mmol), cyclopropanecarboxamide (23 mg; 0.27 mmol), Pd2dba3 (19 mg; 0.020 mmol), xantphos (23 mg; 0.04 mmol), cesium carbonate (200 mg; 0.61 mmol) and 1.5 ml dioxane is heated to 100°C under argon atmosphere in a Schlenck flask. After 4.5 hr no starting material is observed. Excess water and AcOEt are added and the aqueous layer is further extracted with additional AcOEt. The combined extracts are washed with brine, dried and concentrated. The residue (140 mg) is purified through a silica cartridge eluting with DCM/MeOH from 0 to 5% in 40 column volumes to give 41 mg (55%) of title compound as a yellowish solid.

uPLC-MS tr =1.19 min (99%). LRMS (m/z): 366 (M+1)

$^1$H NMR (400 MHz, CDCl3) δ 8.47 (s, 1H), 8.23 (d, J = 8.1 Hz, 1H), 8.12 (d, J = 5.1 Hz, 2H), 7.68 (d, J = 7.8 Hz, 2H), 7.41 (s, 1H), 4.01 (s, 3H), 3.77 (s, 3H), 1.53 (d, J = 4.6 Hz, 1H), 1.13 (dt, J = 8.0, 4.1 Hz, 2H), 0.94 (dq, J = 7.5, 4.1 Hz, 2H)

## Example 2: N-[6-(2-methoxyanilino)pyrimidin-4-yl]cyclopropanecarboxamide

### Step 2-1: Synthesis of 6-chloro-N-(2-methoxyphenyl)pyrimidin-4-amine

A mixture of 4,6-dichloropyrimidine (100 mg; 0.67 mmol), 2-methoxyaniline (0.08 mL; 0.71 mmol) and aqueous 37% HCl solution (0.1 mL; 1 mmol) in 1 mL of IPA are stirred at reflux temperature under Ar atmosphere for 1 hr. Excess water and DCM are added and the aqueous phase further extracted with DCM. The organic extracts were dried, concentrated and purified through a CombiFlash column eluting with DCM/MeOH up to 5% to afford 100 mg (63%) of title compound,

uPLC-MS tr = 2.58 min (98%). LRMS (m/z): 236 (M+1)

### Step 2-2: Synthesis of N-[6-(2-methoxyanilino) pyrimidin-4-yl] cyclopropanecarboxamide

A mixture of 6-chloro-N-(2-methoxyphenyl)pyrimidin-4-amine (Step 2-1; 100 mg; 0.424 mmol), cyclopropanecarboxamide (47 mg; 0.552 mmol), Pd2dba3 (39 mg; 0.04 mmol), xantphos (50 mg; 0.08 mmol), cesium carbonate (415 mg; 1.27 mmol) and 2.3 ml dioxane is heated to 100°C under argon atmosphere for 4.5 hr. Excess water and AcOEt are added and the aqueous layer is further extracted with additional AcOEt. The combined extracts are washed with brine, dried and concentrated. The residue is purified through a silica cartridge eluting with DCM/MeOH from 0 to 10% to give 51 mg (42%) of title compound.

uPLC-MS tr = 2.17 min (97%). LRMS (m/z): 285 (M+1)

$^1$H NMR (400 MHz, d6-DMSO) δ 0.70-0.82 (m 4H), 1.25-1.32 (m 1H), 3.00 (s 3H), 6.08-6.13 (m 1H), 6.23-6.35 (m 2H), 6.60 (s 1H), 6.759 (d, J = 8 Hz, 1H), 7.45 (s 1H), 7.90 (s 1H), 9.90 (s, 1H).

## Example 3: N-[6-(2-methoxy-3-methyl-anilino)pyrimidin-4-yl]cyclopropanecarboxamide

### Step 3-1: Synthesis of 6-chloro-N-(2-methoxy-3-methyl-phenyl)pyrimidin-4-amine

A mixture of 4,6-dichloropyrimidine (100 mg; 0.67 mmol), 2-methoxy-3-methylaniline (93 mg; 0.67 mmol) and aqueous 37% HCl solution (0.1 mL; 1 mmol) in 1 mL of IPA are stirred at reflux temperature under Ar atmosphere for 1 hr. Excess water and DCM are added and the aqueous phase further extracted with DCM. The organic extracts were dried, concentrated and purified through a CombiFlash column eluting with DCM/MeOH up to 5% to afford 84 mg (50 %) of title compound.

HPLC-MS tr = 2.75 min (90%). LRMS (m/z): 250 (M+1)

$^1$H NMR (400 MHz, d6-DMSO) δ 2.25 (s, 3H), 3.67 (s, 3H), 6.85 (m 1H), 7.01-7.04 (cs 2H), 7.68-7.70 (m 1H), 8.40 (s 1H), 9.32 (s 1H).

**Step 3-2: Synthesis of N-[6-(2-methoxy-3-methyl-anilino)pyrimidin-4-yl] cyclopropanecarboxamide**

A mixture of 6-chloro-N-(2-methoxy-3-methyl-phenyl)pyrimidin-4-amine (Step 3-1; 80 mg; 0.32 mmol), cyclopropanecarboxamide (36 mg; 0.42 mmol), Pd2dba3 (30 mg; 0.03 mmol), xantphos (37 mg; 0.06 mmol), cesium carbonate (315 mg; 0.96 mmol) and 2 ml dioxane is heated to 100°C under argon atmosphere for 4.5 hr. Excess water and AcOEt are added and the aqueous layer is further extracted with additional AcOEt. The combined extracts are washed with brine, dried and concentrated. The residue is purified through a silica cartridge eluting with DCM/MeOH from 0 to 10% to give 43 mg (45%) of title compound.

uPLC-MS tr = 2.38 min (97%). LRMS (m/z): 299 (M+1)

$^1$H NMR (400 MHz, CDCl3) δ 0.75-0.83 (m 4H), 1.91-2.02 (m 1H), 2.23 (s 3H), 3.59 (s 3H), 6.91-7.01 (m 2H), 6.98 (s 1H), 7.54-7.58 (m 1H), 8.29 (s 1H), 8.83 (s 1H), 10.70 (s 1H).

**Example 4: N6-(2,6-dimethylpyrimidin-4-yl)-N4-[2-methoxy-3-(1-methyl-1,2,4-triazol-3-yl)phenyl]pyrimidine-4,6-diamine**

**Step 4-1: Synthesis of N6-(2,6-dimethylpyrimidin-4-yl)-N4-[2-methoxy-3-(1-methyl-1,2,4-triazol-3-yl)phenyl]pyrimidine-4,6-diamine**

A mixture of 6-chloro-N-[2-methoxy-3-(1-methyl-1,2,4-triazol-3-yl)phenyl]pyrimidin-4-amine (Step 1-3; 70 mg; 0.22 mmol), 2,6-dimethylpyrimidin-4-amine (35 mg; 0.28 mmol), Pd2dba3 (24 mg; 0.026 mmol), xantphos (30 mg; 0.05 mmol), cesium carbonate (220 mg; 0.67 mmol) and 2 ml dioxane is heated to 100°C under argon atmosphere overnight and stirred at room temperature for 2 days. No starting material is observed. Excess water and AcOEt are added and the aqueous layer is further extracted with additional AcOEt. The combined extracts are washed with brine, dried and concentrated. The residue is purified through a silica cartridge eluting with DCM/MeOH from 0 to 5% to give 50 mg (56%) of title compound.

uPLC-MS (30 min) tr =14.4 min (99%). LRMS (m/z): 404 (M+1)

$^1$H NMR (400 MHz, CDCl3) δ 2.26 (s 3H), 2.35 (s 3H), 3.65 (s 3H), 3.92 (s 3H), 7.14-719 (m 2H), 7.36 (s 1H), 7.55-7.58 (m 1H), 7.72-7.75 (m 1H), 8.30 (s 1H), 8.51 (s 1H), 8.87 (s 1H), 10.01 (s 1H).

**Example 5: N6-(2,6-dimethylpyrimidin-4-yl)-N4-(3-methoxyphenyl)pyrimidine-4,6-diamine**

**Step 5-1: Synthesis of 6-chloro-N-(3-methoxyphenyl)pyrimidin-4-amine**

A mixture of 4,6-dichloropyrimidine (100 mg; 0.67 mmol), 3-methoxyaniline (75 uL; 0.67 mmol) and aqueous 37% HCl solution (0.1 mL; 1 mmol) in 1 mL of IPA are stirred at reflux temperature under Ar atmosphere for 1 hr. Excess water and AcOEt are added and the aqueous phase further extracted with AcOEt. The organic extracts were dried and concentrated to afford 146 mg (93 %) of title compound pure enough to be used in the next step.

HPLC-MS (9 min) tr = 5.95 min (93%). LRMS (m/z): 236 (M+1)

$^1$H NMR (400 MHz, d6-DMSO) δ 3.75 (s, 3H), 6.65 (d J=7.5 Hz, 1H), 6.80 (s 1H), 7.16 (d J = 7.8 Hz, 1H), 7.25-7.33 (m 2H), 8.49 (s 1H), 9.8 (s 1H).

**Step 5-2: Synthesis of N6-(2,6-dimethylpyrimidin-4-yl)-N4-(3-methoxyphenyl) pyrimidine-4,6-diamine**

A mixture of 6-chloro-N-(3-methoxyphenyl)pyrimidin-4-amine (Step 5-3; 50 mg; 0.21 mmol), 2,6-dimethylpyrimidin-4-amine (35 mg; 0.28 mmol), Pd2dba3 (20 mg; 0.022 mmol), xantphos (25 mg; 0.043 mmol), cesium carbonate (210 mg; 0.64 mmol) and 1 ml dioxane is heated to 100°C under argon atmosphere for 4 hr. No starting material is observed. Excess water and AcOEt are added and the aqueous layer is further extracted with additional AcOEt. The combined extracts are washed with brine, dried and concentrated. The residue is purified through a silica cartridge eluting with DCM/MeOH from 0 to 5% to give 43 mg (62%) of title compound.

uPLC-MS (30 min) tr =16.5 min (99%). LRMS (m/z): 324 (M+1)

$^1$H NMR (400 MHz, d6-DMSO) δ 2.31 (s 3H), 3.75 (s 3H), 7.12-7.25 (m, 4H), 7.40 (s 1H), 8.37 (s 1H), 9.41 (s 1H), 10.11 (s 1H).

**Example 6: N6-(2,6-dimethylpyrimidin-4-yl)-N4-(2-methoxy-3-methyl-phenyl)pyrimidine-4,6-diamine**

**Step 6-1: Synthesis of N-(6-chloropyrimidin-4-yl)-2,6-dimethyl-pyrimidin-4-amine**

A mixture of 4,6-dichloropyrimidine (1200 mg; 8.055 mmol), 2,6-dimethylpyrimidin-4-amine (992 mg; 8.055 mmol), Pd2dba3 (737 mg; 0.805 mmol), xantphos (932 mg; 1.611 mmol), cesium carbonate (7.87 g; 24.15 mmol) and 15 ml dioxane is heated to 90°C under argon atmosphere. After 1 hr no starting material is observed. Excess water anc AcOEt are added and the aqueous layer is further extracted twice with additional AcOEt. The combined extracts are washed with brine, dried and concentrated. The residue is stirred with 5 mL of DCM for 15 min and the solid filtered and washed thoroughly with a mixture of hexane/DCM 3:1 to give 1260 mg (66%) of pure title compound.

uPLC-MS tr = 0.79 min (100%). LRMS (m/z): 236/238 (M+1, Cl)

$^1$H NMR (400 MHz, Chloroform-d) δ 8.63 (s, 1H), 7.92 (s, 1H), 7.43 (s, 1H), 7.03 (s, 1H), 2.65 (s, 3H), 2.48 (s, 3H).

**Step 6-2: Synthesis of N6-(2,6-dimethylpyrimidin-4-yl)-N4-(2-methoxy-3-methyl-phenyl)pyrimidine-4,6-diamine**

A mixture of N-(6-chloropyrimidin-4-yl)-2,6-dimethyl-pyrimidin-4-amine (Step 6-1; 80 mg, 0.34 mmol), 2-methoxy-3-methyl-aniline (50 uL, 0.48 mmol) and DIPEA (100 uL, 0.57 mmol) in 1 ml of IPA is stirred at 80°C for 64 hr. The residue after concentration was purified through flash chromatography eluting with DCM/MeOH from 0 to 5% to give 48 mg (42%) of title compound. uPLC-MS (30 min) tr =17.2 min (98%). LRMS (m/z): 337 (M+1)

$^1$H NMR (400 MHz, d6-DMSO) δ 2.15 (s 1H), 2.30 (s 3H), 2.40 (s 3H), 3.90 (s 3H), 6.90-7.03 (m, 2H), 7.19 (s, 1H), 7.23 (s, 1H), 7.49 (d, J=7.8 Hz, 1H), 8.28 (s 1H), 8.93 (s 1H), 10.04 (s 1H).

**Example 7: N6-(2,6-dimethylpyrimidin-4-yl)-N4-(3-fluoro-2-methoxy-phenyl) pyrimidine-4,6-diamine**

**Step 7-1: Synthesis of N6-(2,6-dimethylpyrimidin-4-yl)-N4-(3-fluoro-2-methoxy-phenyl) pyrimidine-4,6-diamine**

A mixture of N-(6-chloropyrimidin-4-yl)-2,6-dimethyl-pyrimidin-4-amine (Step 6-1; 50 mg, 0.21 mmol), 3-fluoro-2-methoxy-aniline (29 mg, 0.20 mmol), Pd2dba3 (20 mg; 0.02 mmol), xantphos (25 mg; 0.043 mmol), cesium carbonate (0.21 g; 0.64 mmol) and 2 ml dioxane is heated to 90°C under argon atmosphere overnight. Excess water and AcOEt are added and the aqueous layer is further extracted twice with additional AcOEt. The combined extracts are washed with brine, dried and concentrated. The residue after concentration is purified through flash chromatography eluting with DCM/MeOH from 0 to 5% to give 14.7 mg (21%) of title compound.

uPLC-MS (30 min) tr =17.18 min (100%). LRMS (m/z): 341 (M+1)

$^1$H NMR (400 MHz, d6-DMSO) δ 2.29 (s 3H), 2.41 (s 3H), 3.80 (s 3H), 6.95-7.09 (m, 2H), 7.21 (s, 1H), 7.34 (s, 1H), 7.57 (d, J=7.8 Hz, 1H), 8.31 (s 1H), 8.95 (s 1H), 10.09 (s 1H).

**Example 8: N4-(2,6-dimethylpyrimidin-4-yl)-N6-(3-methoxy-6-methyl-2-pyridyl)pyrimidine-4,6-diamine**

**Step 8-1: Synthesis of N4-(2,6-dimethylpyrimidin-4-yl)-N6-(3-methoxy-6-methyl-2-pyridyl)pyrimidine-4,6-diamine**

A mixture of N-(6-chloropyrimidin-4-yl)-2,6-dimethyl-pyrimidin-4-amine (Step 6-1; 122 mg, 0.52 mmol), 3-methoxy-6-methyl-pyridin-2-amine (60 mg, 0.43 mmol), Pd2dba3 (40 mg; 0.04 mmol), xantphos (50 mg; 0.086 mmol), cesium carbonate (0.42 g; 1.30 mmol) and 3.5 ml dioxane is heated to 100°C in a microwave oven under argon atmosphere for 3 hr. Excess water and AcOEt are added and the aqueous layer is further extracted twice with additional AcOEt. The combined extracts are washed with brine, dried and concentrated. The residue after concentration is purified through flash chromatography eluting with DCM/MeOH from 0 to 2% to give 66 mg (45%) of title compound as a yellowish solid.

uPLC-MS tr = 0.88 min (99.3%). LRMS (m/z): 338 (M+1)

1H NMR (400 MHz, Chloroform-d) δ 8.50 (d, J = 0.8 Hz, 1H), 8.35 (d, J = 1.0 Hz, 1H), 7.96 (s, 1H), 7.78 (s, 1H), 7.43 (s, 1H), 7.00 (d, J = 8.0 Hz, 1H), 6.73 (d, J = 8.0 Hz, 1H), 3.88 (s, 3H), 2.61 (s, 3H), 2.50 (s, 3H), 2.48 (s, 3H).

**Example 9: N4-(2-methoxy-3-methyl-phenyl)-N6-(1-tetrahydropyran-4-ylpyrazol-4-yl)pyrimidine-4,6-diamine**

**Step 9-1: Synthesis of 6-chloro-N-(1-tetrahydropyran-4-ylpyrazol-4-yl)pyrimidin-4-amine**

By starting with 4,6-dichloropyrimidine and 1-tetrahydropyran-4-ylpyrazol-4-amine and by following a similar procedure to that leading to Intermediate of step 6-2 the title compound is obtained in 53% yield

uPLC-MS (9 min) tr = 4.93 min (100%). LRMS (m/z): 280/282 (M+1; Cl)

1H NMR (400 MHz, d6-DMSO) δ 8.43 (s, 1H), 8.05 (s, 1H), 7.53 (s, 1H), 6.60 (bs, 1H), 4.26 - 4.34 (m, 1H), 3.49 (td, J = 11.7, 2.9 Hz, 4H), 1.98 (ddd, J = 24.4, 11.8, 4.2 Hz, 4H).

**Step 9-2: Synthesis of N4-(2-methoxy-3-methyl-phenyl)-N6-(1-tetrahydropyran-4-ylpyrazol-4-yl)pyrimidine-4,6-diamine**

A mixture of 6-chloro-N-(1-tetrahydropyran-4-ylpyrazol-4-yl)pyrimidin-4-amine (Step 9-1; 60 mg, 0.21 mmol), 2-methoxy-3-methyl-aniline (29 mg, 0.21 mmol), Pd2dba3 (20 mg; 0.021 mmol), xantphos (25 mg; 0.043 mmol), cesium carbonate (0.21 g; 0.64 mmol) and 1 ml dioxane is heated to 100°C overnight under argon atmosphere. Excess water and AcOEt are added and the aqueous layer is further extracted twice with additional AcOEt. The combined extracts are washed with brine, dried and concentrated. The residue after concentration is purified through flash chromatography eluting with DCM/MeOH from 0 to 5% to give 12 mg (13%) of title compound as a yellowish solid.

uPLC-MS tr = 1.07 min (97%). LRMS (m/z): 381 (M+1)

$^1$H NMR (400 MHz, Chloroform-d) δ 8.30 (d, J = 0.8 Hz, 1H), 7.59 (d, J = 10.1 Hz, 1H), 7.55-7.45 (m, 3H), 6.97 (d, J = 7.8 Hz, 1H), 6.86 (s, 1H), 6.19 (s, 1H), 5.94 (d, J = 1.0 Hz, 1H), 4.32 (ddd, J = 15.6, 10.2, 4.8 Hz, 1H), 3.71 (s, 3H), 3.55 (td, J = 11.7, 2.9 Hz, 2H), 3.39-3.30 (m, 2H), 2.31 (s, 3H), 2.06 (ddd, J = 24.4, 11.8, 4.2 Hz, 2H), 1.49 - 1.39 (m, 2H).

**Example 10: N6-(2,6-dimethylpyrimidin-4-yl)-N4-[2-(trifluoromethoxy)phenyl] pyrimidine-4,6-diamine**

**Step 10-1: Synthesis of N6-(2,6-dimethylpyrimidin-4-yl)-N4-[2-(trifluoromethoxy)phenyl] pyrimidine-4,6-diamine**

A mixture of N-(6-chloropyrimidin-4-yl)-2,6-dimethyl-pyrimidin-4-amine (Step 6-1; 50 mg, 0.21 mmol), 2-(trifluoromethoxy)aniline (48 mg, 0.27 mmol), Pd2dba3 (20 mg; 0.021 mmol), xantphos (24 mg; 0.041 mmol), cesium carbonate (0.207 g; 0.635 mmol) and 2.5 ml dioxane is heated to 100°C for 1.5 hr in a microwave oven under argon atmosphere. Excess water and AcOEt are added and the aqueous layer is further extracted twice with additional AcOEt. The combined extracts are washed with brine, dried and concentrated. The residue after concentration is purified through flash chromatography eluting with DCM/MeOH from 0 to 2% to give 19 mg (24%) of title compound as a white solid.

uPLC-MS tr = 1.18 min (99%). LRMS (m/z): 377 (M+1)

$^1$H NMR (400 MHz, Chloroform-d) δ 8.44 (d, J = 0.8 Hz, 1H), 7.93 (dd, J = 8.5, 1.4 Hz, 1H), 7.61 (s, 1H), 7.41 (s, 1H), 7.39 - 7.32 (m, 2H), 7.23 - 7.14 (m, 1H), 6.93 (s, 1H), 6.83 (s, 1H), 2.56 (s, 3H), 2.44 (s, 3H).

**Example 11: N6-(2,6-dimethylpyrimidin-4-yl)-N4-(2-methylsulfonylphenyl)pyrimidine-4,6-diamine**

**Step 11-1: Synthesis of N6-(2,6-dimethylpyrimidin-4-yl)-N4-(2-methylsulfonylphenyl)pyrimidine-4,6-diamine**

A mixture of N-(6-chloropyrimidin-4-yl)-2,6-dimethyl-pyrimidin-4-amine (Step 6-1; 100 mg, 0.42 mmol), 2-methylsulfonylaniline (95 mg, 0.55 mmol), Pd2dba3 (39 mg; 0.042 mmol), xantphos (49 mg; 0.085 mmol), cesium carbonate (0.414 g; 1.271 mmol) and 3.5 ml dioxane is heated to 100°C for 1.5 hr in a microwave oven under argon atmosphere. Excess water and AcOEt are added and the aqueous layer is further extracted twice with additional AcOEt. The combined extracts are washed with brine, dried and concentrated. The residue after concentration is purified through flash chromatography eluting with DCM/MeOH from 0 to 2% to give 28 mg (18%) of title compound as a white solid.

uPLC-MS tr = 1.25 min (99%). LRMS (m/z): 371 (M+1)

$^1$H NMR (400 MHz, Chloroform-d) δ 8.65 (s, 1H), 8.49 (d, J = 0.8 Hz, 1H), 8.22 (d, J = 7.6 Hz, 1H), 7.99 (dd, J = 8.0, 1.6 Hz, 1H), 7.71 - 7.62 (m, 1H), 7.59 - 7.54 (m, 1H), 7.33 (s, 1H), 6.88 (s, 1H), 3.08 (s, 3H), 2.61 (s, 3H), 2.46 (s, 3H).

**Example 12: N-[2-[[6-[(2,6-dimethylpyrimidin-4-yl)amino]pyrimidin-4-yl]amino]phenyl]-N-methyl-methanesulfonamide**

**Step 12-1: Syntesis of N-methyl-N-(2-nitrophenyl)methanesulfonamide**

N-(2-nitrophenyl)methanesulfonamide (2 g; 9.25 mmol) are disolved in DMF (20 mL). Potassium carbonate (5.1 g; 36.9 mmol) and iodomethane (1.72 mL; 27.6 mmol) are added and the mixture is stirred at rt for 1 hr. 50 mL of water are added and, after stirring for 30 min the solid is filtered, washed with water and dried to give 2 g (94%) of title compound.

uPLC-MS tr = 0.99 min (100%). LRMS (m/z): 231 (M+1)

$^1$H NMR (400 MHz, DMSO-d6) δ 7.95 (dd, J = 8.1, 1.2 Hz, 1H), 7.85 - 7.74 (m, 2H), 7.62 (ddd, J = 8.1, 7.0, 1.9 Hz, 1H), 3.28 (s, 2H), 3.05 (s, 2H).

### Step 12-2: Synthesis of N-(2-aminophenyl)-N-methyl-methanesulfonamide

200 mg (0.18 mmol) of Pd 10% on charcoal are added to a solution of N-methyl-N-(2-nitrophenyl)methanesulfonamide (Step 12-1, 2 g; 8.68 mmol) in 20 mL of methanol and 20 mL of THF and the mixture is stirred in $H_2$ atmosphere (40 psi) at room temperature for 2 hr. After filtration of the catalyst the solution is concentrated and the residue purified through flash chromatography eluting with n-hexanes/AcOEt from 0 to 100% to give 1 g (57%) of title compound as a white solid.

uPLC-MS tr = 0.79 min (99%). LRMS (m/z): 201 (M+1)

$^1$H NMR (400 MHz, DMSO-d6) δ 7.19 (dd, J = 7.9, 1.5 Hz, 1H), 7.03 (ddd, J = 8.7, 7.3, 1.5 Hz, 1H), 6.74 (dd, J = 8.1, 1.4 Hz, 1H), 6.56 (ddd, J = 7.8, 7.3, 1.5 Hz, 1H), 5.10 (s, 2H), 3.02 (s, 3H), 3.05 (s, 3H).

### Step 12-3: Synthesis of N-[2-[[6-[(2,6-dimethylpyrimidin-4-yl)amino]pyrimidin-4-yl]amino]phenyl]-N-methyl-methanesulfonamide

A mixture of N-(6-chloropyrimidin-4-yl)-2,6-dimethyl-pyrimidin-4-amine (Step 6-1; 100 mg, 0.42 mmol), N-(2-aminophenyl)-N-methyl-methanesulfonamide (Step 12-2, 118 mg, 0.59 mmol), Pd2dba3 (49 mg; 0.084 mmol), xantphos (49 mg; 0.085 mmol), cesium carbonate (0.414 g; 1.271 mmol) and 3.5 ml dioxane is heated to 100°C for 1.5 hr in a microwave oven under argon atmosphere. Excess water and AcOEt are added and the aqueous layer is further extracted twice with additional AcOEt. The combined extracts are washed with brine, dried and concentrated. The residue after concentration is purified through flash chromatography eluting with DCM/MeOH from 0 to 2% to give 40 mg (24%) of title compound as a white solid. uPLC-MS tr = 1.24 min (99%). LRMS (m/z): 400 (M+1)

$^1$H NMR (400 MHz, Chloroform-d) δ 8.44 (d, J = 0.8 Hz, 1H), 8.00 (dd, J = 8.2, 1.3 Hz, 1H), 7.72 (s, 1H), 7.46 - 7.38 (m, 1H), 7.34 (dd, J = 8.0, 1.5 Hz, 1H), 7.19 (td, J = 7.9, 1.4 Hz, 1H), 6.92 (s, 1H), 5.30 (s, 1H), 3.28 (s, 3H), 2.98 (s, 3H), 2.57 (s, 3H), 2.44 (s, 3H).

## Example 13: N-methyl-N-[2-[[6-[(1-methyl-1,2,4-triazol-3-yl)amino]pyrimidin-4-yl]amino]phenyl]methanesulfonamide

### Step 13-1: Synthesis of 6-chloro-N-(1-methyl-1,2,4-triazol-3-yl)pyrimidin-4-amine

A mixture of 4,6-dichloropyrimidine (100 mg, 0.67 mmol), 1-methyl-1,2,4-triazol-3-amine (72 uL, 0.73 mmol), Pd2dba3 (61 mg; 0.066 mmol), xantphos (77 mg; 0.133 mmol), cesium carbonate (656 mg; 2.01 mmol) and 6 ml dioxane is heated to 100°C for 1.5 hr in a microwave oven under argon atmosphere. Excess water and AcOEt are added and the aqueous layer is further extracted twice with additional AcOEt. The combined extracts are washed with brine, dried and concentrated. The residue after concentration is purified through flash chromatography eluting with DCM/MeOH from 0 to 5% to give 105 mg (52%) of title compound as a white solid.

uPLC-MS tr = 0.84 min (91%). LRMS (m/z): 211 (M+1)

$^1$H NMR (400 MHz, DMSO-d6) δ 10.82 (bs, 1H), 8.51 (s, 1H), 7.84 - 7.76 (m, 1H), 7.47 (s, 1H), 3.84 (s, 3H).

### Step 13-2: Synthesis of N-methyl-N-[2-[[6-[(1-methyl-1,2,4-triazol-3-yl)amino]pyrimidin-4-yl]amino]phenyl]methanesulfonamide

A mixture of 6-chloro-N-(1-methyl-1,2,4-triazol-3-yl)pyrimidin-4-amine (Step 13-1, 105 mg, 0.500 mmol), N-(2-aminophenyl)-N-methyl-methanesulfonamide (Step 12-2, 130 mg; 0.65 mmol), Pd2dba3 (45 mg; 0.049 mmol), xantphos (57 mg; 0.095 mmol), cesium carbonate (487 mg; 1.494 mmol) and 4.5 ml dioxane is heated to 100°C for 1.5 hr in a microwave oven under argon atmosphere. Excess water and AcOEt are added and the aqueous layer is further extracted twice with additional AcOEt. The combined extracts are washed with brine, dried and concentrated. The residue after concentration is purified through flash chromatography eluting with DCM/MeOH from 0 to 5% to give 8 mg (4%) of title compound as a white solid.

uPLC-MS tr = 1.12 min (99%). LRMS (m/z): 375 (M+1)

$^1$H NMR (400 MHz, Chloroform-d) δ 8.42 (d, J = 0.8 Hz, 1H), 8.17 (dd, J = 8.3, 1.4 Hz, 1H), 7.83 (s, 1H), 7.74 (s, 1H), 7.63 (s, 1H), 7.44 - 7.34 (m, 2H), 7.31 (dd, J = 8.0, 1.5 Hz, 1H), 7.13 (td, J = 7.9, 1.5 Hz, 1H), 3.93 - 3.86 (m, 3H), 3.28 (s, 3H), 2.98 (s, 3H).

## Example 14: N-[6-[2-[methyl(methylsulfonyl)amino]anilino]pyrimidin-4-yl]cyclopropanecarboxamide

### Step 14-1: Synthesis of N-[2-[(6-chloropyrimidin-4-yl)amino]phenyl]-N-methyl-methanesulfonamide

A mixture of 4,6-dichloropyrimidine (148 mg; 0.993 mmol), N-(2-aminophenyl)-N-methyl-methanesulfonamide (Step 12-2, 199 mg; 0.993 mmol), silver trifluoromethanesulphonate (255 mg; 0.002 mmol) in 4 mL dioxane was stirred at 120°C in a microwave oven for 90 min. The mixture was concentrated, taken in AcOEt and filtered through a Celite pad washing with further AcOEt. After concentration the residue was purified through flash chromatography on silicagel eluting with hexanes/AcOEt from 0 to 100% to afford 180 mg (58%) of title compound as a cream-coloured solid.

uPLC-MS tr = 1.21 min (92%). LRMS (m/z): 311/313 (M+1; Cl).

$^1$H NMR (400 MHz, DMSO-d6) δ 9.15 (s, 1H), 8.40 (d, J = 0.8 Hz, 1H), 7.84 (dd, J = 8.2, 1.4 Hz, 1H), 7.57 (dd, J = 8.0, 1.5 Hz, 1H), 7.43 - 7.35 (m, 1H), 7.30 - 7.21 (m, 1H), 6.96 (s, 1H), 3.15 (s, 3H), 3.02 (s, 3H).

### Step 14-2: Synthesis of N-[6-[2-[methyl(methylsulfonyl)amino]anilino]pyrimidin-4-yl]cyclopropanecarboxamide

A mixture of N-[2-[(6-chloropyrimidin-4-yl)amino]phenyl]-N-methyl-methanesulfonamide (Step 14-1, 66 mg, 0.210 mmol), cyclopropanecarboxamide (23 mmol, 0.27 mmol), Pd2dba3 (19 mg; 0.021 mmol), xantphos (24 mg; 0.041 mmol), cesium carbonate (206 mg; 0.632 mmol) and 2 ml dioxane is heated to 100°C for 3.5 hr in a microwave oven under argon atmosphere. Excess water and AcOEt are added and the aqueous layer is further extracted twice with additional AcOEt. The combined extracts are washed with brine, dried and concentrated. The residue after concentration is purified through reverse phase flash chromatography eluting with 0% to 100% water-acetonitrile/MeOH (1:1) (0.1% v/v ammonium formate both phases) to give 22 mg (28%) of title compound as a pale orange solid.

uPLC-MS tr = 1.16 min (99%). LRMS (m/z): 362 (M+1)

$^1$H NMR (400 MHz, DMSO-d6) δ 8.69 (s, 1H), 8.33 (d, J = 0.8 Hz, 1H), 7.90 (d, J = 7.0 Hz, 1H), 7.58 (d, J = 0.9 Hz, 1H), 7.53 (dd, J = 7.9, 1.4 Hz, 1H), 7.38 - 7.32 (m, 1H), 7.21 - 7.14 (m, 1H), 3.15 (s, 3H), 3.02 (s, 3H), 2.05 - 1.97 (m, 1H), 0.84 - 0.79 (m, 4H).

## Example 15: N4-(2,6-dimethylpyrimidin-4-yl)-N6-[2-methoxy-3-(1-methylpyrazol-4-yl)phenyl]pyrimidine-4,6-diamine

### Step 15-1: Synthesis of 2-methoxy-3-(1-methylpyrazol-4-yl)aniline

A mixture of 3-bromo-2-methoxyaniline (50 mg; 0.247 mmol), 1-methyl-4-(4,4,5,5-tetramethyl-1,3,2-dioxaborolan-2-yl)pyrazole (67 mg; 0.321 mmol), Pd2dba3 (40 mg; 0.049 mmol), cesium carbonate (371 mg; 1.93 mmol) and 3 ml dioxane is heated to 100°C under argon atmosphere. After 2 hr one additional equivalent of boronate is added as a certain amount of starting bromoderivative is still present and the stirring prosecuted for 2 additional hours. Excess water and AcOEt are added and the aqueous layer is further extracted twice with additional AcOEt. The combined extracts are washed with brine, dried and concentrated. The residue is purified through flash chromatography on silicagel eluting with DCM/MeOH 0-2% to give 27 mg (54%) of pure title compound.

uPLC-MS tr = 0.91min (100%). LRMS (m/z): 204 (M+1)

$^1$H NMR (400 MHz, Chloroform-d) δ 7.88 - 7.83 (m, 1H), 7.78 (s, 1H), 6.96 - 6.83 (m, 2H), 6.64 (dd, J = 7.7, 1.7 Hz, 1H), 3.96 (s, 3H), 3.87 (s, 1H), 3.63 (s, 3H).

### Step 15-2: N4-(2,6-dimethylpyrimidin-4-yl)-N6-[2-methoxy-3-(1-methylpyrazol-4-yl)phenyl]pyrimidine-4,6-diamine

A mixture of N-(6-chloropyrimidin-4-yl)-2,6-dimethyl-pyrimidin-4-amine (Step 6-1; 30 mg, 0.127 mmol), 2-methoxy-3-(1-methylpyrazol-4-yl)aniline (Step 15-1, 26 mg, 0.28 mmol), Pd2dba3 (12 mg; 0.013 mmol), xantphos (15 mg; 0.026 mmol), cesium carbonate (124 mg; 0.380 mmol) and 1.3 ml dioxane is heated to 100°C for 1.5 hr in a microwave oven under argon atmosphere. Excess water and AcOEt are added and the aqueous layer is further extracted twice with additional AcOEt. The combined extracts are washed with brine, dried and concentrated. The residue after concentration is purified through flash chromatography eluting with DCM/MeOH from 0 to 2% to give 1.2 mg (2%) of title compound as a white solid. uPLC-MS tr = 0.99 min (98%). LRMS (m/z): 403 (M+1) $^1$H NMR (400 MHz, Chloroform-d) δ 10.60 (s 1H), 8.44 (s, 1H), 8.70 (s, 1H), 8.37 (s, 1H), 7.70 (d, J = 7.7 Hz, 1H), 7.63 (s 1H), 7.43 (s 1H), 7.23 - 7.14 (m, 2H), 4.12 (s 3H), 3.92 (s, 3H), 2.60 (s, 3H), 2.48 (s, 3H).

**Example 16: 3-[[6-[(2,6-dimethylpyrimidin-4-yl)amino]pyrimidin-4-yl]amino]-2-methoxy-benzonitrile**

**Step 16-1: Synthesis of 3-amino-2-methoxy-benzonitrile**

A mixture of 3-bromo-2-methoxyaniline (158 mg; 0.782 mmol) and copper (I) cyanide (75 mg; 0.506 mmol) in 1.3 mL of 1-methylpirrolidone is stirred at 200°C in a microwave oven for 20 min. After concentration, the residue is purified through silicagel flash chromatography (n-hexanes/AcOEt 0-100%) to afford 75 mg (65%) of title compound.

uPLC-MS tr = 0.97 min (%). LRMS (m/z): 149 (M+1)

$^1$H NMR (400 MHz, Chloroform-d) δ 7.00 - 6.87 (m, 3H), 4.02 (s, 3H).

**Step 16-2: Synthesis of 3-[[6-[(2,6-dimethylpyrimidin-4-yl)amino]pyrimidin-4-yl]amino]-2-methoxy-benzonitrile**

A mixture of N-(6-chloropyrimidin-4-yl)-2,6-dimethyl-pyrimidin-4-amine (Step 6-1; 27 mg, 0.114 mmol), 3-amino-2-methoxy-benzonitrile (Step 16-1, 17 mg, 0.114 mmol), Pd2dba3 (10 mg; 0.011 mmol), xantphos (13 mg; 0.022 mmol), cesium carbonate (112 mg; 0.343 mmol) and 1.5 ml dioxane is heated to 100°C for 1.5 hr in a microwave oven under argon atmosphere. Excess water and AcOEt are added and the aqueous layer is further extracted twice with additional AcOEt. The combined extracts are washed with brine, dried and concentrated. The residue after concentration is purified through flash chromatography eluting with DCM/MeOH from 0 to 5% to give 7 mg (17%) of title compound as a white solid.

uPLC-MS tr = 1.00 min (99%). LRMS (m/z): 348 (M+1)

$^1$H NMR (400 MHz, Chloroform-d) δ 8.47 (s, 1H), 8.36 (dd, J = 8.2, 1.4 Hz, 1H), 7.59 (s, 1H), 7.42 (d, J = 6.7 Hz, 1H), 7.36 - 7.27 (m, 2H), 7.20 (t, J = 8.0 Hz, 1H), 6.83 (s, 1H), 4.13 (s, 3H), 2.61 (s, 3H), 2.45 (s, 3H).

**Example 17: N4-(2,4-dimethoxyphenyl)-N6-(2,6-dimethylpyrimidin-4-yl)pyrimidine-4,6-diamine**

**Step 17-1: Synthesis of N4-(2,4-dimethoxyphenyl)-N6-(2,6-dimethylpyrimidin-4-yl)pyrimidine-4,6-diamine**

A mixture of N-(6-chloropyrimidin-4-yl)-2,6-dimethyl-pyrimidin-4-amine (Step 6-1; 60 mg, 0.254 mmol), 2,4-dimethoxyaniline (43 uL, 0.301 mmol), Pd2dba3 (23 mg; 0.025 mmol), xantphos (29 mg; 0.050 mmol), cesium carbonate (249 mg; 0.764 mmol) and 2 ml dioxane is heated to 100°C for 1.5 hr in a microwave oven under argon atmosphere. Excess water and AcOEt are added and the aqueous layer is further extracted twice with additional AcOEt. The combined extracts are washed with brine, dried and concentrated. The residue after concentration is purified through flash chromatography eluting with DCM/MeOH from 0 to 5% to give 22 mg (24%) of title compound as a white solid.

uPLC-MS tr = 0.96 min (97.5%). LRMS (m/z): 353 (M+1)

1H NMR (400 MHz, Chloroform-d) δ 8.38 (s, 1H), 7.57 (d, J = 8.4 Hz, 1H), 7.17 (s, 1H), 6.90 (s, 1H), 6.77 (s, 1H), 6.59 - 6.50 (m, 2H), 3.84 (d, J = 5.0 Hz, 6H), 2.54 (s, 3H), 2.43 (s, 3H).

**Compounds from Examples 18-27 are prepared in the same way as compounds 15-17 by reacting N-(6-chloropyrimidin-4-yl)-2,6-dimethyl-pyrimidin-4-amine (Step 6-1) with the corresponding aniline.**

**Example 18: N6-(2,6-dimethylpyrimidin-4-yl)-N4-[2-methoxy-3-(trifluoromethyl) phenyl] pyrimidine-4,6-diamine**

uPLC-MS tr = 1.26 min (100%). LRMS (m/z): 391 (M+1)

1H NMR (400 MHz, Chloroform-d) δ 8.48 - 8.44 (m, 1H), 8.08 (d, J = 7.4 Hz, 1H), 7.62 (s, 1H), 7.38 (dd, J = 9.8, 1.8 Hz, 2H), 7.27 (d, J = 15.9 Hz, 1H), 7.06 (s, 1H), 6.81 (s, 1H), 3.86 (s, 3H), 2.58 (s, 3H), 2.44 (s, 3H).

**Example 19: N4-(3,4-difluoro-2-methoxy-phenyl)-N6-(2,6-dimethylpyrimidin-4-yl)pyrimidine-4,6-diamine**

uPLC-MS tr = 1.13 min (99%). LRMS (m/z): 359 (M+1)

1H NMR (400 MHz, Methanol-d4) δ 8.17 (d, J = 0.9 Hz, 1H), 7.43 - 7.31 (m, 2H), 7.01 - 6.87 (m, 2H), 3.85 (s, 3H), 2.39 (s, 3H), 2.28 (s, 3H).

**Example 20: N4-(2,3-dimethoxyphenyl)-N6-(2,6-dimethylpyrimidin-4-yl)pyrimidine-4,6-diamine**

uPLC-MS tr = 0.99 min (100%). LRMS (m/z): 353 (M+1)

1H NMR (400 MHz, Chloroform-d) δ 8.43 (d, J = 0.9 Hz, 1H), 7.64 (s, 1H), 7.49 (dd, J = 8.3, 1.2 Hz, 1H), 7.33 (s, 1H), 7.09 (t, J = 8.3 Hz, 1H), 6.87 (s, 1H), 6.71 (dd, J = 8.4, 1.3 Hz, 1H), 3.89 (s, 3H), 3.87 (s, 3H), 2.60 (s, 3H), 2.44 (s, 3H).

**Example 21: N4-(2,6-dimethylpyrimidin-4-yl)-N6-(4-fluoro-2-methoxy-phenyl)pyrimidine-4,6-diamine**

HPLC-MS tr = 1.81 min (99.2%). LRMS (m/z): 341 (M+1)

NMR 1H (DMSO, 400 MHz) δ ppm 2.29 (s, 3H), 2.38 (s, 3H), 3.80 (s, 3H), 6.79 (td, J = 8.5 and 2.8 Hz, 1H), 7.02 (dd, J = 11 and 2.8 Hz, 1H), 7.18 (s, 2H), 7.51 (dd, J = 8.6 and 6.4 Hz, 1H), 8.24 (s, 1H), 8.62 (s, 1H), 9.97 (s, 1H).

**Example 22: N6-(2,6-dimethylpyrimidin-4-yl)-N4-[2-methoxy-4-(trifluoromethyl)phenyl]pyrimidine-4,6-diamine**

HPLC-MS tr = 2.28 min (99%). LRMS (m/z): 391 (M+1)

1H NMR (400 MHz, DMSO-d6) δ 2.31 (s, 3H), 2.45 (s, 3H), 3.92 (s, 3H), 7.24 (s, 1H), 7.28-7.33 (m, 2H), 7.45 (s, 1H), 8.13 (d, J = 8.1 Hz, 1H), 8.37 (s, 1H), 8.95 (s, 1H), 10.11 (s, 1H).

**Example 23: N6-(2,6-dimethylpyrimidin-4-yl)-N4-(2-methoxyphenyl)pyrimidine-4,6-diamine**

HPLC-MS tr = 1.77 min (99%). LRMS (m/z): 323 (M+1)

1H NMR (400 MHz, DMSO-d6) δ 2.29 (s, 3H), 2.39 (s, 3H), 3.80 (s, 3H), 6.93 - 6.98 (m, 1H), 7.09 (d, J = 8.2 Hz, 1H), 7.12 - 7.17 (m, 1H), 7.19 (s, 1H), 7.28 (s, 1H), 7.57 - 7.62 (m, 1H), 8.27 (s, 1H), 8.59 (s, 1H), 9.99 (s, 1H).

**Example 24: N-[2-[[6-[(2,6-dimethylpyrimidin-4-yl)amino]pyrimidin-4-yl]amino]-5-(3-methylimidazol-4-yl)phenyl]-N-methyl-methanesulfonamide**

Intermediate compound N-[2-amino-5-(3-methylimidazol-4-yl)phenyl]-N-methyl-methanesulfonamide was prepared according to WO2018075937 compound 212.1 uPLC-MS tr = 0.60 min (100%). LRMS (m/z): 480 (M+1)

1H NMR (400 MHz, DMSO-d6) δ 10.16 (s, 1H), 8.55 (s, 1H), 8.36 (d, J = 0.8 Hz, 1H), 7.92 (d, J = 8.5 Hz, 1H), 7.72 (s, 1H), 7.67 (d, J = 2.0 Hz, 1H), 7.51 (dd, J = 8.4, 2.0 Hz, 1H), 7.41 (s, 1H), 7.30 (s, 1H), 7.10 (d, J = 1.1 Hz, 1H), 3.72 (s, 3H), 3.08 (s, 3H), 2.44 (s, 3H), 2.31 (s, 3H).

**Example 25: N-[5-(1,5-dimethylpyrazol-4-yl)-2-[[6-[(2,6-dimethylpyrimidin-4-yl)amino]pyrimidin-4-yl]amino]phenyl]-N-methyl-methanesulfonamide**

Intermediate compound N-[2-amino-5-(1,5-dimethylpyrazol-4-yl)phenyl]-N-methyl-methanesulfonamide was prepared according to WO2018075937 compound 198.1 uPLC-MS tr = 0.98 min (99.9%). LRMS (m/z): 494 (M+1)

1H NMR (400 MHz, DMSO-d6) δ 10.12 (s, 1H), 8.46 (s, 1H), 8.32 (d, J = 0.8 Hz, 1H), 7.76 (d, J = 8.4 Hz, 1H), 7.62 (s, 1H), 7.53 (d, J = 2.0 Hz, 1H), 7.44 - 7.35 (m, 2H), 7.26 (s, 1H), 3.80 (s, 3H), 3.21 (s, 3H), 3.07 (s, 3H), 2.40 (s, 3H), 2.31 (s, 3H).

**Example 26: N4-(2,6-dimethylpyrimidin-4-yl)-N6-[4-(3-methylimidazol-4-yl)-2-methylsulfonyl-phenyl]pyrimidine-4,6-diamine**

Intermediate compound 4-(3-methylimidazol-4-yl)-2-methylsulfonyl-aniline was prepared according to WO2018075937 compound 214.2

HPLC-MS tr = 1.77 min (99%). LRMS (m/z): 323 (M+1)

1H NMR (400 MHz, DMSO-d6) δ 8.78 (s, 1H), 8.55 (s, 1H), 8.51 (s, 1H), 8.38 (d, J = 8.5 Hz, 1H), 8.01 (s, 1H), 7.68 (d, J = 2.0 Hz, 1H), 7.57 (dd, J = 8.4, 2.0 Hz, 2H), 7.18 (s, 1H), 6.92 (s, 1H), 3.71 (s, 3H), 3.11 (s, 3H), 2.61 (s, 3H), 2.46 (s, 3H).

**Example 27: 6-[[6-[(2,6-dimethylpyrimidin-4-yl)amino]pyrimidin-4-yl]amino]-5-methoxy-pyridine-3-carbonitrile**

Intermediate compound 6-amino-5-methoxynicotinonitrile was prepared according to WO2013037755 example 1.

uPLC-MS tr = 1.03 min (100%). LRMS (m/z): 349 (M+1)

1H NMR (400 MHz, Chloroform-d) δ 8.82 (s, 1H), 8.53 (s, 1H), 8.26 (d, J = 1.7 Hz, 1H), 8.20 (s, 1H), 7.50 (s, 1H), 7.33 (s, 1H), 7.20 (d, J = 1.7 Hz, 1H), 3.99 (s, 3H), 2.67 (s, 3H), 2.49 (s, 3H).

**Example 28: 4-N-(2-dimethylphosphorylphenyl)-6-N-(2,6-dimethylpyrimidin-4-yl)pyrimidine-4,6-diamine**

**Step 28-1: Synthesis of 4-N-(2-dimethylphosphorylphenyl)-6-N-(2,6-dimethylpyrimidin-4-yl)pyrimidine-4,6-diamine**

A mixture of 4,6-dichloropyrimidine (180 mg; 0.575 mmol), (2-aminophenyl)dimethylphosphine oxide (168 mg; 0.993 mmol) and silver trifluoromethanesulfonate (255 mg; 0.992 mmol) in 4 ml dioxane was heated in MW oven for 45 min at 120°C. The mixture was evaporated, taken up in EtOAc and filtered through Celite washing the filtercake with further ethyl acetate. The filtrate and washing were combined and evaporated to give a residue that was purified by silica gel chromatography (Isolera, 0-100% EtOAc in hexanes) to give the title product as a cream-coloured solid (132 mg, 47%).

uPLC-MS tr = 1.55 min (98.8%). LRMS (m/z): 369 (M+1, Cl)

1H NMR (400 MHz, CD3OD) δ 8.52 (s, 1H), 8.17 (dd, J = 8.2, 1.4 Hz, 1H), 7.71-7.62 (cs, 3H), 7.32 (m, 1H), 2.68 (s, 3H), 2.58 (s, 3H), 1.87 (s, 3H), 1.84 (s, 3H).

**Example 29: N6-(2,6-dimethylpyrimidin-4-yl)-N4-[2-methoxy-3-[1-methyl-5-(trifluoromethyl)-1,2,4-triazol-3-yl]phenyl]pyrimidine-4,6-diamine**

**Step 29-1: Synthesis of 2-methoxy-3-[1-methyl-5-(trifluoromethyl)-1,2,4-triazol-3-yl]aniline**

A mixture of 2-Methoxy-3-(4,4,5,5-tetramethyl-1,3,2-dioxaborolan-2-yl)aniline (Step 1-1, 100 mg, 0.401 mmol), 3-bromo-1-methyl-5-(trifluoromethyl)-1,2,4-triazole (138 mg, 0.600 mmol), Pd(dppf)Cl2 (29 mg, 0.040 mmol), cesium carbonate (602 uL 2 M in water) in dioxane (2 mL) was stirred at 100 °C for 3 hr under argon atmosphere. The mixture was diluted in ethyl acetate and washed with brine, dried, filtered and concentrated. The residue was purified by flash chromatography (5% methanol in dichloromethane gradient) to obtain the title compound as a yellow oil (103 mg, 94%).

HPLC-MS tr = 2.25 min (98.7%). LRMS (m/z): 273 (M+1)

1H NMR (400 MHz, DMSO-d6) δ 6.96 (dd, J = 7.6, 1.8 Hz, 1H), 6.90 (t, J = 7.7 Hz, 1H), 6.80 (dd, J = 7.8, 1.8 Hz, 1H), 5.07 (s, 2H), 4.10 (d, J = 1.2 Hz, 3H), 3.68 (s, 3H).

**Step 29-2: Synthesis of N6-(2,6-dimethylpyrimidin-4-yl)-N4-[2-methoxy-3-[1-methyl-5-(trifluoromethyl)-1,2,4-triazol-3-yl]phenyl]pyrimidine-4,6-diamine**

A solution of 2-methoxy-3-[1-methyl-5-(trifluoromethyl)-1,2,4-triazol-3-yl]aniline (Step 29-1, 50 mg, 0.184 mmol), N-(6-chloropyrimidin-4-yl)-2,6-dimethyl-pyrimidin-4-amine (Step 6-1, 65 mg, 0.275 mmol), Pd2(dba)3 (18 mg, 0.019 mmol), Xantphos (21 mg, 0.036 mmol) and cesium carbonate (180 mg, 0.552 mmol) was heated in dioxane (1,8 mL) at 100 °C for 90 min. The mixture was diluted in ethyl acetate and washed with brine, dried, filtered and concentrated. The crude was purified by flash chromatography (10% methanol in dichloromethane gradient) to obtain title compound as a yellowish solid (46,4 mg, 53%).

HPLC-MS tr = 2.07 min (100%). LRMS (m/z): 472 (M+1)

1H NMR (400 MHz, DMSO-d6) δ 10.04 (s, 1H), 8.95 (s, 1H), 8.30 (d, J = 1.0 Hz, 1H), 7.82 (dd, J = 8.0, 1.7 Hz, 1H), 7.57 (dd, J = 7.8, 1.7 Hz, 1H), 7.36 (d, J = 1.0 Hz, 1H), 7.23 (t, J = 7.9 Hz, 1H), 7.19 (s, 1H), 4.11 (q, J = 1.1 Hz, 3H), 3.67 (s, 3H), 2.36 (s, 3H), 2.30 - 2.25 (m, 3H).

**Example 30: N4-(2,6-dimethylpyrimidin-4-yl)-N6-[2-methoxy-3-[5-(methoxymethyl)-1,2,4-oxadiazol-3-yl]phenyl]pyrimidine-4,6-diamine**

**Step 30-1: Synthesis of 3-[[6-[(2,6-dimethylpyrimidin-4-yl)amino]pyrimidin-4-yl]amino]-N'-hydroxy-2-methoxy-benzamidine**

A mixture of hydroxilamine hydrochloride (350 mg, 5.04 mmol) and potassium hydroxide (280 mg, 5.00 mmol) in 10 ml of etanol is stirred at rt for 1 hr. After filtering the solids, the filtrate is mixed with 3-[[6-[(2,6-dimethylpyrimidin-4-yl)amino]pyrimidin-4-yl]amino]-2-methoxy-benzonitrile (step 16-2; 175 mg, 0.504 mmol) and the resulting mixture is stirred at 85°C for 8 hr. After concentration the residue is purified through flash chromatohgraphy (10% methanol in dichloromethane gradient) to obtain title compound as a white solid (192 mg, 35%).

uPLC-MS tr = 0.73 min (91%). LRMS (m/z): 381 (M+1)

1H NMR (400 MHz, DMSO-d6) δ 10.05 (s, 1H), 9.50 (s, 1H), 8.86 (s, 1H), 8.31 (d, J = 0.9 Hz, 1H), 7.74 (dd, J = 7.9, 1.7 Hz, 1H), 7.32 (s, 1H), 7.27 (s, 1H), 7.17 (dd, J = 7.7, 1.8 Hz, 1H), 7.10 (t, J = 7.8 Hz, 1H), 5.71 (s, 1H), 3.68 (s, 3H), 2.43 (s, 3H), 2.30 (s, 3H).

**Step 30-2: Synthesis of N4-(2,6-dimethylpyrimidin-4-yl)-N6-[2-methoxy-3-[5-(methoxymethyl)-1,2,4-oxadiazol-3-yl]phenyl]pyrimidine-4,6-diamine**

A solution of 3-[[6-[(2,6-dimethylpyrimidin-4-yl)amino]pyrimidin-4-yl]amino]-N'-hydroxy-2-methoxy-benzamidine (Step 30-1; 75 mg, 0.179 mmol), 2-methoxyacetic acid (48 mg, 0.532 mmol), N,N'-Diisopropylcarbodiimide (90.56 mg, 0.439 mmol) and tetrabutylammonium fluoride (1.076 ml of 1M solution in THF; 1.07 mmol) in 1.7 mL DMF is stirred at 50°C for 1 hr. After dilution with DCM/water the organic phase is washed with 4% sodium hydrogen carbonate solution, 4xwater, dried and concentrated. The residue is purified through flash chromatohgraphy (5% methanol in dichloromethane gradient) to obtain title compound as a white solid (18 mg, 23%).

uPLC-MS tr = 1.10 min (100%). LRMS (m/z): 435 (M+1)

1H NMR (400 MHz, Chloroform-d) δ 8.47 (d, J = 0.9 Hz, 1H), 8.12 (dd, J = 8.1, 1.5 Hz, 1H), 7.79 (dd, J = 7.9, 1.6 Hz, 1H), 7.68 (s, 1H), 7.40 (s, 1H), 7.31 (t, J = 8.0 Hz, 2H), 6.83 (s, 1H), 4.79 (s, 2H), 3.86 (s, 3H), 3.58 (s, 3H), 2.59 (s, 3H), 2.44 (s, 3H).

**Example 31: N4-(2,6-dimethylpyrimidin-4-yl)-N6-[3-(5-fluoropyrimidin-2-yl)-2-methoxy-phenyl]pyrimidine-4,6-diamine**

**Step 31-1: Synthesis of N4-(2,6-dimethylpyrimidin-4-yl)-N6-[3-(5-fluoropyrimidin-2-yl)-2-methoxy-phenyl]pyrimidine-4,6-diamine**

A miture of N-(6-chloropyrimidin-4-yl)-2,6-dimethyl-pyrimidin-4-amine (Step 6-1; 17 mg, 0.041 mmol), 3-(5-fluoropyrimidin-2-yl)-2-methoxy-aniline (64 mg. 292 mmol), Pd2dba3 (26.8 mg; 0.029 mmol), xantphos (34 mg; 0.058 mmol), cesium carbonate (286 mg; 0.88 mmol) and 3 ml dioxane is heated in MW to 100°C for 1.5 hr under argon atmosphere. Excess water anc AcOEt are added and the aqueous layer is further extracted twice with additional AcOEt. The combined extracts are washed with brine, dried and concentrated. The residue after concentration was purified through flash chromatography eluting with DCM/MeOH from 0 to 2% to give 17 mg (17%) of title compound.

uPLC-MS tr = 1.01 min (98.7%). LRMS (m/z): 419 (M+1)

1H NMR (400 MHz, Chloroform-d) δ 8.75 (s, 2H), 8.46 (s, 1H), 8.03 (dd, J = 8.1, 1.3 Hz, 1H), 7.69 (s, 1H), 7.59 (dd, J = 7.8, 1.5 Hz, 1H), 7.39 (s, 1H), 7.33 - 7.25 (m, 1H), 6.84 (s, 1H), 5.30 (s, 1H), 3.71 (s, 3H), 2.59 (s, 3H), 2.44 (s, 3H).

**Example 32: N-[2-[[6-[(6-cyano-2-pyridyl)amino]pyrimidin-4-yl]amino]phenyl]-N-methyl-methanesulfonamide**

**Step 32-1: Synthesis of N-[2-[[6-[(6-cyano-2-pyridyl)amino]pyrimidin-4-yl]amino]phenyl]-N-methyl-methanesulfonamide**

A miture of N-[2-[(6-chloropyrimidin-4-yl)amino]phenyl]-N-methyl-methanesulfonamide (Step 28-1; 44 mg, 0.141 mmol), 6-aminopyridine-2-carbonitrile (20 mg, 0.168 mmol), Pd2dba3 (12.8 mg, 0.014 mmol), xantphos (16.3 mg; 0.028 mmol), cesium carbonate (135 mg; 0.41 mmol) and 1.5 ml dioxane is heated to 100°C for 4 hr under argon atmosphere. Excess water anc AcOEt are added and the aqueous layer is further extracted twice with additional AcOEt. The combined extracts are washed with brine, dried and concentrated. The residue after concentration was purified through flash chromatography eluting with DCM/MeOH from 0 to 5% to give 14 mg (25%) of title compound.

uPLC-MS tr = 1.01 min (92.7%). LRMS (m/z): 396 (M+1)

1H NMR (400 MHz, Chloroform-d) δ 8.42 (s, 1H), 7.98 - 7.91 (m, 1H), 7.77 - 7.63 (m, 2H), 7.62 - 7.44 (m, 3H), 7.40 - 7.32 (m, 2H), 5.30 (s, 1H), 3.28 (s, 3H), 2.97 (s, 3H).

**Example 33: N6-[2-methoxy-3-(1-methyl-1,2,4-triazol-3-yl)phenyl]-N4-(6-methylpyrimidin-4-yl)pyrimidine-4,6-diamine**

**Step 33-1: Synthesis of N6-[2-methoxy-3-(1-methyl-1,2,4-triazol-3-yl)phenyl]-N4-(6-methylpyrimidin-4-yl)pyrimidine-4,6-diamine**

By using 2-methoxy-3-(1-methyl-1,2,4-triazol-3-yl)aniline (Step 1-2; 30 mg, 0.075 mmol) and 6-methylpyrimidin-4-amine (17 mg, 0.156 mmol) as starting materials and following the same methodology as in step 31-1, 9 mg (30%) of title compound are obtained after flash chromatography purification using dichloromethane/MeOH (up to 10%) as eluent.

uPLC-MS tr = 0.89 min (96%). LRMS (m/z): 390 (M+1)

1H NMR (400 MHz, Methanol-d4) δ 8.49 (s, 1H), 8.38 (s, 1H), 8.27 - 8.22 (m, 1H), 7.80 (d, J = 6.8 Hz, 1H), 7.49 (dd, J = 7.8, 1.6 Hz, 1H), 7.35 (s, 1H), 7.28 (s, 1H), 7.14 (t, J = 7.9 Hz, 1H), 4.49 (s, 1H), 3.92 (s, 3H), 3.57 (s, 3H), 2.33 (s, 3H).

**Example 34: N-[2-[[6-[(2,6-dimethylpyrimidin-4-yl)amino]pyrimidin-4-yl]amino]phenyl]-N-(trideuteriomethyl)methanesulfonamide**

The title compound was prepared in a analogous way as exemple 12 but replacing the methyl iodide by the trideuterio(iodo)methane in the first step.

uPLC-MS tr = 0.87 min (91%). LRMS (m/z): 403 (M+1)

1H NMR (400 MHz, DMSO-d6) δ 10.12 (s, 1H), 8.46 (s, 1H), 8.36 - 8.29 (m, 1H), 7.80 - 7.73 (m, 1H), 7.57 (dd, J = 8.0, 1.4 Hz, 1H), 7.43 - 7.35 (m, 2H), 7.29 - 7.19 (m, 2H), 3.04 (s, 3H), 2.41 (s, 3H), 2.31 (s, 3H).

**Example 35: N-[3-[[6-[(2,6-dimethylpyrimidin-4-yl)amino]pyrimidin-4-yl]amino]pyridin-4-yl]-N-methylmethanesulfonamide**

**Step 35-1: Synthesis of N-[3-[[6-[(2,6-dimethylpyrimidin-4-yl)amino]pyrimidin-4-yl]amino]pyridin-4-yl]-N-methylmethanesulfonamide**

Starting from N-(6-chloropyrimidin-4-yl)-2,6-dimethyl-pyrimidin-4-amine (Step 6-1, 60 mg; 0.254 mmol) and N-(3-aminopyridin-4-yl)-N-methylmethanesulfonamide (78 mg; 0.384 mmol) and following the methodology described in step 28-2 25 mg (24%) of title compound were obtained as a white solid.

HPLC-MS tr = 1.48 min (100%). LRMS (m/z): 401 (M+1)

NMR 1H (D2O, 400 MHz) δ ppm 2.56 (s, 3H), 2.73 (s, 3H), 3.16 (s, 3H), 4.08 (s, 3H), 7.25-7.27 (m, 2H), 7.63 (d, J = 8 Hz, 1H), 8.15 (dd, J = 6.8 Hz, J = 1.6 Hz, 1H), 8.52 (s, 1H), 8.88 (s, 1H).

**Example 36: N6-(2,6-dimethylpyrimidin-4-yl)-N4-[2-methoxy-3-[2-methyl-5-(trifluoromethyl)pyrazol-3-yl]phenyl]pyrimidine-4,6-diamine**

**Step 36-1: Synthesis of 2-methoxy-3-[2-methyl-5-(trifluoromethyl)pyrazol-3-yl]aniline**

Starting with 190 mg (0.108 mmol) of 3-bromo-2-methoxy-aniline and 201 mg (0.103 mmol) of [2-methyl-5-(trifluoromethyl)pyrazol-3-yl]boronic acid and following the procedure described in step 15-1 70 mg (27%) of title compound are o btained as a colorless oil.

HPLC-MS: tr = 2.70 min. (99 %). LRMS (m/z): 272 (M+1)

1H NMR (400 MHz, DMSO-d6) δ 3.34 (s, 3H), 3.73 (s, 3H), 5.18 (s, 2H), 6.48 - 6.51 (m, 1H), 6.76 (s, 1H), 6.84 (dd, J = 8.0, 1.6 Hz, 1H), 6.94 (t, J = 7.7 Hz, 1H).

**Step 36-2: Synthesis of N6-(2,6-dimethylpyrimidin-4-yl)-N4-[2-methoxy-3-[2-methyl-5-(trifluoromethyl)pyrazol-3-yl]phenyl]pyrimidine-4,6-diamine**

To a solution of N-(6-chloropyrimidin-4-yl)-2,6-dimethyl-pyrimidin-4-amine (Step 6-1; 33 mg, 0.14 mmol) in 1.5 mL of anh. dioxane was added, under Ar, 2-methoxy-3-[2-methyl-5-(trifluoromethyl)pyrazol-3-yl]aniline (Step 36-1; 25 mg, 0.094 mmol), Pd2(dba)3 (9 mg, 0.009 mmol), Xantphos (11 mg, 0.019 mmol) and cesium carbonate (92 mg, 0.28 mmol) and the mixture was heated at 100 °C. After 1 h 30 min. the reaction is finished. The mixture was cooled down to room temperature and EtOAc was added. The mixture was washed with brine, dried and concentrated. The crude was purified via flash chromatography in CH2Cl2/CH2Cl2 with 10 % MeOH to obtain the title compound as a yellow solid (30 mg, 49 %).

HPLC-MS: tr = 2.32 min. (99 %). LRMS (m/z): 471 (M+1)

1H NMR (400 MHz, DMSO-d6) δ 2.31 (s, 3H), 2.43 (s, 3H), 3.41 (s, 3H), 3.78 (s, 3H), 6.86 (s, 1H), 7.17 (dd, J = 7.6, 1.7 Hz, 1H), 7.23 - 7.29 (m, 2H), 7.34 (s, 1H), 7.94 (dd, J = 8.0, 1.6 Hz, 1H), 8.31 - 8.35 (m, 1H), 9.06 (s, 1H), 10.09 (s, 1H).

**Example 37: N4-(2,6-dimethylpyrimidin-4-yl)-N6-[2-methoxy-3-(5-methyl-1,2,4-oxadiazol-3-yl)phenyl]pyrimidine-4,6-diamine**

**Step 37-1: Synthesis of 3-amino-N'-hydroxy-2-methoxy-benzamidine**

4 g (57.56 mmol) of hydroxylamine hydrochloride are added to a solution of 3.257 g (57.56 mmol) of potassium hydroxide in 10 mL of ethanol and the mixture is stirred 1 hr at rt. The salts are filtered and 860 mg (5.80 mmol) of 3-amino-2-methoxy-benzonitrile are added to the filtrate. The mixture is stirred at 85°C for 9hr. Once at rt the solution is concentrated and the residue purified through flash chromatography eluting with DCM to DCM/MeOH 95:5 to obtain

0.78 g (74%) of pure title compound

uPLC-MS: tr = 0.21 and 0.27 min. (100 %). LRMS (m/z): 182 (M+1)

1H NMR (400 MHz, Chloroform-d) δ 7.01 - 6.88 (m, 2H), 6.79 (dd, J = 7.7, 1.8 Hz, 1H), 5.34 (s, 1H), 3.91 - 3.81 (m, 1H), 3.78 (s, 3H).

**Step 37-2: Synthesis of 2-methoxy-3-(5-methyl-1,2,4-oxadiazol-3-yl)aniline**

200 mg (0.735 mmol) of compound from step 37-1 and 0.75 mL of acetic anhydride are stirred at 100°C for 2hr. The solution is concentrated and the residue is stirred at reflux with 4 mL of methanol and 4 mL of concentrated hydrochloric acid for 1hr. After concentration the residue is partitioned between saturated solution of sodium hydrogen carbonate and EtOAc. The organic phase is washed with water, dried and concentrated to give 102 mg (90%) of title compound.

uPLC-MS: tr = 1.01 min. (97 %). LRMS (m/z): 206 (M+1)

1H NMR (400 MHz, Chloroform-d) δ 7.33 (dd, J = 7.8, 1.6 Hz, 1H), 7.03 (t, J = 7.8 Hz, 1H), 6.90 (dd, J = 7.9, 1.6 Hz, 1H), 3.81 (s, 3H), 2.66 (s, 3H).

**Step 37-3: Synthesis of N4-(2,6-dimethylpyrimidin-4-yl)-N6-[2-methoxy-3-(5-methyl-1,2,4-oxadiazol-3-yl)phenyl]pyrimidine-4,6-diamine**

By starting with 115 mg (0.488 mmol) of intermediate 6-1 and 100 mg (0.487 mmol) of intermediate 37-2 and following the procedure described in step 17-1 50 mg (25%) of title compound are obtained.

uPLC-MS: tr = 1.05 min. (100 %). LRMS (m/z): 405 (M+1)

1H NMR (400 MHz, Chloroform-d) δ 8.47 (d, J = 0.9 Hz, 1H), 8.10 (dd, J = 8.1, 1.5 Hz, 1H), 7.73 (dd, J = 7.9, 1.6 Hz, 1H), 7.67 (s, 1H), 7.30 (dd, J = 9.3, 6.8 Hz, 2H), 6.84 (s, 1H), 3.85 (s, 3H), 2.64 (s, 3H), 2.59 (s, 3H), 2.44 (s, 3H).

**Example 38: N6-(2,6-dimethylpyrimidin-4-yl)-N4-[2-methoxy-3-[5-methyl-1-(2,2,2-**

**Step 38-1: Synthesis of 3-bromo-5-methyl-1-(2,2,2-trifluoroethyl)-1,2,4-triazole**

3-Bromo-5-methyl-1H-1,2,4-triazole (150 mg, 0.925 mmol), potassium carbonate (275 mg, 2.77 mmol) was dissolved in DMF anh (4,6 mL). Then, 2,2,2-trifluoroethyl trifluoromethanesulfonate (200 uL, 1.38 mmol) was added and the reaction was stirred overnight at room temperature. The solution was diluted in ethyl acetate and washed x3 with brine. The organic phase was dried, filtered and concentrated to obtain 313 mg of crude that was purified by flash chromatography (2% methanol in DCM gradient) thus obtaining 205 mg (90%) of title compound.

HPLC-MS: tr = 2.06 min. (100 %). LRMS (m/z): 245 (M+1)

1H NMR (400 MHz, Chloroform-d) δ 4.64 (q, J = 8.0 Hz, 2H), 2.51 (s, 3H).

**Step 38-2: Synthesis of 2-methoxy-3-[5-methyl-1-(2,2,2-trifluoroethyl)-1,2,4-triazol-3-**yl]aniline

Starting from 2-methoxy-3-(4,4,5,5-tetramethyl-1,3,2-dioxaborolan-2-yl)aniline (Step 1-1, 100 mg; 0.40 mmol) and 3-bromo-5-methyl-1-(2,2,2-trifluoroethyl)-1,2,4-triazole (step 38-1, 205 mg, 0.84 mmol) and following the procedure of step 1-2 70 mg (61%) of title compound are obtained.

HPLC-MS: tr = 1.90 min. (100 %). LRMS (m/z): 287 (M+1)

1H NMR (400 MHz, Chloroform-d) δ 7.31 (dd, J = 7.8, 1.6 Hz, 1H), 6.98 (t, J = 7.8 Hz, 1H), 6.81 (dd, J = 7.9, 1.6 Hz, 1H), 4.75 (q, J = 8.2 Hz, 2H), 3.94 (bs, 2H), 3.74 (s, 3H), 2.56 (s, 3H).

**Step 38-3: Synthesis of N6-(2,6-dimethylpyrimidin-4-yl)-N4-[2-methoxy-3-[5-methyl-1-(2,2,2-trifluoroethyl)-1,2,4-triazol-3-yl]phenyl]pyrimidine-4,6-diamine**

A solution of 2-methoxy-3-[5-methyl-1-(2,2,2-trifluoroethyl)-1,2,4-triazol-3-yl]aniline (step 38-2; 75mg, 0.262 mmol), N-(6-chloropyrimidin-4-yl)-2,6-dimethyl-pyrimidin-4-amine (Step 6-1; 93 mg, 0.395 mmol), Pd2(dba)3 (24 mg, 0.026 mmol), Xantphos (30 mg, 0.052 mmol) and cesium carbonate (256 mg, 0.786 mmol) was heated in dioxane (2.6 mL) at 100 °C for 1 h. The mixture was diluted in ethyl acetate and washed with brine, dried, filtered and concentrated. The crude was purified by flash chromatography (5% methanol in dichloromethane gradient) to get a yellow oil that crystallises (59 mg, 38%).

HPLC-MS: tr = 1.92 min. LRMS (m/z): 486 (M+1)

1H NMR (400 MHz, DMSO-d6) δ 10.05 (s, 1H), 8.88 (s, 1H), 8.32 (d, J = 1.0 Hz, 1H), 7.79 (dd, J = 8.0, 1.7 Hz, 1H), 7.58 (dd, J = 7.8, 1.7 Hz, 1H), 7.40 - 7.35 (m, 1H), 7.25 - 7.16 (m, 2H), 5.29 (q, J = 9.0 Hz, 2H), 3.65 (s, 3H), 2.53

(s, 3H), 2.38 (s, 3H), 2.30 (s, 3H).

**Example 39: N6-(2,6-dimethylpyrimidin-4-yl)-N4-[3-(1,5-dimethyl-1,2,4-triazol-3-yl)-2-methoxy-phenyl]pyrimidine-4,6-diamine**

**Step 39-1: Synthesis of 3-(1,5-dimethyl-1,2,4-triazol-3-yl)-2-methoxy-aniline**

Starting from 2-methoxy-3-(4,4,5,5-tetramethyl-1,3,2-dioxaborolan-2-yl)aniline (Step 1-1, 100 mg; 0.40 mmol) and 3-bromo-1,5-dimethyl-1,2,4-triazole (101 mg, 0.573 mmol) and following the procedure of step 1-2 54 mg (61%) of title compound are obtained.

HPLC-MS: tr = 1.30 min. (100 %). LRMS (m/z): 219 (M+1)

1H NMR (400 MHz, DMSO-d6) δ 6.92 (dd, J = 7.7, 1.7 Hz, 1H), 6.83 (t, J = 7.7 Hz, 1H), 6.71 (dd, J = 7.8, 1.7 Hz, 1H), 4.93 (s, 2H), 3.80 (s, 3H), 3.65 (s, 3H), 2.42 (s, 3H).

**Step 39-2: Synthesis of N6-(2,6-dimethylpyrimidin-4-yl)-N4-[3-(1,5-dimethyl-1,2,4-triazol-3-yl)-2-methoxy-phenyl]pyrimidine-4,6-diamine**

Starting from 54 mg (0.247 mmol) of intermediate from step 39-1 and 87 mg (0.369 mmol) of intermediate 6-1 and following the procedure described in step 4-1 37 mg (35%) of title compound are obtained as a yellowish solid.

HPLC-MS: tr = 1.57 min. (100 %). LRMS (m/z): 418 (M+1)

1H NMR (400 MHz, DMSO-d6) δ 10.05 (s, 1H), 8.85 (s, 1H), 8.31 (d, J = 1.0 Hz, 1H), 7.73 (dd, J = 8.0, 1.7 Hz, 1H), 7.55 (dd, J = 7.8, 1.7 Hz, 1H), 7.37 (s, 1H), 7.21 (s, 1H), 7.17 (t, J = 7.9 Hz, 1H), 3.83 (s, 3H), 3.67 (s, 3H), 2.45 (s, 3H), 2.38 (s, 3H), 2.30 (s, 3H).

**Example 40: N6-(2,6-dimethylpyrimidin-4-yl)-N4-[3-(2,5-dimethyl-1,2,4-triazol-3-yl)-2-methoxy-phenyl]pyrimidine-4,6-diamine**

**Step 40-1: Synthesis of N6-(2,6-dimethylpyrimidin-4-yl)-N4-[3-(2,5-dimethyl-1,2,4-triazol-3-yl)-2-methoxy-phenyl]pyrimidine-4,6-diamine**

Starting from N-(6-chloropyrimidin-4-yl)-2,6-dimethyl-pyrimidin-4-amine (Intermediate 6-1, 19 mg, 0.08 mmol) and 3-(2,5-dimethyl-1,2,4-triazol-3-yl)-2-methoxy-aniline (prepared according to WO2018075937 intermediate 541.3, 15 mg, 0.068 mg) and following the procedure described in step 4-1 29 mg (40%) of title compound are obtained as a yellowish solid.

HPLC-MS: tr = 1.60 min. (100 %). LRMS (m/z): 418 (M+1)

1H NMR (400 MHz, DMSO-d6) δ 10.07 (s, 1H), 9.02 (s, 1H), 8.32 (d, J = 1.0 Hz, 1H), 7.93 (dd, J = 7.9, 1.8 Hz, 1H), 7.33 (d, J = 1.0 Hz, 1H), 7.27 - 7.20 (m, 2H), 7.18 (dd, J = 7.6, 1.8 Hz, 1H), 3.62 (s, 3H), 3.41 (s, 3H), 2.41 (s, 3H), 2.29 (s, 3H), 2.27 (s, 3H).

**Example 41: N4-(2,6-dimethylpyrimidin-4-yl)-N6-[2-methoxy-3-(2-methyltetrazol-5-yl)phenyl]pyrimidine-4,6-diamine**

**Step 41-1: Synthesis of N4-(2,6-dimethylpyrimidin-4-yl)-N6-[2-methoxy-3-(2-methyltetrazol-5-yl)phenyl]pyrimidine-4,6-diamine**

Starting from N-(6-chloropyrimidin-4-yl)-2,6-dimethyl-pyrimidin-4-amine (Intermediate 6-1, 50 mg, 0.212 mmol) and 2-methoxy-3-(2-methyltetrazol-5-yl)aniline (prepared according to WO2014074661 preparation 12, 65 mg, 0.316 mg) and following the procedure described in step 4-1 30 mg (35%) of title compound are obtained as a white solid.

HPLC-MS: tr = 0.98 min. (100 %). LRMS (m/z): 405 (M+1)

1H NMR (400 MHz, Chloroform-d) δ 8.49 - 8.45 (m, 1H), 8.05 (dd, J = 8.1, 1.5 Hz, 1H), 7.81 (dd, J = 7.9, 1.6 Hz, 1H), 7.70 (s, 1H), 7.40 (s, 1H), 7.35 - 7.27 (m, 1H), 6.83 (s, 1H), 5.30 (s, 1H), 4.46 (s, 3H), 3.83 (s, 3H), 2.59 (s, 3H), 2.44 (s, 3H).

**Example 42: N6-(2,6-dimethylpyrimidin-4-yl)-N4-(2-methoxy-3-pyridazin-4-yl-phenyl)pyrimidine-4,6-diamine**

**Step 42-1: Synthesis of 2-methoxy-3-pyridazin-4-yl-aniline**

In a similar way to that described in step 1-2 but replacing the 3-bromo-1-methyl-1,2,4-triazole by 4-bromopyridazine the title compound was obtained as a yellowish solid in 54% yield after purification by flash chromatography by using DCM to 5% MeOH.

HPLC-MS: tr = 1.57 min. (100 %). LRMS (m/z): 202 (M+1)

1H NMR (400 MHz, Chloroform-d) δ 3.46 (s, 3H), 6.78 (dd, J = 7.7, 1.3 Hz, 1H), 6.87 (dd, J = 7.9, 1.2 Hz, 1H), 7.06 (t, J = 7.8 Hz, 1H), 7.76 (d, J = 3.1 Hz, 1H), 9.23 (d, J = 4.8 Hz, 1H), 9.48 (s, 1H).

**Step 42-2: Synthesis of N6-(2,6-dimethylpyrimidin-4-yl)-N4-(2-methoxy-3-pyridazin-4-yl-phenyl)pyrimidine-4,6-diamine**

Starting from N-(6-chloropyrimidin-4-yl)-2,6-dimethyl-pyrimidin-4-amine (Intermediate 6-1, 24 mg, 0.101 mmol) and 2-methoxy-3-pyridazin-4-yl-aniline (step 42-1, 21 mg, 0.104 mg) and following the procedure described in step 4-1 20 mg (35%) of title compound are obtained as na orange solid.

HPLC-MS: tr = 1.67 min. (99 %). LRMS (m/z): 401 (M+1)

1H NMR (400 MHz, DMSO-d6) δ 2.30 (s, 3H), 2.40 (s, 3H), 3.44 (s, 3H), 7.24 - 7.39 (m, 4H), 7.86 (ddd, J = 15.2, 6.5, 2.1 Hz, 2H), 8.33 (s, 1H), 9.10 (s, 1H), 9.28 - 9.34 (m, 1H), 9.39 - 9.48 (m, 1H), 10.08 (s, 1H)

**Example 43: N4-(2,6-dimethylpyrimidin-4-yl)-N6-[2-methoxy-3-[1-(oxetan-3-yl)-1,2,4-triazol-3-yl]phenyl]pyrimidine-4,6-diamine**

**Step 43-1: Synthesis of 3-bromo-1-(oxetan-3-yl)-1,2,4-triazole**

To a solution of 3-bromo-1H-1,2,4-triazole (50 mg, 0.338 mmol) and 3-iodooxetane (75 mg, 0.408 mmol) in DMF (1 ml) was added K2CO3 (67 mg, 0.676 mmol) and the mixture was stirred at 120°C for 20 hr. The reaction was cooled down and water was added. A solid appeared and was filtrated and analysed by HPLC being the major isomer. The filtrate also contained the desired isomer with the minor one, but was discarded. Once dried the solid weighted 28 mg (41%).

HPLC-MS: tr = 1.28 min. (100 %). LRMS (m/z): 204/206 (M+1)

**Step 43-2: Synthesis of 2-methoxy-3-[1-(oxetan-3-yl)-1,2,4-triazol-3-yl]aniline**

Argon was bubbled through a solution fo 2-methoxy-3-(4,4,5,5-tetramethyl-1,3,2-dioxaborolan-2-yl)aniline (Step 43-1, 40 mg, 0.161 mmol), 3-bromo-1-(oxetan-3-yl)-1,2,4-triazole (Step 1-1, 28 mg, 0.137mmol) in dioxane/H2O (2/0.5 ml). Then Pd(dppf)Cl2 (10 mg, 0.014 mmol) and cesium carbonate (134 mg, 0.411 mmol) were added and the mixture was heated at 100 °C for 1hr. The solution was diluted in ethyl acetate and washed with brine. The organic phase was dried, filtered an concentrated to get an oil. It was purified by flash chromatography to obtain 23 mg (68%) of pure title compound.

HPLC-MS: tr = 1.33 min. (99 %). LRMS (m/z): 247 (M+1)

1H NMR (CDCl3, 400 MHz) δ ppm 3.90 (s, 3H), 5.07-5.13 (m, 2H), 5.14-5.20 (m, 2H), 5.57-5.65 (m, 1H), 7.07-7.12 (t, J = 7.8 Hz, 1H), 7.29 (d, J = 8 Hz, 1H), 8.38 (br s, 1H)

**Step 43-3: Synthesis of N4-(2,6-dimethylpyrimidin-4-yl)-N6-[2-methoxy-3-[1-(oxetan-3-yl)-1,2,4-triazol-3-yl]phenyl]pyrimidine-4,6-diamine**

In a similar way to that described in step 1-2 but replacing the 3-bromo-1-methyl-1,2,4-triazole by the compound obtained in step 43-2 the title compound was obtained as a yellowish solid in 34% yield after purification by flash chromatography by using DCM to 10% MeOH.

HPLC-MS: tr = 1.62 min. (97 %). LRMS (m/z): 446 (M+1)

1H NMR (DMSO, 400 MHz) δ ppm 2.29 (s, 3H), 2.39 (s, 3H), 3.71 (s, 3H), 7.59 (s, 1H), 4.93-5.01 (m, 4H), 5.72-5.80 (m, 1H), 7.20 (s, 1H), 7.22 (t, J = 7.9 Hz, 1H), 7.40 (s, 1H), 7.63 (dd, J = 7.9 and 1.5 Hz, 1H), 7.81 (dd, J = 7.9 and 1.5 Hz, 1H), 8.32 (s, 1H), 8.70 (s, 1H), 8.91 (s, 1H), 10.05 (s, 1H).

**Compounds from Examples 44-49 are prepared in the same way as compound 36 by reacting N-(6-chloropyrimidin-4-yl)-2,6-dimethyl-pyrimidin-4-amine (Step 6-1) with the corresponding intermediate anilines obtained through parallel routes to the previously described.**

**Example 44: N6-(2,6-dimethylpyrimidin-4-yl)-N4-[2-methoxy-3-[1-(2-methoxyethyl)-1,2,4-triazol-3-yl]phenyl]pyrimidine-4,6-diamine**

HPLC-MS: tr = 1.65 min. (99 %). LRMS (m/z): 448 (M+1)

1H NMR (400 MHz, DMSO-d6) δ 2.30 (s, 3H), 2.38 (s, 3H), 3.25 (s, 3H), 3.66 (s, 3H), 3.75 (t, J = 5.2 Hz, 2H), 4.33 - 4.44 (m, 2H), 7.20 (t, J = 7.9 Hz, 2H), 7.38 (s, 1H), 7.59 (dd, J = 7.8, 1.7 Hz, 1H), 7.77 (dd, J = 8.0, 1.6 Hz, 1H), 8.30 - 8.37 (m, 1H), 8.54 (s, 1H), 8.89 (s, 1H), 10.07 (s, 1H).

**Example 45: N4-(2,6-dimethylpyrimidin-4-yl)-N6-[2-ethoxy-3-(1-methyl-1,2,4-triazol-3-**yl)phenyl]pyrimidine-4,6-diamine

HPLC-MS: tr = 1.62 min. (99 %). LRMS (m/z): 452 (M+1)

1H NMR (DMSO, 400 MHz) δ ppm 1.20 (t, J = 7 Hz, 3H), 2.29 (s, 3H), 2.38 (s, 3H), 3.88 (q, J = 7 Hz, 2H), 3.93 (s, 3H), 7.17-7.22 (m, 2H), 7.38 (s, 1H), 7.59 (dd, J = 8 and 1.6 Hz, 1H), 7.71 (dd, J = 8 and 1.6 Hz, 1H), 8.11 (s, 1H), 8.53 (s, 1H), 8.77 (d, J = 1 Hz, 1H), 10.03 (s, 1H).

**Example 46: N4-(2,6-dimethylpyrimidin-4-yl)-N6-[3-(1-ethyl-1,2,4-triazol-3-yl)-2-methoxy-phenyl]pyrimidine-4,6-diamine**

HPLC-MS: tr = 1.72 min. (98 %). LRMS (m/z): 418 (M+1)

1H NMR (400 MHz, DMSO-d6) δ 1.43 - 1.47 (m, 3H), 2.29 (s, 3H), 2.37 (s, 3H), 3.66 (s, 3H), 4.26 (q, J = 7.3 Hz, 2H), 7.20 (q, J = 5.9, 3.9 Hz, 2H), 7.38 (s, 1H), 7.59 (dd, J = 7.8, 1.7 Hz, 1H), 7.76 (dd, J = 8.0, 1.6 Hz, 1H), 8.28 - 8.32 (m, 1H), 8.57 (s, 1H), 8.87 (s, 1H), 10.03 (s, 1H).

**Example 47: N4-(2,6-dimethylpyrimidin-4-yl)-N6-[2-methoxy-3-[1-(tetrahydropyran-2-ylmethyl)-1,2,4-triazol-3-yl]phenyl]pyrimidine-4,6-diamine**

HPLC-MS: tr = 1.95 min. (99 %). LRMS (m/z): 488 (M+1)

1H NMR (400 MHz, DMSO-d6) δ 1.17 - 1.32 (m, 1H), 1.40 - 1.52 (m, 3H), 1.59 - 1.67 (m, 1H), 1.76 - 1.83 (m, 1H), 2.29 (s, 3H), 2.38 (s, 3H), 3.66 (s, 3H), 3.68 - 3.74 (m, 1H), 3.85 (dt, J = 13.2, 2.0 Hz, 2H), 4.23 - 4.27 (m, 2H), 7.16 - 7.23 (m, 2H), 7.38 (s, 1H), 7.59 (dd, J = 7.8, 1.7 Hz, 1H), 7.77 (dd, J = 8.0, 1.6 Hz, 1H), 8.31 (s, 1H), 8.50 (s, 1H), 8.87 (s, 1H), 10.04 (s, 1H).

**Example 48: N4-(2,6-dimethylpyrimidin-4-yl)-N6-[2-methoxy-3-[5-methyl-2-(oxetan-3-yl)-1,2,4-triazol-3-yl]phenyl]pyrimidine-4,6-diamine**

HPLC-MS: tr = 1.65 min. (99 %). LRMS (m/z): 460 (M+1)

1H NMR (DMSO, 400 MHz) δ ppm 2.31 (s, 3H), 2.39 (s, 3H), 2.40 (s, 3H), 3.30 (s, 3H), 4.78 (t, J = 7 Hz, 2H), 4.88 (t, J = 6.3 Hz, 2H), 5.18-5.26 (m, 1H), 7.20 (dd, J = 7.6 and 1,8 Hz, 1H), 7.24-7.30 (m, 2H), 7.35 (s, 1H), 7.91 (dd, J = 8 and 1 Hz, 1H), 8.35 (s, 1H), 9.04 (s, 1H), 10.12 (s, 1H).

**Example 49: N4-(2,6-dimethylpyrimidin-4-yl)-N6-[2-methoxy-3-[5-methyl-1-(oxetan-3-yl)-1,2,4-triazol-3-yl]phenyl]pyrimidine-4,6-diamine**

HPLC-MS: tr = 1.62 min. (99 %). LRMS (m/z): 460 (M+1)

1H NMR (DMSO, 400 MHz) δ ppm 2.29 (s, 3H), 2.39 (s, 3H), 2.43 (s, 3H), 3.73 (s, 3H), 4.88-5.00 (m, 4H), 5.67-5.75 (m, 1H), 7.17-7.23 (m, 2H), 7.40 (s, 1H), 7.62 (dd, J = 8 and 1.5 Hz, 1H), 7.79 (dd, J = 8 and 1.5 Hz, 1H), 8.32 (s, 1H), 8.89 (s, 1H), 10.05 (s, 1H).

**Example 50: N6-(3-bromo-2-methoxy-phenyl)-N4-(2,6-dimethylpyrimidin-4-yl)pyrimidine-4,6-diamine**

By reacting intermediate from step 6-1 with 3-bromo-2-methoxyaniline and by following the method described in step 6-2 the title compound is obtained as a white solid in 46% yield. uPLC-MS: tr = 1.27 min. (98 %). LRMS (m/z): 401/403 (M+1) 1H NMR (400 MHz, Chloroform-d) δ 8.45 (d, J = 0.9 Hz, 1H), 7.88 (dd, J = 8.1, 1.4 Hz, 1H), 7.64 (s, 1H),

7.30 (dd, J = 8.1, 1.4 Hz, 2H), 7.17 (s, 1H), 7.09 - 7.02 (m, 1H), 6.83 (s, 1H), 3.87 (s, 3H), 2.60 (s, 3H), 2.45 (s, 3H).

Compounds from Examples 51-64 are prepared in the same way as compound 36 by reacting N-(6-chloropyrimidin-4-yl)-2,6-dimethyl-pyrimidin-4-amine (Step 6-1) with the corresponding intermediate anilines obtained through parallel routes to the previously described.

**Example 51: N6-(2,6-dimethylpyrimidin-4-yl)-N4-[2-methoxy-3-[1-(oxetan-3-ylmethyl)-1,2,4-triazol-3-yl]phenyl]pyrimidine-4,6-diamine**

HPLC-MS: tr = 1.63 min. (99 %). LRMS (m/z): 460 (M+1)

1H NMR (400 MHz, DMSO-d6) δ 2.29 (s, 3H), 2.38 (s, 3H), 3.44 - 3.53 (m, 1H), 3.65 (s, 3H), 4.48 (t, J = 6.2 Hz, 2H), 4.52 - 4.60 (m, 2H), 4.68 (dd, J = 7.8, 6.2 Hz, 2H), 7.17 - 7.23 (m, 2H), 7.38 (s, 1H), 7.54 - 7.63 (m, 1H), 7.74 - 7.81 (m, 1H), 8.31 (s, 1H), 8.62 (s, 1H), 8.89 (s, 1H), 10.03 (s, 1H).

**Example 52: N4-(2,6-dimethylpyrimidin-4-yl)-N6-[2-methoxy-3-[1-(tetrahydropyran-4-ylmethyl)-1,2,4-triazol-3-yl]phenyl]pyrimidine-4,6-diamine**

HPLC-MS: tr = 1.77 min. (99 %). LRMS (m/z): 488 (M+1)

1H NMR (DMSO, 400 MHz) δ ppm 1.21-1.33 (m, 2H), 1.42-1.48 (m, 2H), 2.06-2.15 (m, 1H), 2.29 (s, 3H), 2.38 (s, 3H), 3.23-3.31 (m, 2H), 3.65 (s, 3H), 3.84 (ddd, J= 11, 4 and 1.6 Hz, 2H), 4.15 (d, J = 7 Hz, 2H), 7.17-7.22 (m, 2H), 7.39 (s, 1H), 7.59 (dd, J = 7.9 and 1.6 Hz, 1H), 7.78 (dd, J = 7.9 and 1.6 Hz, 1H), 8.32 (s, 1H), 8.58 (s, 1H), 8.88 (s, 1H), 10.04 (s, 1H).

**Example 53: N6-[3-[1-[(3,3-difluorocyclobutyl)methyl]-1,2,4-triazol-3-yl]-2-methoxyphenyl]-N4-(2,6-dimethylpyrimidin-4-yl)pyrimidine-4,6-diamine**

HPLC-MS: tr = 1.97 min. (99 %). LRMS (m/z): 494 (M+1)

1H NMR (400 MHz, DMSO-d6) δ 2.29 (s, 3H), 2.38 (s, 3H), 2.62 - 2.77 (m, 5H), 3.66 (s, 3H), 4.38 (d, J = 6.1 Hz, 2H), 7.17 - 7.24 (m, 2H), 7.39 (s, 1H), 7.60 (dd, J = 7.8, 1.7 Hz, 1H), 7.78 (dd, J = 8.0, 1.6 Hz, 1H), 8.29 - 8.32 (m, 1H), 8.63 (s, 1H), 8.89 (s, 1H), 10.04 (s, 1H).

**Example 54: N4-[3-[1-(difluoromethyl)-1,2,4-triazol-3-yl]-2-methoxy-phenyl]-N6-(2,6-dimethylpyrimidin-4-yl)pyrimidine-4,6-diamine**

HPLC-MS: tr = 1.78 min. (97 %). LRMS (m/z): 440 (M+1)

1H NMR (400 MHz, Chloroform-d) δ 2.46 (s, 3H), 2.59 (s, 3H), 3.81 (s, 3H), 6.92 (s, 1H), 7.25 - 7.33 (m, 1H), 7.30 - 7.54 (m, 1H), 7.67 (s, 1H), 7.80 (dd, J = 7.9, 1.5 Hz, 1H), 7.97 - 8.05 (m, 1H), 8.47 (s, 1H), 8.61 (s, 1H).

**Example 55: N6-[3-[1-(2,2-difluorocyclopropyl)-1,2,4-triazol-3-yl]-2-methoxy-phenyl]-N4-(2,6-dimethylpyrimidin-4-yl)pyrimidine-4,6-diamine**

HPLC-MS: tr = 1.83 min. (98 %). LRMS (m/z): 466 (M+1)

1H NMR (400 MHz, DMSO-d6) δ 2.29 (s, 3H), 2.37 (s, 3H), 2.46 (d, J = 4.9 Hz, 1H), 2.54 - 2.59 (m, 1H), 3.65 (s, 3H), 4.66 - 4.82 (m, 1H), 7.21 (dd, J = 12.8, 4.8 Hz, 2H), 7.38 (s, 1H), 7.60 (dd, J = 7.8, 1.7 Hz, 1H), 7.79 (dd, J = 8.0, 1.5 Hz, 1H), 8.31 (s, 1H), 8.81 (s, 1H), 8.90 (s, 1H), 10.04 (s, 1H).

**Example 56: N6-(2,6-dimethylpyrimidin-4-yl)-N4-(2-methoxy-3-tetrahydropyran-2-yl-phenyl)pyrimidine-4,6-diamine**

uPLC-MS: tr = 1.27 min. (100 %). LRMS (m/z): 407 (M+1)

1H NMR (400 MHz, DMSO-d6) δ 10.02 (s, 1H), 8.85 (s, 1H), 8.28 (s, 1H), 7.49 (d, J = 7.8 Hz, 1H), 7.27 (s, 1H), 7.20 (d, J = 4.4 Hz, 2H), 7.11 (t, J = 7.8 Hz, 1H), 4.59 (d, J = 10.8 Hz, 1H), 4.02 (d, J = 10.6 Hz, 2H), 3.64 (s, 3H), 2.38 (s, 3H), 2.29 (s, 3H), 1.76-1.23 (cs, 6H).

**Example 57: N4-(2,6-dimethylpyrimidin-4-yl)-N6-[2-methoxy-3-[1-(tetrahydropyran-3-ylmethyl)-1,2,4-triazol-3-yl]phenyl]pyrimidine-4,6-diamine**

HPLC-MS: tr = 1.83 min. (95 %). LRMS (m/z): 488 (M+1)

1H NMR (400 MHz, DMSO-d6) δ 1.21 - 1.34 (m, 1H), 1.40 - 1.52 (m, 1H), 1.59 - 1.67 (m, 1H), 1.67 - 1.75 (m,

1H), 2.07 - 2.18 (m, 1H), 2.29 (s, 3H), 2.38 (s, 3H), 3.23 (dd, J = 11.0, 8.7 Hz, 1H), 3.66 (s, 3H), 3.67 - 3.75 (m, 2H), 4.17 (dd, J = 7.1, 2.1 Hz, 2H), 7.16 - 7.24 (m, 2H), 7.39 (s, 1H), 7.59 (dd, J = 7.8, 1.7 Hz, 1H), 7.78 (dd, J = 8.0, 1.6 Hz, 1H), 8.32 (s, 1H), 8.58 (s, 1H), 8.88 (s, 1H), 10.04 (s, 1H).

**Example 58: 3-[3-[3-[[6-[(2,6-dimethylpyrimidin-4-yl)amino]pyrimidin-4-yl]amino]-2-methoxy-phenyl]-1,2,4-triazol-1-yl]cyclobutanol**

HPLC-MS: tr = 1.57 min. (97 %). LRMS (m/z): 460 (M+1)

NMR 1H (DMSO, 400 MHz) δ ppm 2.57-2.65 (m, 2H), 3.03-3.10 (m, 2H), 3.95 (s, 3H), 4.29 (p, J = 6.8 Hz, 1H), 4.54 (p, J = 7.6 Hz, 1H), 7.30 (t, J = 8 Hz, 1H), 7.80 (dd, J = 8 and 1.8 Hz, 1H), 8.17 (s, 1H), 8.25 (dd, J = 8 and 1.8 Hz, 1H).

**Example 59: N4-(2,6-dimethylpyrimidin-4-yl)-N6-[2-methoxy-3-[1-(tetrahydrofuran-2-ylmethyl)-1,2,4-triazol-3-yl]phenyl]pyrimidine-4,6-diamine**

HPLC-MS: tr = 1.78 min. (99 %). LRMS (m/z): 474 (M+1)

1H NMR (CDCl3, 400 MHz) δ ppm 1.65-1.86 (m, 3H), 1.94-2.00 (m, 1H), 2.29 (s, 3H), 2.38 (s, 3H), 3.61-3.67 (m, 1H), 3.66 (s, 3H), 3.71-3.78 (m, 1H), 4.19-4.34 (m, 3H), 7.20 (dd, J = 8 and 7.8 Hz, 1H), 7.38 (s, 1H), 7.59 (dd, J = 7.8 and 1.6 Hz, 1H), 7.77 (dd, J = 8 and 1.6 Hz, 1H), 8.31 (s, 1H), 8.53 (s, 1H), 8.88 (s, 1H), 10.05 (s, 1H).

**Example 60: N6-(2,6-dimethylpyrimidin-4-yl)-N4-[2-methoxy-3-[1-(tetrahydrofuran-3-ylmethyl)-1,2,4-triazol-3-yl]phenyl]pyrimidine-4,6-diamine**

HPLC-MS: tr = 1.72 min. (99 %). LRMS (m/z): 474 (M+1)

1H NMR (400 MHz, DMSO-d6) δ 1.59 - 1.77 (m, 1H), 1.87 - 2.05 (m, 1H), 2.29 (s, 1H), 2.37 (s, 1H), 2.76 (dt, J = 13.4, 6.5 Hz, 1H), 3.45 - 3.60 (m, 2H), 3.68 - 3.81 (m, 5H), 4.25 (dd, J = 7.4, 2.5 Hz, 2H), 7.17 - 7.26 (m, 2H), 7.39 (s, 1H), 7.60 (dd, J = 7.8, 1.6 Hz, 1H), 7.75 - 7.81 (m, 1H), 8.31 (s, 1H), 8.62 (s, 1H), 8.88 (s, 1H), 10.04 (s, 1H).

**Example 61: N-(2-((6-((2,6-dimethylpyrimidin-4-yl)amino)pyrimidin-4-yl)amino)-4-methylpyridin-3-yl)-N-methylmethanesulfonamide**

HPLC-MS: tr = 0.84 min. (97.5 %). LRMS (m/z): 415 (M+1)

1H NMR (400 MHz, DMSO-d6) δ 10.29 (s, 1H), 8.86 (s, 1H), 8.42 (d, J = 1.0 Hz, 1H), 8.36 (s, 1H), 8.21 (d, J = 5.0 Hz, 1H), 7.25 (s, 1H), 7.05 (d, J = 5.2 Hz, 1H), 3.26 (s, 3H), 3.19 (s, 3H), 2.36 (s, 3H), 2.33 (s, 3H).

**Example 62: N6-(2,6-dimethylpyrimidin-4-yl)-N4-[2-methoxy-3-[5-(morpholinomethyl)-1,2,4-oxadiazol-3-yl]phenyl]pyrimidine-4,6-diamine**

HPLC-MS: tr = 1.73 min. (99 %). LRMS (m/z): 490 (M+1)

1H NMR (400 MHz, DMSO-d6) δ 10.06 (s, 1H), 9.06 (s, 1H), 8.31 (d, J = 1.0 Hz, 1H), 7.94 (dd, J = 8.1, 1.7 Hz, 1H), 7.63 (dd, J = 7.8, 1.7 Hz, 1H), 7.41 - 7.36 (m, 1H), 7.29 (t, J = 7.9 Hz, 1H), 7.19 (s, 1H), 3.98 (s, 2H), 3.68 (s, 3H), 3.62 - 3.56 (m, 4H), 2.59 - 2.51 (m, 4H), 2.37 (s, 3H), 2.28 (s, 3H).

**Example 63: 6-N-(2,6-dimethylpyrimidin-4-yl)-4-N-[2-methoxy-4-methyl-3-(1-methyl-1,2,4-triazol-3-yl)phenyl]pyrimidine-4,6-diamine**

HPLC-MS: tr = 2.21 min. (100 %). LRMS (m/z): 418 (M+1)

1H NMR (400 MHz, DMSO-d6) δ 10.00 (s, 1H), 8.78 (s, 1H), 8.28 (s, 1H), 7.58 (d, J = 8 Hz, 1H), 7.29 (s, 1H), 7.20 (s, 1H), 7.05 (d, J = 8 Hz, 1H), 3.93 (s, 3H), 3.68 (s, 3H), 3.48 (s, 3H), 2.40 (s, 3H), 2.29 (s, 3H), 2.06 (s, 3H).

**Example 64: N4-(2,6-dimethylpyrimidin-4-yl)-N6-[4-fluoro-2-methoxy-3-(1-methyl-1,2,4-triazol-3-yl)phenyl]pyrimidine-4,6-diamine**

HPLC-MS: tr = 1.58 min. (99 %). LRMS (m/z): 422 (M+1)

1H NMR (DMSO, 400 MHz) δ ppm 2.29 (s, 3H), 2.40 (s, 3H), 3.55 (s, 3H), 3.94 (s, 3H), 7.11 (t, J = 9.2 Hz, 1H), 7.20 (s, 1H), 7.28 (s, 1H), 7.71 (dd, J = 9.2 and 6.4 Hz, 1H), 8.28 (d, J = 1 Hz, 1H), 8.60 (s, 1H), 8.90 (s, 1H), 10.03 (s, 1H).

**Example 65: N4-(5-fluoro-2-pyridyl)-N6-[2-methoxy-3-(1-methyl-1,2,4-triazol-3-yl)phenyl]pyrimidine-4,6-diamine**

**Step 65-1: Synthesis of N4-(5-fluoro-2-pyridyl)-N6-[2-methoxy-3-(1-methyl-1,2,4-triazol-3-yl)phenyl]pyrimidine-4,6-diamine**

Starting with 50 mg (0.158 mmol) of 6-chloro-N-[2-methoxy-3-(1-methyl-1,2,4-triazol-3-yl)phenyl]pyrimidin-4-amine (Step 1-3) and 27 mg (0.240 mmol) of 5-fluoropyridin-2-amine and following the method described in Step 11-1 25 mg (40%) of title compound are obtained as a white solid.

HPLC-MS: tr = 1.75 min. (99 %). LRMS (m/z): 393 (M+1)

NMR 1H (CDCl3, 400 MHz) δ ppm 3.79 (s, 3H), 4.02 (s, 3H), 7.17 (br s, 1H), 7.23 (t, J = 8 Hz, 1H), 7.25 (br s, 1H), 7.36-7.44 (m, 3H), 7.70 (dd, J = 8 and 1.6 Hz, 1H), 8.00 (dd, J = 8 and 1.6 Hz, 1H), 8.12 (s, 1H), 8.15 (d, J = 2.5 Hz, 1H), 8.44 (s, 1H).

**Compounds from Examples 66-79 are prepared in the same way as compounds 15-17 by reacting N-(6-chloropyrimidin-4-yl)-2,6-dimethyl-pyrimidin-4-amine (Step 6-1) with the corresponding anilines.**

**Example 66: N4-(2,6-dimethylpyrimidin-4-yl)-N6-[2-(methylsulfonylmethyl)phenyl]pyrimidine-4,6-diamine**

HPLC-MS: tr = 1.48 min. (99 %). LRMS (m/z): 385 (M+1)

1H NMR (CDCl3, 400 MHz) δ ppm 2.29 (s, 3H), 2.36 (s, 3H), 2.91 (s, 3H), 4.60 (s, 2H), 7.19 (br s, 1H), 7.26 (td, J = 7.5 and 1.1 Hz, 1H), 7.35 (br s, 1H), 7.44 (td, J = 7.8 and 1.5 Hz, 1H), 7.50 (dd, J = 7.6 and 1.4 Hz, 1H), 7.55 (dd, J = 7.8 and 1 Hz, 1H), 8.29 (s, 1H), 8.70 (s, 1H), 10.07 (s, 1H).

**Example 67: N-[5-bromo-2-[[6-[(2,6-dimethylpyrimidin-4-yl)amino]pyrimidin-4-yl]amino]phenyl]-N-methyl-methanesulfonamide**

uPLC-MS: tr = 1.11 min. (100 %). LRMS (m/z): 478,480 (M+1, Br)

1H NMR (400 MHz, DMSO-d6) δ 10.15 (s, 1H), 8.61 (s, 1H), 8.34 (d, J = 0.8 Hz, 1H), 7.83 - 7.78 (m, 2H), 7.57 (dd, J = 8.8, 2.3 Hz, 1H), 7.31 (s, 2H), 3.17 (s, 3H), 3.07 (s, 3H), 2.43 (s, 3H), 2.31 (s, 3H).

**Example 69: N-[2-[[6-[(2,6-dimethylpyrimidin-4-yl)amino]pyrimidin-4-yl]amino]-5-methyl-phenyl]-N-methyl-methanesulfonamide**

uPLC-MS: tr = 0.98 min. (100 %). LRMS (m/z): 414 (M+1)

1H NMR (400 MHz, DMSO-d6) δ 10.07 (s, 1H), 8.36 (s, 1H), 8.29 (d, J = 0.8 Hz, 1H), 7.57 (d, J = 8.2 Hz, 1H), 7.39 (d, J = 1.4 Hz, 1H), 7.30 (s, 1H), 7.24 (s, 1H), 7.23 - 7.17 (m, 1H), 3.14 (s, 3H), 3.03 (s, 3H), 2.40 (s, 3H), 2.34 (s, 3H), 2.30 (s, 3H).

**Example 70: N-[5-cyclopropyl-2-[[6-[(2,6-dimethylpyrimidin-4-yl)amino]pyrimidin-4-yl]amino]phenyl]-N-methyl-methanesulfonamide**

uPLC-MS: tr = 1.14 min. (99 %). LRMS (m/z): 440 (M+1)

1H NMR (400 MHz, DMSO-d6) δ 10.06 (s, 1H), 8.35 (s, 1H), 8.28 (d, J = 0.8 Hz, 1H), 7.54 (d, J = 8.4 Hz, 1H), 7.28 (d, J = 2.2 Hz, 2H), 7.23 (s, 1H), 7.08 (dd, J = 8.4, 2.0 Hz, 1H), 3.15 (s, 3H), 3.02 (s, 3H), 2.39 (s, 3H), 2.30 (s, 3H), 1.96 (ddd, J = 13.4, 8.3, 5.0 Hz, 1H), 1.04 - 0.92 (m, 2H), 0.77 - 0.64 (m, 2H).

**Example 71: N-[2-[[6-[(2,6-dimethylpyrimidin-4-yl)amino]pyrimidin-4-yl]amino]phenyl]-1,1,1-trifluoro-N-methyl-methanesulfonamide**

uPLC-MS: tr = 1.24 min. (90 %). LRMS (m/z): 454 (M+1)

1H NMR (400 MHz, Methanol-d4) δ 8.17 (s, 1H), 7.58 (d, J = 7.7 Hz, 1H), 7.48 - 7.39 (m, 3H), 7.33 - 7.24 (m, 1H), 6.95 (s, 1H), 3.33 (s, 3H), 2.34 (s, 3H), 2.28 (s, 3H).

**Example 72: N4-(2,6-dimethylpyrimidin-4-yl)-N6-[2-(1-methylsulfonylethyl)phenyl]pyrimidine-4,6-diamine**

HPLC-MS: tr = 1.58 min. (99 %). LRMS (m/z): 399 (M+1)

1H NMR (DMSO, 400 MHz) δ ppm 1.63 (d, J = 7 Hz, 3H), 2.29 (s, 3H), 2.33 (s, 3H), 2.76 (s, 3H), 4.68 (q, J = 7 Hz, 1H), 7.19 (br s, 1H), 7.21 (br s, 1H), 7.34 (ddd, J = 8.7, 6.5 and 2.2 Hz, 1H), 7.39-7.46 (m, 2H), 7.61 (dd, J = 7.6

and 1.2 Hz, 1H), 8.27 (d, J = 0.8 Hz, 1H), 8.92 (s, 1H), 10.06 (s, 1H).

**Example 73: N6-(2,6-dimethylpyrimidin-4-yl)-N4-[2-(1-piperidylsulfonyl)phenyl]pyrimidine-4,6-diamine**

uPLC-MS: tr = 1.33 min. (96 %). LRMS (m/z): 440 (M+1)
1H NMR (400 MHz, DMSO-d6) δ 10.22 (s, 1H), 8.71 (s, 1H), 8.40 (d, J = 0.9 Hz, 1H), 8.00 (d, J = 7.4 Hz, 1H), 7.82 (dd, J = 8.0, 1.5 Hz, 1H), 7.75 - 7.69 (m, 1H), 7.55 (s, 1H), 7.38 - 7.29 (m, 1H), 7.23 (s, 1H), 3.02 - 2.90 (m, 4H), 2.43 (s, 3H), 2.32 (s, 3H), 1.42 (s, 4H), 1.37 - 1.30 (m, 2H).

**Example 75: N-[2-[[6-[(2,6-dimethylpyrimidin-4-yl)amino]pyrimidin-4-yl]amino]-4-fluorophenyl]-N-methylmethanesulfonamide**

HPLC-MS: tr = 2.48 min. (95 %). LRMS (m/z): 418 (M+1)
1H NMR (400 MHz, Chloroform-d) δ 8.48 (s, 1H), 8.03 (d, J = 8 Hz, 1H), 7.79 (s, 1H), 7.49 (s, 1H) 7.27 - 7.30 (m, 2H), 6.91 (s, 1H), 6.84.6.82 (m, 1H), 3.27 (s, 3H), 3.00 (s, 3H), 2.62 (s, 3H), 2.46 (s, 3H).

**Example 76: N-[2-[[6-[(2,6-dimethylpyrimidin-4-yl)amino]pyrimidin-4-yl]amino]-6-fluorophenyl]-N-methylmethanesulfonamide**

HPLC-MS: tr = 2.44 min. (95.8 %). LRMS (m/z): 418 (M+1)
1H NMR (400 MHz, Chloroform-d) δ 8.47 (s, 1H), 7.91 (d, J = 8 Hz, 1H), 7.79 (s, 1H), 7.44 (s, 1H) 7.43 - 7.35 (m, 2H), 7.01-6.90 (cs, 2H), 3.30 (s, 3H), 3.04 (s, 3H), 2.61 (s, 3H), 2.47 (s, 3H).

**Example 77: 4-[[6-[(2,6-dimethylpyrimidin-4-yl)amino]pyrimidin-4-yl]amino]-N,N-dimethyl-3-[methyl(methylsulfonyl)amino]benzamide**

HPLC-MS: tr = 2.16 min. (97.4 %). LRMS (m/z): 471 (M+1)
1H NMR (400 MHz, CD3OD) δ 8.36 (s, 1H), 8.07 (d, J = 8 Hz, 1H), 7.69-7.67 (m, 2H), 7.51 (dd, J = 8.1, 2 Hz, 1H), 7.13 (s, 1H), 3.29 (s, 3H), 3.11 (bs, 6H), 3.06 (s, 3H), 2.52 (s, 3H), 2.39 (s, 3H).

**Example 78: [4-[[6-[(2,6-dimethylpyrimidin-4-yl)amino]pyrimidin-4-yl]amino]-3-methoxy-phenyl]-pyrrolidin-1-yl-methanone**

HPLC-MS: tr = 1.03 min. (95.4 %). LRMS (m/z): 420 (M+1)
1H NMR (400 MHz, Methanol-d4) δ 8.26 - 8.21 (m, 1H), 7.87 (d, J = 8.2 Hz, 1H), 7.59 - 7.55 (m, 1H), 7.16 - 7.06 (m, 2H), 6.99 (s, 1H), 3.85 (s, 3H), 3.49 (dt, J = 12.8, 6.7 Hz, 4H), 2.44 (s, 3H), 2.29 (s, 3H), 1.88 (ddt, J = 33.5, 13.0, 6.9 Hz, 4H).

**Example 79: N6-(2,6-dimethylpyrimidin-4-yl)-N4-[2-(1-methyl-1,2,4-triazol-3-yl)phenyl]pyrimidine-4,6-diamine**

HPLC-MS: tr = 1.85 min. (100 %). LRMS (m/z): 374 (M+1)
1H NMR (400 MHz, DMSO-d6) δ 2.32 (s, 3H), 2.50 (s, 3H), 4.00 (s, 3H), 7.13 (t, J = 8.1 Hz, 1H), 7.18 (s, 1H), 7.36 - 7.51 (m, 1H), 7.77 (s, 1H), 8.14 (dd, J = 7.9, 1.6 Hz, 1H), 8.31 - 8.38 (m, 1H), 8.44 (s, 1H), 8.73 (s, 1H), 10.19 (s, 1H), 10.76 (s, 1H).

**Example 80: N6-[4-(1,5-dimethylpyrazol-4-yl)-2-methoxy-phenyl]-N4-(2,6-dimethylpyrimidin-4-yl)pyrimidine-4,6-diamine**

**Step 80-1: Synthesis of 2-methoxy-4-(4,4,5,5-tetramethyl-1,3,2-dioxaborolan-2-yl)aniline**

A mixture of 4-bromo-2-methoxyaniline (1 g; 4.95 mmol), 4,4,5,5-tetramethyl-2-(4,4,5,5-tetramethyl-1,3,2-dioxaborolan-2-yl)-1,3,2-dioxaborolane (1.90 g; 7.48 mmol), cyclopenta-1,4-dien-1-yl(diphenyl)phosphane;dichloromethane;dichloropalladium;iron(2+) (202 mg; 0.247 mmol) and potassium acetate (1.45 g; 14.77 mmol) in 12 mL dioxane is stirred at 100°C in aergon atmosphere for 4hr. Excess water and EtOAc are added. The aqueous layer is extracted with additional EtOAc. The combined organic phases are dried and concentrated. The residue is purified by flash chromatography (hexane/ethyl ether from 0 to 60%) to give 0.93 g (75%) of title compound.
uPLC-MS: tr = 1.51 min. (100 %). LRMS (m/z): 250 (M+1)

**Step 80-2: Synthesis of 4-(1,5-dimethylpyrazol-4-yl)-2-methoxy-aniline**

A mixture of 2-methoxy-4-(4,4,5,5-tetramethyl-1,3,2-dioxaborolan-2-yl)aniline (Step 80-1; 142 mg, 0.57 mmol), 4-bromo-1,5-dimethylpyrazole (100 mg, 0.57 mmol), cyclopenta-1,4-dien-1-yl(diphenyl)phosphane;dichloromethane;dichloropalladium;iron(2+) (93 mg, 0.113 mmol) and 2N solution of cessium carbonate (0.856 mL, 1.71 mmol) in 7 mL of dioxane is stirred at 100°C under argon for 3hr. Excess water and AcOEt are added. The aqueous layer is extracted with additional EtOAc. The combined organic phases are dried and concentrated. The residue is purified by flash chromatography (hexane/ethyl ether from 0 to 100%) to give 53 mg (43%) of title compound.

uPLC-MS: tr = 0.79 min. (100 %). LRMS (m/z): 218 (M+1)

1H NMR (400 MHz, Chloroform-d) δ 7.49 (s, 1H), 6.80 - 6.71 (m, 3H), 3.85 (s, 3H), 3.83 (s, 3H), 2.63 (s, 3H).

**Step 80-3: Synthesis of N6-[4-(1,5-dimethylpyrazol-4-yl)-2-methoxy-phenyl]-N4-(2,6-dimethylpyrimidin-4-yl)pyrimidine-4,6-diamine**

Starting from the compound of Step 80-2 (50 mg, 0.23 mmol) and the compound from Step 6-1 (55 mg, 0.23 mmol) and following the directions described in Step 10-1 10 mg (10%) of title compound are obtained after flash chromatography purification using DCM/MeOH (0-5%). uPLC-MS: tr = 1.05 min. (97.5 %). LRMS (m/z): 417 (M+1)

1H NMR (400 MHz, Chloroform-d) δ 8.44 (d, J = 0.7 Hz, 1H), 8.00 (d, J = 16.8 Hz, 1H), 7.85 (d, J = 8.2 Hz, 1H), 7.63 (s, 1H), 7.55 (s, 1H), 7.31 (s, 1H), 6.99 (dd, J = 8.2, 1.8 Hz, 1H), 6.93 (d, J = 1.8 Hz, 1H), 6.85 (s, 1H), 3.92 (s, 3H), 3.86 (s, 3H), 2.58 (s, 3H), 2.43 (s, 3H), 2.40 (s, 3H).

**Compounds from Examples 81-100 are prepared in the same way as compound 80 by reacting N-(6-chloropyrimidin-4-yl)-2,6-dimethyl-pyrimidin-4-amine (Step 6-1) with the corresponding anilines.**

**Example 81: N4-(2,6-dimethylpyrimidin-4-yl)-N6-[4-(5-fluoropyrimidin-2-yl)-2-methoxyphenyl]pyrimidine-4,6-diamine**

uPLC-MS: tr = 1.24 min. (97 %). LRMS (m/z): 419 (M+1)

1H NMR (400 MHz, Chloroform-d) δ 8.64 (s, 2H), 8.48 (d, J = 0.9 Hz, 1H), 8.15 - 8.06 (m, 2H), 8.01 (d, J = 1.4 Hz, 1H), 7.66 (s, 1H), 7.42 (d, J = 5.7 Hz, 1H), 7.37 (s, 1H), 6.89 (s, 1H), 4.04 (s, 3H), 2.64 (s, 3H), 2.45 (s, 3H).

**Example 82: N6-(2,6-dimethylpyrimidin-4-yl)-N4-[2-methoxy-4-[1-methyl-5-(trifluoromethyl)-1,2,4-triazol-3-yl]phenyl]pyrimidine-4,6-diamine**

uPLC-MS: tr = 1.33 min. (98 %). LRMS (m/z): 472 (M+1)

1H NMR (400 MHz, Methanol-d4) δ 8.18 (s, 2H), 7.79 (d, J = 7.3 Hz, 2H), 7.61 -7.51 (m, 9H), 7.33 (dd, J = 15.9, 8.0 Hz, 1H), 7.17 - 7.08 (m, 1H), 6.90 (s, 2H), 4.01 (s, 3H), 3.86 (s, 3H), 2.38 (s, 3H), 2.25 (s, 3H).

**Example 83: N6-(2,6-dimethylpyrimidin-4-yl)-N4-[2-methoxy-3-methyl-4-(1-methyl-1,2,4-triazol-3-yl)phenyl]pyrimidine-4,6-diamine**

HPLC-MS: tr = 1.68 min. (96 %). LRMS (m/z): 418 (M+1)

1H NMR (400 MHz, DMSO-d6) δ 2.30 (s, 3H), 2.42 (s, 3H), 3.66 (s, 3H), 3.93 (s, 3H), 7.24 (s, 1H), 7.42 (s, 1H), 7.61 (d, J = 8.6 Hz, 1H), 7.73 (d, J = 8.6 Hz, 1H), 8.33 - 8.35 (m, 1H), 8.51 - 8.52 (m, 1H), 8.89 (s, 1H), 10.07 (s, 1H).

**Example 84: N4-[4-(1,5-dimethylpyrazol-4-yl)-2-methoxy-3-methyl-phenyl]-N6-(2,6-dimethylpyrimidin-4-yl)pyrimidine-4,6-diamine**

HPLC-MS: tr = 1.88 min. (99 %). LRMS (m/z): 431 (M+1)

1H NMR (400 MHz, DMSO-d6) δ 10.02 (s, 1H), 8.81 (s, 1H), 8.30 (d, J = 1.0 Hz, 1H), 7.52 (d, J = 8.3 Hz, 1H), 7.37 (d, J = 1.1 Hz, 1H), 7.34 (s, 1H), 7.19 (s, 1H), 6.90 (d, J = 8.3 Hz, 1H), 3.79 (s, 3H), 3.66 (s, 3H), 2.39 (s, 3H), 2.30 (s, 3H), 2.16 (s, 3H), 2.13 (s, 3H).

**Example 85: N6-(2,6-dimethylpyrimidin-4-yl)-N4-[2-methoxy-4-(1-methyl-1,2,4-triazol-3-yl)phenyl]pyrimidine-4,6-diamine**

uPLC-MS: tr = 0.89 min. (99 %). LRMS (m/z): 404 (M+1)

1H NMR (400 MHz, Chloroform-d) δ 8.45 (d, J = 0.8 Hz, 1H), 8.06 (s, 1H), 8.00 (d, J = 8.3 Hz, 1H), 7.78 (dd,

J = 8.3, 1.7 Hz, 1H), 7.69 (d, J = 1.7 Hz, 1H), 7.61 (s, 1H), 7.30 (d, J = 6.4 Hz, 2H), 6.90 (s, 1H), 4.01 (s, 3H), 4.00 (s, 3H), 2.62 (s, 3H), 2.45 (s, 3H).

**Example 86: N4-[4-(1,5-dimethylpyrazol-4-yl)-2-methylsulfonyl-phenyl]-N6-(2,6-dimethylpyrimidin-4-yl)pyrimidine-4,6-diamine**

HPLC-MS: tr = 1.77 min. (99%).vLRMS (m/z): 465 (M+1)

1H NMR (400 MHz, DMSO-d6) δ 2.31 (s, 3H), 2.38 - 2.44 (m, 6H), 3.25 (s, 3H), 3.81 (s, 3H), 7.24 (s, 1H), 7.47 (s, 1H), 7.66 (s, 1H), 7.77 (dd, J = 8.4, 2.3 Hz, 1H), 7.86 - 7.91 (m, 2H), 8.36 (s, 1H), 8.90 (s, 1H), 10.20 (s, 1H).

**Example 87: N-[2-[[6-[(2,6-dimethylpyrimidin-4-yl)amino]pyrimidin-4-yl]amino]-5-[2-methyl-5-(trifluoromethyl)pyrazol-3-yl]-3-pyridyl]-N-methyl-methanesulfonamide**

HPLC-MS: tr = 2.12 min. (99 %). LRMS (m/z): 549 (M+1).

1H NMR (400 MHz, DMSO-d6) δ 2.33 (s, 3H), 2.53 (s, 3H), 3.20 (s, 3H), 3.29 (s, 3H), 4.00 (s, 3H), 7.08 (s, 1H), 7.24 (s, 1H), 8.29 (d, J = 2.1 Hz, 1H), 8.46 - 8.48 (m, 1H), 8.55 - 8.57 (m, 1H), 8.58 (s, 1H), 9.06 (s, 1H), 10.38 (s, 1H).

**Example 88: N-[2-[[6-[(2,6-dimethylpyrimidin-4-yl)amino]pyrimidin-4-yl]amino]-5-(1,3,5-trimethylpyrazol-4-yl)-3-pyridyl]-N-methyl-methanesulfonamide**

HPLC-MS: tr = 1.74 min. (98.4 %). LRMS (m/z): 509 (M+1).

1H NMR (400 MHz, DMSO-d6) δ 10.31 (s, 1H), 9.03 (s, 1H), 8.45 (s, 1H), 8.42 (d, J = 1.1 Hz, 1H), 8.25 (d, J = 2.1 Hz, 1H), 7.95 (d, J = 2.1 Hz, 1H), 7.21 (s, 1H), 3.73 (s, 3H), 3.27 (s, 3H), 3.17 (s, 3H), 2.52 (s, 3H), 2.33 (s, 3H), 2.27 (s, 3H), 2.18 (s, 3H).

**Example 89: N-[2-[[6-[(2,6-dimethylpyrimidin-4-yl)amino]pyrimidin-4-yl]amino]-5-(5-methyl-1H-pyrazol-4-yl)phenyl]-N-methyl-methanesulfonamide**

uPLC-MS: tr = 1.32 min. (86 %). LRMS (m/z): 480 (M+1).

**Example 90: N4-(2,6-dimethylpyrimidin-4-yl)-N6-[2-methoxy-4-(1-methylimidazol-4-yl)phenyl]pyrimidine-4,6-diamine**

uPLC-MS: tr = 0.68 min. (87 %). LRMS (m/z): 403 (M+1).

1H NMR (400 MHz, Chloroform-d) δ 8.42 (d, J = 0.8 Hz, 1H), 7.82 (d, J = 8.2 Hz, 1H), 7.50 (dd, J = 5.2, 3.5 Hz, 2H), 7.30 (dd, J = 8.2, 1.7 Hz, 2H), 7.20 - 7.14 (m, 1H), 6.92 (s, 1H), 5.30 (s, 1H), 3.97 (s, 3H), 3.75 (s, 3H), 2.59 (s, 3H), 2.44 (s, 3H).

**Example 91: N4-(2,6-dimethylpyrimidin-4-yl)-N6-[2-methoxy-4-(1-methylimidazol-2-yl)phenyl]pyrimidine-4,6-diamine**

uPLC-MS: tr = 0.65 min. (99 %). LRMS (m/z): 403 (M+1).

1H NMR (400 MHz, Chloroform-d) δ 8.47 (s, 1H), 8.12 (d, J = 8.3 Hz, 1H), 7.57 (s, 1H), 7.42 - 7.29 (m, 3H), 7.21 - 7.11 (m, 2H), 6.98 (d, J = 1.0 Hz, 1H), 6.91 (s, 1H), 3.98 (s, 3H), 3.79 (s, 3H), 2.62 (s, 3H), 2.45 (s, 3H).

**Example 92: N-[5-(3,4-dihydro-2H-pyran-6-yl)-2-[[6-[(2,6-dimethylpyrimidin-4-yl)amino]pyrimidin-4-yl]amino]phenyl]-N-methylmethanesulfonamide**

uPLC-MS: tr = 1.30 min. (99 %). LRMS (m/z): 482 (M+1).

1H NMR (400 MHz, DMSO-d6) δ 10.13 (s, 1H), 8.49 (s, 1H), 8.34 (s, 1H), 7.79 (d, J = 8.6 Hz, 1H), 7.63 (d, J = 2.0 Hz, 1H), 7.53 (dd, J = 8.6, 2.0 Hz, 1H), 7.34 (s, 1H), 7.29 (s, 1H), 5.50 (t, J = 4.0 Hz, 1H), 4.17 - 4.10 (m, 2H), 3.17 (s, 3H), 3.06 (s, 3H), 2.43 (s, 3H), 2.31 (s, 3H), 2.19 (q, J = 6.2 Hz, 2H), 1.90 - 1.81 (m, 2H).

**Example 93: N6-(2,6-dimethylpyrimidin-4-yl)-N4-[2-methoxy-4-(5-methyl-1,2,4-oxadiazol-3-yl)phenyl]pyrimidine-4,6-diamine**

uPLC-MS: tr = 1.17 min. (100 %). LRMS (m/z): 405 (M+1).

1H NMR (400 MHz, Methanol-d4) δ 8.25 (s, 1H), 7.97 (d, J = 8.8 Hz, 1H), 7.63 - 7.55 (m, 3H), 6.99 (s, 1H),

5.39 (s, 1H), 4.49 (s, 3H), 3.89 (s, 3H), 2.56 (s, 3H), 2.43 (s, 3H).

**Example 94: N-[5-(3,6-dihydro-2H-pyran-5-yl)-2-[[6-[(2,6-dimethylpyrimidin-4-yl)amino]pyrimidin-4-yl]amino]phenyl]-N-methyl-methanesulfonamide**

uPLC-MS: tr = 1.08 min. (100 %). LRMS (m/z): 482 (M+1).

1H NMR (400 MHz, DMSO-d6) δ 10.13 (s, 1H), 8.45 (s, 1H), 8.33 (d, J = 0.8 Hz, 1H), 7.77 (d, J = 8.5 Hz, 1H), 7.53 (d, J = 2.1 Hz, 1H), 7.43 - 7.32 (m, 2H), 7.28 (s, 1H), 6.34 (dt, J = 4.1, 2.3 Hz, 1H), 4.52 - 4.41 (m, 2H), 3.75 (t, J = 5.5 Hz, 2H), 3.19 (s, 3H), 3.05 (s, 3H), 2.42 (s, 3H), 2.31 (s, 3H), 2.28 (d, J = 4.1 Hz, 2H), 1.24 (s, 1H).

**Example 95: N-[5-(3,6-dihydro-2H-pyran-4-yl)-2-[[6-[(2,6-dimethylpyrimidin-4-yl)amino]pyrimidin-4-yl]amino]phenyl]-N-methyl-methanesulfonamide**

uPLC-MS: tr = 1.06 min. (97 %). LRMS (m/z): 482 (M+1).

1H NMR (400 MHz, DMSO-d6) δ 10.13 (s, 1H), 8.46 (s, 1H), 8.33 (d, J = 0.7 Hz, 1H), 7.77 (d, J = 8.5 Hz, 1H), 7.60 (d, J = 2.1 Hz, 1H), 7.47 (dd, J = 8.5, 2.1 Hz, 1H), 7.36 (s, 1H), 7.28 (s, 1H), 6.31 (s, 1H), 4.25 (d, J = 2.7 Hz, 2H), 3.84 (t, J = 5.4 Hz, 2H), 3.30 (s, 2H), 3.20 (s, 3H), 3.06 (s, 3H), 2.42 (s, 3H), 2.31 (s, 3H).

**Example 96: N4-[4-(1,5-dimethylpyrazol-4-yl)-2-fluoro-6-methylsulfonyl-phenyl]-N6-(2,6-dimethylpyrimidin-4-yl)pyrimidine-4,6-diamine**

HPLC-MS: tr = 1.73 min. (99 %). LRMS (m/z): 483 (M+1)

1H NMR (400 MHz, DMSO-d6) δ 2.31 (s, 3H), 2.41 (s, 3H), 2.44 (s, 3H), 3.21 (s, 3H), 3.82 (s, 3H), 7.25 (s, 1H), 7.30 (s, 1H), 7.71 - 7.79 (m, 3H), 8.24 (s, 1H), 9.02 (s, 1H), 10.14 (s, 1H).

**Example 97: N-[2-[[6-[(2,6-dimethylpyrimidin-4-yl)amino]pyrimidin-4-yl]amino]-5-(1,5-dimethyl-1,2,4-triazol-3-yl)phenyl]-N-methyl-methanesulfonamide**

uPLC-MS: tr = 0.89 min. (100 %). LRMS (m/z): 495 (M+1).

1H NMR (400 MHz, Methanol-d4) δ 8.23 (s, 1H), 8.03 (d, J = 1.7 Hz, 1H), 8.00 - 7.84 (m, 2H), 7.59 (s, 1H), 6.98 (s, 1H), 4.49 (s, 3H), 3.80 (s, 3H), 2.99 (s, 3H), 2.41 (s, 3H), 2.40 (s. 3H), 2.28 (s, 3H).

**Example 98: N-[5-(1,5-dimethylpyrazol-4-yl)-2-[[6-[(2,6-dimethylpyrimidin-4-yl)amino]pyrimidin-4-yl]amino]phenyl]-N-(trideuteriomethyl)methanesulfonamide**

HPLC-MS: tr = 1.78 min. (99 %). LRMS (m/z): 497 (M+1)

1H NMR (400 MHz, DMSO-d6) δ 2.28 (s, 3H), 2.30 (s, 3H), 2.40 (s, 3H), 3.05 (s, 3H), 3.78 (s, 3H), 7.22 - 7.27 (m, 1H), 7.32 (s, 2H), 7.57 (d, J = 8.3 Hz, 1H), 7.63 (s, 1H), 7.73 - 7.79 (m, 1H), 8.33 (s, 1H), 8.47 (s, 1H), 10.14 (s, 1H).

**Example 99: N-[2-[[6-[(2,6-dimethylpyrimidin-4-yl)amino]pyrimidin-4-yl]amino]-5-(2,5-dimethyl-1,2,4-triazol-3-yl)phenyl]-N-methyl-methanesulfonamide**

HPLC-MS: tr = 0.91 min. (99 %). LRMS (m/z): 495 (M+1)

1H NMR (400 MHz, Methanol-d4) δ 8.28 (d, J = 0.9 Hz, 1H), 8.15 (d, J = 8.6 Hz, 1H), 7.79 (d, J = 2.0 Hz, 1H), 7.69 - 7.59 (m, 2H), 7.04 (s, 1H), 4.49 (s, 3H), 3.87 (s, 3H), 3.01 (s, 3H), 2.44 (s, 3H), 2.29 (s, 3H), 2.28 (s, 3H).

**Example 100: N-[2,4-dimethyl-6-[[6-[(6-methylpyrimidin-4-yl)amino]pyrimidin-4-yl]amino]phenyl]-N-methyl-methanesulfonamide**

HPLC-MS: tr = 2.48 min. (96.3 %). LRMS (m/z): 414 (M+1)

1H NMR (400 MHz, Chloroform-d) δ 8.69 (s, 1H), 8.42 (s, 1H), 7.44 (d, J = 8Hz, 1H), 7.15 (s, 1H), 6.94 (s, 1H), 3.18 (s, 3H), 3.05 (s, 3H), 2.49 (s, 3H), 2.37 (s, 3H).

**Example 101: N-[2-[[6-[(5-fluoro-4-methyl-2-pyridyl)amino]pyrimidin-4-yl]amino]phenyl]-N-methyl-methanesulfonamide**

**Step 101-1: Synthesis of N-[2-[(6-chloropyrimidin-4-yl)amino]phenyl]-N-methyl-methanesulfonamide**

A mixture of 4,6-dichloropyrimidine (148 mg; 0.993 mmol), N-(2-aminophenyl)-N-methyl-methanesulfonamide (Step 12-2; 199 mg, 0.993 mmol) and silver trifluoromethanesulphonate (255 mg, 0.993 mmol) in 4 mL dioxane is subjected to microwave irradiation at 120°C for 50 min. The mixture was evaporated and taken up in EtOAc, filtered through Celite® washing with more EtOAc. The filtrates are concentrated and the residue purificated by flash chromatography (0-100% EtOAc in hexanes) to give the title compound (180 mg; 58%) of the title compound as a cream coloured solid.

uPLC-MS: tr = 1.21 min. (97 %). LRMS (m/z): 313 (M+1, Cl)

1H NMR (400 MHz, DMSO-d6) δ 9.15 (s, 1H), 8.40 (d, J = 0.8 Hz, 1H), 7.84 (dd, J = 8.2, 1.4 Hz, 1H), 7.57 (dd, J = 8.0, 1.5 Hz, 1H), 7.43 - 7.35 (m, 1H), 7.30 - 7.21 (m, 1H), 6.96 (s, 1H), 3.15 (s, 3H), 3.02 (s, 3H).

**Step 101-2: Synthesis of N-[2-[[6-[(5-fluoro-4-methyl-2-pyridyl)amino]pyrimidin-4-yl]amino]phenyl]-N-methyl-methanesulfonamide**

To a solution of N-[2-[(6-chloropyrimidin-4-yl)amino]phenyl]-N-methyl-methanesulfonamide (Step 101-1; 46 mg, 0.15 mmol) in 1.5 mL of anh. dioxane was added, under Ar, 5-fluoro-4-methyl-pyridin-2-amine (19 mg, 0.15 mmol), Pd2(dba)3 (14 mg, 0.015 mmol), Xantphos (17 mg, 0.029 mmol) and cesium carbonate (144 mg, 0.44 mmol) and the mixture was heated at 100 °C. for 1.5 hr. Once at room temperature EtOAc was added and the solution was washed with brine, dried over anh. MgSO4, filtered and concentrated. The crude was purified via flash chromatography in CH2Cl2/CH2Cl2 with 10 % MeOH to have a white solid (33 mg, 56 %).

uPLC-MS: tr = 1.98 min. (99%). LRMS (m/z): 403 (M+1).

1H NMR (400 MHz, DMSO-d6) δ 2.25 (s, 3H), 3.04 (s, 3H), 3.17 (s, 3H), 7.12 - 7.21 (m, 2H), 7.37 (t, J = 7.8 Hz, 1H), 7.50 - 7.56 (m, 2H), 7.83 - 7.89 (m, 1H), 8.12 (s, 1H), 8.27 (s, 1H), 8.34 (s, 1H), 9.78 (s, 1H).

**Compounds from Examples 102-104 are prepared in the same way as compound 101 by reacting the corresponding aryl amines with the different chloropyrimidine derivatives as in step 101-2.**

**Example 102: N-[2-[[6-[(5-cyano-2-pyridyl)amino]pyrimidin-4-yl]amino]phenyl]-N-methyl-methanesulfonamide**

HPLC-MS: tr = 1.97 min. (98%). LRMS (m/z): 396 (M+1).

1H NMR (400 MHz, DMSO-d6) δ 10.41 (s, 1H), 8.68 (dd, J = 2.3, 0.8 Hz, 1H), 8.61 (s, 1H), 8.33 (d, J = 1.0 Hz, 1H), 8.09 (dd, J = 8.8, 2.3 Hz, 1H), 7.84 (dd, J = 8.2, 1.5 Hz, 1H), 7.76 (dd, J = 8.9, 0.8 Hz, 1H), 7.55 (dd, J = 8.0, 1.5 Hz, 1H), 7.38 (td, J = 7.8, 1.6 Hz, 1H), 7.29 (d, J = 1.1 Hz, 1H), 7.20 (td, J = 7.6, 1.5 Hz, 1H), 3.17 (s, 3H), 3.03 (s, 3H).

**Example 103: N-[5-(1,5-dimethylpyrazol-4-yl)-2-[[6-[(5-fluoro-4-methyl-2-pyridyl)amino]pyrimidin-4-yl]amino]-3-pyridyl]-N-methyl-methanesulfonamide**

HPLC-MS: tr = 2.10 min. (99%). LRMS (m/z): 498 (M+1).

NMR 1H (DMSO, 400 MHz) δ ppm 2.27 (s, 3H), 2.41 (s, 3H), 3.18 (s, 3H), 3.27 (s, 3H), 3.81 (s, 3H), 7.67 (d, J = 5.6 Hz, 1H), 7.70 (s, 1H), 8.00 (d, J = 2 Hz, 1H), 8.20 (d, J = 0.04Hz, 1H), 8.31 (s, 1H), 8.35 (d, J = 0.8 Hz, 1H), 8.38 (d, J = 2 Hz, 1H), 8.51 (s, 1H), 9.98 (s, 1H).

**Example 104: 4-N-[2-methoxy-5-methyl-3-(1-methyl-1,2,4-triazol-3-yl)phenyl]-6-N-(6-methylpyrimidin-4-yl)pyrimidine-4,6-diamine**

HPLC-MS: tr = 2.22 min. (97%). LRMS (m/z): 404 (M+1).

1H NMR (400 MHz, DMSO-d6) δ 10.13 (s, 1H), 8.86 (s, 1H), 8.61 (s. 1H), 8.53 (s, 1H), 8.35 (s, 1H), 7.67-7.63 (bs, 1H), 7.52-7.48 (bs, 1H), 7.39 (s, 1H), 7.29 (s, 1H), 3.94 (s, 3H), 3.64 (s, 3H), 2.36 (s, 3H), 2.32 (s, 3H).

**Example 105: N-[2-[[6-[(2,6-dimethylpyrimidin-4-yl)amino]pyrimidin-4-yl]amino]-5-tetrahydropyran-3-yl-phenyl]-N-methyl-methanesulfonamide**

**Step 105-01: Synthesis of N-[2-[[6-[(2,6-dimethylpyrimidin-4-yl)amino]pyrimidin-4-yl]amino]-5-tetrahydropyran-3-yl-phenyl]-N-methyl-methanesulfonamide**

A mixture of N-[5-(3,6-dihydro-2H-pyran-5-yl)-2-[[6-[(2,6-dimethylpyrimidin-4-yl)amino]pyrimidin-4-yl]amino]phenyl]-N-methyl-methanesulfonamide (Example 94; 37 mg, 0.076 mmol) and 10% palladium on carbon (8.2 mg 0.007 mmol) in 1.5 mL of metanol is hydrogenated at atmospheric pressure for 24 hr. An additional amount of catalyst is added and the hydrogenation prosecuted for 48 hr. After filtering the catalyst and concentrating the residue is purified through reverse phase chromatography (water to MeCN/MeOH 1:1 100%) to give 26 mg (70%) of title compound as a white solid.

uPLC-MS: tr = 1.08 min. (100%). LRMS (m/z): 484 (M+1).

1H NMR (400 MHz, DMSO-d6) δ 10.09 (s, 1H), 8.38 (s, 1H), 8.30 (s, 1H), 7.63 (d, J = 8.3 Hz, 1H), 7.48 (d, J = 1.8 Hz, 1H), 7.36 - 7.27 (m, 2H), 7.23 (s, 1H), 3.87 (dd, J = 9.9, 5.3 Hz, 2H), 3.45 - 3.33 (m, 3H), 3.16 (s, 3H), 3.03 (s, 3H), 2.87 - 2.77 (m, 1H), 2.40 (s, 3H), 2.30 (s, 3H), 1.84 - 1.61 (m, 3H).

**Example 106: N-[2-[[6-[(2,6-dimethylpyrimidin-4-yl)amino]pyrimidin-4-yl]amino]-5-tetrahydropyran-4-yl-phenyl]-N-methyl-methanesulfonamide**

This compound was obtained from the compound described in example 95 in a similar way to that described for compound 105 uPLC-MS: tr = 1.04 min. (99%). LRMS (m/z): 484 (M+1).

1H NMR (400 MHz, DMSO-d6) δ 10.09 (s, 1H), 8.37 (s, 1H), 8.30 (s, 1H), 7.63 (d, J = 8.3 Hz, 1H), 7.47 (d, J = 2 Hz, 1H), 7.35 (s, 1H), 7.28 (d, J = 2Hz, 1H), 7.21 (s, 1H), 4.00-3.92 (m, 2H), 3.48 - 3.40 (m, 2H), 3.18 (s, 3H), 3.03 (s, 3H), 2.39 (s, 3H), 2.30 (s, 3H), 1.75 - 1.65 (m, 4H).

**Example 107: N-[2-[[6-[(2,6-dimethylpyrimidin-4-yl)amino]pyrimidin-4-yl]amino]-5-tetrahydropyran-2-yl-phenyl]-N-methyl-methanesulfonamide**

**Step 107-1: Synthesis of N-[5-(3,4-dihydro-2H-pyran-6-yl)-2-nitro-phenyl]-N-methyl-methanesulfonamide**

A mixtrure of N-(5-bromo-2-nitro-phenyl)-N-methyl-methanesulfonamide (1.01 g, 3.26 mmol), 2-(3,4-dihydro-2H-pyran-6-yl)-4,4,5,5-tetramethyl-1,3,2-dioxaborolane (1.02 g, 4.85 mmol), 2N solution of cesium carbonate (4.9 mL, 9.8 mmol), cyclopenta-1,4-dien-1-yl(diphenyl)phosphane;dichloromethane;dichloropalladium;iron(2+) (79 mg, 0.096 mmol) in 30 mL dioxane is stirred at 100°C for 2.5 hr. After concentration the residue is partitioned in EtOAc/water and the organic phase washed with brine, dried and concentrated. The residue is purified by flash chromatography (0-70% DCM in hexanes) to give the title compound (741 mg, 73%) as a pale yellow solid.

uPLC-MS: tr = 1.51 min. (99%). LRMS (m/z): 313 (M+1).

1H NMR (400 MHz, Chloroform-d) δ 7.89 (d, J = 8.6 Hz, 1H), 7.70 (d, J = 1.9 Hz, 1H), 7.62 (dd, J = 8.6, 1.9 Hz, 1H), 5.58 (t, J = 4.2 Hz, 1H), 4.23 - 4.16 (m, 2H), 3.32 (s, 3H), 3.01 (s, 3H), 2.26 (td, J = 6.4, 4.3 Hz, 2H), 1.98 - 1.88 (m, 2H).

**Step 107-2: Synthesis of N-(2-amino-5-tetrahydropyran-2-yl-phenyl)-N-methyl-methanesulfonamide**

A mixture of N-[5-(3,4-dihydro-2H-pyran-6-yl)-2-nitro-phenyl]-N-methyl-methanesulfonamide (Step 107-1; 731 mg, 2.35 mmol) and palladium hydroxide 50% in water (50 mg, 0.035 mmol) in 20 mL of metanol is hydrogenated at atmospheric pressure for 2hr. After filtration and concentration the residue is purified by flash chromatography (MeOH 0-2% in DCM) to give 528 mg (79%) of title compound as a white solid.

uPLC-MS: tr = 1.19 min. (100%). LRMS (m/z): 285 (M+1).

1H NMR (400 MHz, Chloroform-d) δ 7.16 - 7.06 (m, 2H), 6.76 (d, J = 8.3 Hz, 1H), 4.20 (dd, J = 10.8, 2.0 Hz, 1H), 4.14 - 4.06 (m, 1H), 3.58 (td, J = 11.5, 2.7 Hz, 1H), 3.23 (s, 3H), 2.97 (s, 3H), 1.96 - 1.88 (m, 1H), 1.83 - 1.75 (m, 1H), 1.59 (qdd, J = 27.5, 11.7, 3.9 Hz, 6H).

**Step 107-3: Synthesis of N-[2-[[6-[(2,6-dimethylpyrimidin-4-yl)amino]pyrimidin-4-yl]amino]-5-tetrahydropyran-2-yl-phenyl]-N-methyl-methanesulfonamide**

The compound of Step 107-2 is reacted with the compound of the step 6-1 using the method described in step 10-1 to give the title compound in 45% yield.

uPLC-MS: tr = 1.20 min. (98%). LRMS (m/z): 484 (M+1).

1H NMR (400 MHz, DMSO-d6) δ 8.47 (s, 1H), 8.32 (s, 1H), 7.68 (d, J = 8.4 Hz, 1H), 7.48 (d, J = 1.7 Hz, 1H), 7.37 - 7.29 (m, 2H), 4.34 (d, J = 10.5 Hz, 1H), 4.04 (d, J = 11.3 Hz, 1H), 3.60 - 3.49 (m, 1H), 3.16 (s, 3H), 3.03 (s, 3H), 2.42 (s, 3H), 2.32 (s, 3H), 1.85 (d, J = 12.1 Hz, 1H), 1.66 - 1.54 (m, 2H), 1.52 - 1.38 (m, 1H).

**Example 108: N-[2-[[6-[(2,6-dimethylpyrimidin-4-yl)amino]pyrimidin-4-yl]amino]-5-vinyl-phenyl]-N-methyl-methanesulfonamide**

Starting from N-[5-bromo-2-[[6-[(2,6-dimethylpyrimidin-4-yl)amino]pyrimidin-4-yl]amino]phenyl]-N-methyl-methanesulfonamide (Example 67) and 2-ethenyl-4,4,5,5-tetramethyl-1,3,2-dioxaborolane and following the procedure described in step 107-1 the title compound is obtained in 60% yield as a white solid

uPLC-MS: tr = 1.10 min. (100%). LRMS (m/z): 426 (M+1).

1H NMR (400 MHz, DMSO-d6) δ 10.13 (s, 1H), 8.50 (s, 1H), 8.34 (d, J = 0.8 Hz, 1H), 8.25 (s, 1H), 7.78 (d, J = 8.4 Hz, 1H), 7.66 (d, J = 1.9 Hz, 1H), 7.50 (dd, J = 8.5, 1.9 Hz, 1H), 7.36 (s, 1H), 7.28 (s, 1H), 6.74 (dd, J = 17.6, 11.0 Hz, 1H), 5.86 (d, J = 17.6 Hz, 1H), 5.28 (d, J = 11.5 Hz, 1H), 3.17 (s, 3H), 3.06 (s, 3H), 2.42 (s, 3H), 2.31 (s, 3H).

**Example 109: N-[5-benzyl-2-[[6-[(2,6-dimethylpyrimidin-4-yl)amino]pyrimidin-4-yl]amino]phenyl]-N-methyl-methanesulfonamide**

**Step 109-1: Synthesis of N-[5-benzyl-2-[[6-[(2,6-dimethylpyrimidin-4-yl)amino]pyrimidin-4-yl]amino]phenyl]-N-methyl-methanesulfonamide**

A Schlenck tube was charged with NiCl2•glyme (66 mg, 0.3 mmol) and 4,4'-di-tert-butyl-2,2'-bipyridine (80 mg, 0.3 mmol). DMF (6.0 mL) was added and the resulting mixture was stirred at room temperature under argon atmosphere overnight to give a homogeneous green solution. Concentration: 0,05M solution of catalyst in DMF. A mixture of 62.7 uL of this solution, potassium benzyl(trifluoro)boranuide (26 mg, 0.13 mmol), N-[5-bromo-2-[[6-[(2,6-dimethylpyrimidin-4-yl)amino]pyrimidin-4-yl]amino]phenyl]-N-methyl-methanesulfonamide

(Example 67; 50 mg, 0.104 mmol), 2,6-dimethylpyridine (43 uL, 0.369 mmol) and (Ir[dF(CF3)ppy]2(dtbpy))PF6 (CAS:870987-63-6; 1.2 mg, 0.107 mmol) in 1 mL DMFis degassed by bubbling an argon stream for 15 min and subjected to a LED intensity of 100% in a Penn phD Photoreactor M2 apparatus for 6hr. Further amounts of fluoborate salt, catalyst and lutidine were added and the reaction prosecuted for a further 6hr. The reaction mixture was partitioned between water and EtOAc and the organic layer was washed with water, brine, dried and concentrated. The residue was purified by reverse phase chromatography (0% to 100% water-acetonitrile/MeOH (1:1) (0.1% v/v ammonium formate both phases)) to give 18 mg (35%) of the title compound as a white solid.

uPLC-MS: tr = 1.38 min. (100%). LRMS (m/z): 490 (M+1).

1H NMR (400 MHz, DMSO-d6) δ 10.09 (s, 1H), 8.41 (s, 1H), 8.28 (s, 1H), 7.58 (d, J = 8.3 Hz, 1H), 7.50 (d, J = 1.9 Hz, 1H), 7.36 - 7.16 (m, 8H), 3.97 (s, 2H), 3.14 (s, 3H), 3.01 (s, 3H), 2.31 (s, 3H), 2.29 (s, 3H).

**Starting again from the compound of exemple 67 and following the methodics shown previously compounds 110 and 111 are obtained.**

**Example 110: N-[2-[[6-[(2,6-dimethylpyrimidin-4-yl)amino]pyrimidin-4-yl]amino]-5-pyrrolidin-3-yl-phenyl]-N-methyl-methanesulfonamide**

uPLC-MS: tr = 0.60 min. (100%). LRMS (m/z): 469 (M+1).

1H NMR (600 MHz, DMSO-d6) δ 9.91 (s, 1H), 8.27 (s, 1H), 8.19 (s, 1H), 7.57 (d, J = 8.3 Hz, 1H), 7.40 (d, J = 1.8 Hz, 1H), 7.32 - 7.25 (m, 2H), 7.20 (s, 1H), 3.21 (s, 3H), 3.14 (d, J = 5.3 Hz, 9H), 3.00 (s, 3H), 2.93 - 2.86 (m, 1H), 2.68 - 2.62 (m, 1H), 2.54 - 2.42 (m, 19H), 2.37 (s, 2H), 2.28 (s, 2H), 2.17 - 2.09 (m, 1H), 1.75 - 1.64 (m, 1H), 1.22 (s, 1H).

**Example 111: N-[2-[[6-[(2,6-dimethylpyrimidin-4-yl)amino]pyrimidin-4-yl]amino]-5-tetrahydrofuran-3-yl-phenyl]-N-methyl-methanesulfonamide**

uPLC-MS: tr = 0.96 min. (100%). LRMS (m/z): 470 (M+1).

1H NMR (400 MHz, DMSO-d6) δ 10.10 (s, 1H), 8.43 (s, 1H), 8.30 (d, J = 0.8 Hz, 1H), 8.26 (s, 1H), 7.64 (d, J = 8.3 Hz, 1H), 7.47 (d, J = 2.0 Hz, 1H), 7.35 - 7.28 (m, 2H), 7.23 (s, 1H), 4.09 - 3.92 (m, 3H), 3.81 (q, J = 7.9 Hz, 2H), 3.62 - 3.54 (m, 2H), 3.16 (s, 2H), 3.03 (s, 3H), 2.42 - 2.28 (m, 6H), 2.03 - 1.88 (m, 2H).

**Example 112: N6-(2,6-dimethylpyrimidin-4-yl)-N4-[2-(N,S-dimethylsulfonimidoyl)phenyl]pyrimidine-4,6-diamine**

**Step 112-1: Synthesis of imino(methyl)(2-nitrophenyl)-λ$^6$-sulfanone**

A mixture of 1-methylsulfanyl-2-nitro-benzene (1.5 g, 8.86 mmol), ammonium carbamate (1 g, 12.81 mmol) and (Diacetoxyiodo)benzene (6 g, 18.6 mmol) in 45 mL metanol is stirred at 20°C in an open flask. After 1hr the mixture is concentrated and partitioned between EtOAc/brine. The aqueous layer is further extracted with DCM and the organic extracts are dried and concentrated. The residue is purified by flash chromatography (DCM/MeOH 0-2%) to give 1.21 g (68%) of the title compound

uPLC-MS: tr = 0.68 min. (100%). LRMS (m/z): 201 (M+1).

1H NMR (400 MHz, Chloroform-d) δ 8.28 - 8.19 (m, 1H), 7.88 - 7.68 (m, 3H), 3.43 (s, 3H).

**Step 112-2: Synthesis of methyl(methylimino)(2-nitrophenyl)- λ$^6$-sulfanone**

A mixture of imino-methyl-(2-nitrophenyl)-oxo-$l^{6}-sulfane (Step 112-1; 500 mg, 2.49 mmol), aqueous formaldehyde (0.153 mL, 5.49 mmol) and formic acid (10 mL, 265 mmol) is stirred at 100°C in a stopped flask for 16 hr. Adiitional formaldehyde (0.153 mL, 5.49 mmol) is added and the reaction prosecuted for 24 hr. The solution is concentrated and the residue purified by reverse-phase flash chromatography (water to MeCN/MeOH 1:1 100%) to give 328 mg (50%) of 81% pure title compound.

uPLC-MS: tr = 0.89 min. (81%). LRMS (m/z): 215 (M+1).

1H NMR (400 MHz, Chloroform-d) δ 8.11 - 7.98 (m, 1H), 7.80 - 7.67 (m, 3H), 3.36 (s, 3H), 2.64 (s, 3H).

**Step 112-3: Synthesis of 2-(N,S-dimethylsulfonimidoyl)aniline**

A mixture of methyl-methylimino-(2-nitrophenyl)-oxo-$l^{6}-sulfane (Step 112-3, 141 mg, 0.658 mmol), ammonium formate (83 mg, 1.32 mmol) and palladium hydroxide (9.3 mg, 0.066 mmol) in 1.5 mL methanol is stirred at 80°C under argon atmosphere overnight. After concentration the residue is purified by reverse-phase flash chromatography (water to 100% MeOH/MeCN) to give after liophilization 91 mg (75%) of an oily residue of title compound.

uPLC-MS: tr = 0.67 min. (98%). LRMS (m/z): 185 (M+1).

1H NMR (400 MHz, Chloroform-d) δ 7.76 - 7.62 (m, 1H), 7.27 (ddd, J = 8.2, 7.3, 1.6 Hz, 1H), 6.79 - 6.59 (m, 2H), 5.22 (s, 2H), 3.01 (s, 3H), 2.62 (s, 3H).

**Step 112-4: Synthesis of N6-(2,6-dimethylpyrimidin-4-yl)-N4-[2-(N,S-dimethylsulfonimidoyl)phenyl]pyrimidine-4,6-diamine**

Starting from 2-(N,S-dimethylsulfonimidoyl)aniline (Step 112-3) and N-(6-chloropyrimidin-4-yl)-2,6-dimethylpyrimidin-4-amine (Step 6-1) and following the procedure of Step 17-1 to give the title compound in 20% yield.

uPLC-MS: tr = 0.98 min. (95%). LRMS (m/z): 384 (M+1).

1H NMR (400 MHz, Chloroform-d) δ 8.52 (d, J = 17.1 Hz, 1H), 8.39 (d, J = 8.4 Hz, 1H), 7.96 (dd, J = 8.0, 1.6 Hz, 1H), 7.67 - 7.58 (m, 1H), 7.55 (s, 1H), 7.25 - 7.16 (m, 2H), 6.91 (s, 1H), 5.30 (s, 1H), 3.09 (s, 3H), 2.83 (s, 3H), 2.63 (d, J = 6.1 Hz, 3H), 2.48 (d, J = 10.1 Hz, 3H).

**Example 113: N4-[4-(1,5-dimethylpyrazol-4-yl)-2-(N,S-dimethylsulfonimidoyl)phenyl]-N6-(2,6-dimethylpyrimidin-4-yl)pyrimidine-4,6-diamine**

**Step 113-1: Synthesis of 4-bromo-2-(N,S-dimethylsulfonimidoyl)aniline**

A mixture of 2-(N,S-dimethylsulfonimidoyl)aniline (Step 112-3; 1 g, 5.4 mmol) and N-bromosuccinimide (967 mg, 5.4 mmol) in 37 mL acetonitrile is stirred at room temperature under argon and in absence of light for 24 hr. After concentration the residue is partitioned between water and EtOAc and the aqueous layer further extracted with EtOAc. The organic extracts are washed with brine, dried and concentrated to give the title compound 1.19 g (81%) as an orange oil.

uPLC-MS: tr = 1.16 min. (97%). LRMS (m/z): 263/265 (M+1, Br).

1H NMR (400 MHz, Chloroform-d) δ 7.85 (d, J = 2.4 Hz, 1H), 7.53 - 7.29 (m, 2H), 6.64 (dd, J = 8.6, 4.0 Hz, 1H), 5.31 (s, 1H), 3.08 (s, 3H), 2.62 (s, 3H).

**Step 113-2: Synthesis of 4-(1,5-dimethylpyrazol-4-yl)-2-(N,S-dimethylsulfonimidoyl)aniline**

A mixture of 4-bromo-2-(N,S-dimethylsulfonimidoyl)aniline (Step 113-1; 100 mg, 0.38 mmol), 1,5-dimethyl-4-(4,4,5,5-tetramethyl-1,3,2-dioxaborolan-2-yl)pyrazole (102 mg, 0.46 mmol), cyclopenta-1,4-dien-1-yl(diphenyl)phosphane;dichloromethane;dichloropalladium;iron(2+)

(19 mg, 0.023 mmol) and 2N aqueous solution of cesium carbonate (0.57 mL, 1.14 mmol) in 5 mL dioxane are stirred under argon at 100°C for 3hr. After concentration the residue is partitioned betwen water and EtOAc and the organic layer further extracted with additional EtOAc. The organic extractes are washed with brine, dried and concentrated. The residue is purified by flash chromatography MeOH in DCM 0 to 4%) to give 68 mg (64%) of title compound as an off-white foam.

uPLC-MS (3 min): tr = 0.87 min. (98%). LRMS (m/z): 279 (M+1).

1H NMR (400 MHz, Chloroform-d) $\delta$ 7.71 (d, J = 2.1 Hz, 1H), 7.51 (s, 1H), 7.35 (dd, J = 8.3, 2.1 Hz, 1H), 6.78 (d, J = 8.3 Hz, 1H), 5.27 (br s, 2H), 3.83 (s, 3H), 3.10 (s, 3H), 2.72 (s, 3H), 2.36 (s, 3H).

**Step 113-3: Synthesis of N4-[4-(1,5-dimethylpyrazol-4-yl)-2-(N,S-dimethylsulfonimidoyl)phenyl]-N6-(2,6-dimethylpyrimidin-4-yl)pyrimidine-4,6-diamine**

Starting from 4-(1,5-dimethylpyrazol-4-yl)-2-(N,S-dimethylsulfonimidoyl)aniline (Step 113-2) and N-(6-chloropyrimidin-4-yl)-2,6-dimethyl-pyrimidin-4-amine (Step 6-1) and following the procedure of Step 17-1 to give the title compound in 26% yield.

uPLC-MS: tr = 1.09 min. (99%). LRMS (m/z): 478 (M+1).

1H NMR (400 MHz, DMSO-d6) $\delta$ 10.28 (s, 1H), 10.03 (s, 1H), 8.48 - 8.44 (m, 1H), 8.33 (d, J = 8.6 Hz, 1H), 7.82 (d, J = 2.2 Hz, 1H), 7.72 (dd, J = 8.6, 2.2 Hz, 1H), 7.64 (d, J = 14.7 Hz, 2H), 7.22 (s, 3H), 3.80 (s, 3H), 3.21 (s, 3H), 2.69 (s, 3H), 2.48 (s, 3H), 2.33 (s, 3H).

**Example 114: N-[2-[[6-[(2,6-dimethylpyrimidin-4-yl)amino]pyrimidin-4-yl]amino]-5-tetrahydrofuran-2-yl-phenyl]-N-methyl-methanesulfonamide**

**Step 114-1: Synthesis of N-[2-[[6-[(2,6-dimethylpyrimidin-4-yl)amino]pyrimidin-4-yl]amino]-5-tetrahydrofuran-2-yl-phenyl]-N-methyl-methanesulfonamide**

An 8 mL vial equipped with a screw top with a septum incorporated was charged with N-[5-bromo-2-[[6-[(2,6-dimethylpyrimidin-4-yl)amino]pyrimidin-4-yl]amino]phenyl]-N-methyl-methanesulfonamide (Example 67; 50 mg, 0.104 mmol), oxolane-2-carboxylic acid (36 mg, 0.310 mmol), (Ir[dF(CF3)ppy]2(dtbpy))PF6 (CAS:870987-63-6; 1.1 mg (980 nmol), a solution of the preformed nickel catalyst (Step 109-1; 0.105 mL, 0.310 mmol), 2-tert-butyl-1,1,3,3-tetramethylguanidine (Barton's base; 0,064 mL, 0.313 mmol) and dimethylsulfoxide (1 mL). The mixture was degassed by bubbling an argon stream through it for 15 minutes. The vial was sealed and irradiated with blue LEDs (490 nM) in a Penn phD Photoreactor M2 for 16 hours.

The reaction mixture was partitioned between water and EtOAc and the organic layer was washed with water, brine, dried and concentrated. The residue was purified by reverse phase chromatography (0% to 100% water-acetonitrile/MeOH (1:1) (0.1% v/v ammonium formate both phases)) to give 7.5 mg (16%) of the title compound as a pale yellow solid.

uPLC-MS: tr = 1.04 min. (92%). LRMS (m/z): 470 (M+1).

1H NMR (400 MHz, DMSO-d6) $\delta$ 8.45 (s, 1H), 8.31 - 8.26 (m, 1H), 7.68 (d, J = 8.3 Hz, 1H), 7.47 (d, J = 1.8 Hz, 1H), 7.37 - 7.29 (m, 2H), 7.25 (s, 1H), 4.87 - 4.78 (m, 1H), 4.05 - 3.96 (m, 1H), 3.85 - 3.78 (m, 1H), 3.16 (s, 3H), 3.03 (s, 3H), 2.40 (s, 3H), 2.36 - 2.24 (m, 4H), 2.01 - 1.90 (m, 2H), 1.77 - 1.64 (m, 1H).

**Example 115: N-[2-[[6-[(2,6-dimethylpyrimidin-4-yl)amino]pyrimidin-4-yl]amino]-5-(methoxymethyl)phenyl]-N-methyl-methanesulfonamide**

**Step 115-1: Synthesis of N-[2-[[6-[(2,6-dimethylpyrimidin-4-yl)amino]pyrimidin-4-yl]amino]-5-(methoxymethyl)phenyl]-N-methyl-methanesulfonamide**

Starting from N-[5-bromo-2-[[6-[(2,6-dimethylpyrimidin-4-yl)amino]pyrimidin-4-yl]amino]phenyl]-N-methyl-methanesulfonamide (Example 67) and following the same procedure as described in Step 109 but replacing the potassium benzyl(trifluoro)boranuide for potassium trifluoro(methoxymethyl)boranuide title compound is obtained in 10% yield

uPLC-MS (3 min): tr = 0.94 min. (95%). LRMS (m/z): 444 (M+1).

1H NMR (400 MHz, Chloroform-d) δ 8.43 (d, J = 0.8 Hz, 1H), 7.97 (d, J = 8.9 Hz, 1H), 7.71 (s, 1H), 7.36 - 7.30 (m, 3H), 6.95 (s, 1H), 4.46 (s, 2H), 3.42 (s, 3H), 3.27 (s, 3H), 2.98 (s, 3H), 2.57 (s, 3H), 2.44 (s, 3H).

**Example 116: N6-(2,6-dimethylpyrimidin-4-yl)-N4-[2-(N,S-dimethylsulfonimidoyl)-3-methyl-phenyl]pyrimidine-4,6-diamine**

In a paralel way as described for the synthesis of exemple 112 but starting from 1-methyl-2-methylsulfanyl-3-nitro-benzene instead of 1-methylsulfanyl-2-nitro-benzene title compound is obtained in 21% yield as a white solid.
uPLC-MS (3 min): tr = 1.09 min. (97%). LRMS (m/z): 398 (M+1).
1H NMR (400 MHz, DMSO-d6) δ 10.60 (s, 1H), 10.21 (s, 1H), 8.40 (s, 1H), 8.02 (d, J = 7 Hz, 1H), 7.62 - 7.58 (m, 1H), 7.44-7.42 (m, 1H), 7.18 (s, 1H), 7.03-7.01 (m, 1H), 3.17 (s, 3H), 2.59 (s, 3H), 2.42 (s, 3H), 2.25 (s, 3H).

**Example 117: N4-(2,6-dimethylpyrimidin-4-yl)-N6-(2-methoxy-4-tetrahydrofuran-3-yl-phenyl)pyrimidine-4,6-diamine**

**Step 117-1: Synthesis of N6-(4-bromo-2-methoxy-phenyl)-N4-(2,6-dimethylpyrimidin-4-yl)pyrimidine-4,6-diamine**

A mixture of 4-bromo-2-methoxy-aniline (412 mg, 2.04 mmol), N-(6-chloropyrimidin-4-yl)-2,6-dimethyl-pyrimidin-4-amine (Step 6-1) and 0.28 mL of concentrated hydrochloric acid in 15 mL of ethanol is stirred at 100°C overnight. After concentration the residue is purified by flash chromatography (DCM/MeOH 0-5 %) to give 526 mg (77%) of title compound as an off.white solid.
HPLC-MS: tr = 2.12 min. (98%). LRMS (m/z): 401/403 (M+1, Br).
1H NMR (CDCl3, 400 MHz) δ ppm 2.44 (s, 3H), 2.60 (s, 3H), 3.90 (s, 3H), 6.86 (s, 1H), 7.06 (d, J = 2.1 Hz, 1H), 7.10 (br s, 1H), 7.14 (dd, J = 8.6 and 2.1 Hz, 1H), 7.39 (br s, 1H), 7.51 (s, 1H), 7.84 (d, J = 8.6 Hz, 1H), 8.42 (s, 1H).

**Step 117-2: Synthesis of N6-[4-(2,5-dihydrofuran-3-yl)-2-methoxy-phenyl]-N4-(2,6-dimethylpyrimidin-4-yl)pyrimidine-4,6-diamine**

Starting from compound 117-1 and 2-(2,5-dihydrofuran-3-yl)-4,4,5,5-tetramethyl-1,3,2-dioxaborolane and following the procedure described in step 107-1 the title compound is obtained in 74% yield.

HPLC-MS: tr = 1.90 min. (98%). LRMS (m/z): 391 (M+1,).
1H NMR (CDCl3, 400 MHz) δ ppm 2.49 (s, 3H), 2.63 (s, 3H), 3.93 (s, 3H), 4.87 (td, J = 4.8 and 2 Hz, 2H), 5.01 (td, J = 4.8 and 2 Hz, 1H), 6.20 (t, J = 2 Hz, 1H), 6.90-7.00 (m, 3H), 7.02-7.08 (m, 1H), 7.33-7.43 (m, 1H), 7.52 (br s, 1H), 7.90 (d, J = 7.6 Hz, 1H), 8.44 (s, 1H).

**Step 117-3: Synthesis of N4-(2,6-dimethylpyrimidin-4-yl)-N6-(2-methoxy-4-tetrahydrofuran-3-yl-phenyl)pyrimidine-4,6-diamine**

The solution of N6-[4-(2,5-dihydrofuran-3-yl)-2-methoxy-phenyl]-N4-(2,6-dimethylpyrimidin-4-yl)pyrimidine-4,6-diamine (Step 117-2; 134 mg, 0.540 mmol) and Pd/C (5%, 13 mgl) in EtOH (6 ml) was stirred at room temperature under a hydrogen atmosphere. The reaction was filtered and concentrated. The yellow solid obtained was purified by flash chromatography (Biotage, SNAP 4g gold) by using Et2O/Et2O-MeOH (10%) to obtain 22 mg (60%) of title compound as a white solid.
HPLC-MS: tr = 1.82 min. (99%). LRMS (m/z): 393 (M+1,).
1H NMR (DMSO, 400 MHz) δ ppm 1.91-2.01 (m, 1H), 2.28 (s, 3H), 2.28-2.35 (m, 2H), 2.37 (s, 3H), 3.58 (t, J = 8 Hz, 1H), 3.77-3.84 (m, 1H), 3.79 (s, 3H), 3.97 (td, J = 8.3 and 4.4 Hz, 1H), 4.04 (t, J = 8 Hz, 1H), 6.87 (dd, J = 8 and 1.8 Hz, 1H), 7.00 (d, J = 1.6 Hz, 1H), 7.14 (s, 1H), 7.25 (s, 1H), 7.45 (d, J = 8 Hz, 1H), 8.24 (d, J = 1 Hz, 1H), 8.55 (s, 1H), 9.92 (s, 1H).

**Example 118: N4-[4-cyclopropyl-2-(N,S-dimethylsulfonimidoyl)phenyl]-N6-(2,6-dimethylpyrimidin-4-yl)pyrimidine-4,6-diamine**

**Step 118-1: Synthesis of 4-cyclopropyl-2-(N,S-dimethylsulfonimidoyl)aniline**

Starting from 4-bromo-2-(N,S-dimethylsulfonimidoyl)aniline (Step 113-1) and cyclopropylboronic acid and following the procedure described in Step 113-2 the title compound is obtained in 23% yield

uPLC-MS (3 min): tr = 1.10 min. (73%). LRMS (m/z): 225 (M+1).

1H NMR (400 MHz, Chloroform-d) δ 7.42 (s, 1H), 7.04-7.02 (m, 1H), 6.63-7.62 (m, 1H), 5.37-5.20 (br s, 2H), 3.03 (s, 3H), 2.63 (s, 3H), 1.86-1.73 (m 1H), 0.82-0.60 (m, 4H).

**Step 118-2: Synthesis of N4-[4-cyclopropyl-2-(N,S-dimethylsulfonimidoyl)phenyl]-N6-(2,6-dimethylpyrimidin-4-yl)pyrimidine-4,6-diamine**

Starting from intermediates of steps 118-1 and 6-1 and following the procedure described in step 17-1 the title compound is obtained in 12% yield as a white solid uPLC-MS (3 min): tr = 1.29 min. (100%). LRMS (m/z): 424 (M+1).

1H NMR (400 MHz, Chloroform-d) δ 8.46 (s, 1H), 8.16 (d, J = 8.5 Hz, 1H), 7.67 (d, J = 2.2 Hz, 1H), 7.53 (s, 1H), 7.31 (dd, J = 8.5, 2.2 Hz, 1H), 6.96 (d, J = 12.1 Hz, 1H), 3.07 (s, 3H), 2.81 (s, 3H), 2.62 (s, 3H), 2.46 (s, 3H), 1.96 (ddd, J = 13.5, 8.5, 5.1 Hz, 1H), 1.39 - 1.23 (m, 2H), 0.92 - 0.67 (m, 2H).

**Example 119: N6-(2,6-dimethylpyrimidin-4-yl)-N4-[3-methoxy-4-(1-methyl-1,2,4-triazol-3-yl)-2-pyridyl]pyrimidine-4,6-diamine**

**Step 119-1: N6-(2,6-dimethylpyrimidin-4-yl)-N4-[3-methoxy-4-(1-methyl-1,2,4-triazol-3-yl)-2-pyridyl]pyrimidine-4,6-diamine**

A mixture of 3-methoxy-4-(1-methyl-1,2,4-triazol-3-yl)pyridin-2-amine (WO2019183186 Ex 1 step5; 20mg, 0.097 mmol), N-(6-chloropyrimidin-4-yl)-2,6-dimethyl-pyrimidin-4-amine (Step 6-1; 34 mg, 0.144 mmol), Pd2(dba)3 (9 mg, 0.010 mmol), Xantphos (11 mg, 0.019 mmol) and cesium carbonate (95 mg, 0.292 mmol) was heated in dioxane (1 mL) at 100 °C for 3 h. The mixture was diluted in ethyl acetate and washed with brine, dried, filtered and concentrated. The crude was purified by flash chromatography (10% methanol in dichloromethane gradient) to get a yellowish solid which was further treated wirth DCM/MeOH, filtered and the filtrate concentrated to give 24 mg (33%) of title compound as a yellowish solid.

HPLC-MS: tr = 1.48 min. (97.3%). LRMS (m/z): 405 (M+1).

1H NMR (400 MHz, DMSO-d6) δ 10.30 (s, 1H), 9.01 (s, 1H), 8.66 (d, J = 0.7 Hz, 1H), 8.60 (s, 1H), 8.45 (d, J = 1.1 Hz, 1H), 8.17 (d, J = 5.2 Hz, 1H), 7.52 (d, J = 5.2 Hz, 1H), 7.25 (s, 1H), 3.99 (d, J = 0.6 Hz, 3H), 3.82 (s, 3H), 2.52 (s, 3H), 2.33 (s, 3H).

**Compounds from Examples 120-178 are prepared in the same way as compound 119 by reacting the corresponding aryl amines with the different $R_2NH_2$ derivatives as in step 119-1.**

**Example 120: N-[2-[[6-[(2,6-dimethylpyrimidin-4-yl)amino]pyrimidin-4-yl]amino]-3-pyridyl]-N-methyl-methanesulfonamide**

uPLC-MS: tr = 0.76 min. (100%). LRMS (m/z): 401 (M+1).

1H NMR (400 MHz, Chloroform-d) δ 8.73 (d, J = 0.9 Hz, 1H), 8.51 (d, J = 1.0 Hz, 1H), 8.38 (dd, J = 4.9, 1.7 Hz, 1H), 8.23 (s, 1H), 7.67 (dd, J = 7.8, 1.7 Hz, 1H), 7.51 (s, 1H), 7.39 (s, 1H), 7.02 (dd, J = 7.8, 4.9 Hz, 1H), 3.28 (s, 3H), 3.03 (s, 3H), 2.65 (s, 3H), 2.48 (s, 3H).

**Example 121: N-[2-[[6-[(2,6-dimethylpyrimidin-4-yl)amino]pyrimidin-4-yl]amino]-3-pyridyl]-N-(trideuteriomethyl)methanesulfonamide**

uPLC-MS: tr = 0.79 min. (96.4%). LRMS (m/z): 404 (M+1).

1H NMR (400 MHz, Chloroform-d) δ 8.73 (s, 1H), 8.51 (d, J = 0.9 Hz, 1H), 8.38 (dd, J = 4.8, 1.7 Hz, 1H), 8.23 (s, 1H), 7.67 (dd, J = 7.8, 1.7 Hz, 1H), 7.49 (s, 1H), 7.39 (s, 1H), 7.02 (dd, J = 7.8, 4.9 Hz, 1H), 3.03 (s, 3H), 2.65 (s, 3H), 2.48 (s, 3H).

**Example 122: N-[2-[[6-[(2,6-dimethylpyrimidin-4-yl)amino]pyrimidin-4-yl]amino]-5-[1-methyl-5-(trifluoromethyl)-1,2,4-triazol-3-yl]-3-pyridyl]-N-methyl-methanesulfonamide**

HPLC-MS: tr = 1.97 min. (99%). LRMS (m/z): 550 (M+1).

1H NMR (DMSO, 400 MHz) δ ppm 2.34 (s, 3H), 2.55 (s, 3H), 3.23 (s, 3H), 3.31 (s, 3H), 4.14 (s, 3H), 7.31 (s, 1H), 8.46 (d, J = 2.1 Hz, 1H), 8.47 (d, J = 1 Hz, 1H), 8.65 (s, 1H), 8.91 (d, J = 2.1 Hz, 1H), 9.01 (s, 1H), 10.38 (s, 1H).

**Example 123: N-[2-[[6-[(2,6-dimethylpyrimidin-4-yl)amino]pyrimidin-4-yl]amino]-5-(1-methyl-1,2,4-triazol-3-yl)-3-pyridyl]-N-methyl-methanesulfonamide**

HPLC-MS: tr = 1.60 min. (99%). LRMS (m/z): 482 (M+1).

1H NMR (DMSO, 400 MHz) δ ppm 2.34 (s, 3H), 2.35 (s, 3H), 3.21 (s, 3H), 3.30 (s, 3H), 3.95 (s, 3H), 7.27 (s, 1H), 8.44 (d, J = 2 Hz, 1H), 8.45 (d, J = 1 Hz, 1H), 8.58 (s, 1H), 8.60 (s, 1H), 8.92 (d, J = 2 Hz, 1H), 9.04 (s, 1H), 10.35 (s, 1H).

**Example 124: N-[5-(1,5-dimethylpyrazol-4-yl)-2-[[6-[(2,6-dimethylpyrimidin-4-yl)amino]pyrimidin-4-yl]amino]-3-pyridyl]-N-methyl-methanesulfonamide**

HPLC-MS: tr = 1.70 min. (99%). LRMS (m/z): 495 (M+1).

1H NMR (DMSO, 400 MHz) δ ppm 2.33 (s, 3H), 2.40 (s, 3H), 2.53 (s, 3H), 3.18 (s, 3H), 3.27 (s, 3H), 3.81 (s, 3H), 7.22 (br s, 1H), 7.69 (s, 1H), 8.04 (d, J = 2.1 Hz, 1H), 8.37 (d, J = 2.1 Hz, 1H), 8.42 (d, J = 1 Hz, 1H), 8.43 (s, 1H), 9.00 (s, 1H), 10.30 (s, 1H).

**Example 125: N4-(2,6-dimethylpyrimidin-4-yl)-N6-(3-methylsulfonyl-2-pyridyl)pyrimidine-4,6-diamine**

uPLC-MS: tr = 0.89 min. (96%). LRMS (m/z): 372 (M+1).

1H NMR (400 MHz, Chloroform-d) δ 9.54 (s, 1H), 8.72 (s, 1H), 8.63 - 8.53 (m, 2H), 8.25 (dd, J = 7.8, 1.9 Hz, 1H), 7.54 (s, 1H), 7.35 (s, 1H), 7.14 (dd, J = 7.8, 4.8 Hz, 1H), 3.15 (s, 3H), 2.65 (s, 3H), 2.49 (s, 3H).

**Example 126: N-[2-[[6-[(2,6-dimethylpyrimidin-4-yl)amino]pyrimidin-4-yl]amino]-5-(1-methylpyrazol-4-yl)-3-pyridyl]-N-methyl-methanesulfonamide**

HPLC-MS: tr = 1.67 min. (99 %). LRMS (m/z): 481 (M+1).

1H NMR (400 MHz, DMSO-d6) δ 2.33 (s, 3H), 2.53 (s, 3H), 3.18 (s, 3H), 3.27 (s, 3H), 3.90 (s, 3H), 7.26 (s, 1H), 7.97 (s, 1H), 8.21 (d, J = 2.1 Hz, 1H), 8.24 (s, 1H), 8.41 (s, 2H), 8.54 - 8.57 (m, 1H), 8.86 (s, 1H), 10.29 (s, 1H).

**Example 127: N-[2-[[6-[(2,6-dimethylpyrimidin-4-yl)amino]pyrimidin-4-yl]amino]-5-(2-methylpyrazol-3-yl)-3-pyridyl]-N-methyl-methanesulfonamide**

HPLC-MS: tr = 1.67 min. (99 %). LRMS (m/z): 481 (M+1).

1H NMR (DMSO, 400 MHz) δ ppm 2.33 (s, 3H), 2.53 (s, 3H), 3.20 (s, 3H), 3.29 (s, 3H), 3.92 (s, 3H), 6.55 (d, J = , 1H), 7.23 (s, 1H), 7.52 (d, J = 1.6 Hz, 1H), 8.24 (s, 1H), 8.45 (s, 1H), 8.48 (d, J = 1.6 Hz, 1H), 8.55 (s, 1H), 9.05 (s, 1H), 10.35 (s, 1H).

**Example 128: N-[2-[[6-[(2,6-dimethylpyrimidin-4-yl)amino]pyrimidin-4-yl]amino]-5-(1-methylimidazol-2-yl)-3-pyridyl]-N-methyl-methanesulfonamide**

HPLC-MS: tr = 1.27 min. (99 %). LRMS (m/z): 481 (M+1).

1H NMR (DMSO, 400 MHz) δ ppm 2.33 (s, 3H), 2.54 (s, 3H), 3.20 (s, 3H), 3.30 (s, 3H), 3.82 (s, 3H), 7.04 (d, J = 1.1 Hz, 1H), 7.25 (br s, 1H), 7.33 (d, J = 1.1 Hz, 1H), 8.30 (d, J = 2.1 Hz, 1H), 8.46 (d, J = 1 Hz, 1H), 8.55 (s, 1H), 8.64 (d, J = 2.1 Hz, 1H), 9.07 (s, 1H), 10.36 (s, 1H).

**Example 129: N-[5-(2,5-dimethylpyrazol-3-yl)-2-[[6-[(2,6-dimethylpyrimidin-4-yl)amino]pyrimidin-4-yl]amino]-3-pyridyl]-N-methyl-methanesulfonamide**

HPLC-MS: tr = 1.77 min. (99 %). LRMS (m/z): 495 (M+1).

1H NMR (DMSO, 400 MHz) δ ppm 2.19 (s, 3H), 2.33 (s, 3H), 2.52 (s, 3H), 3.19 (s, 3H), 3.28 (s, 3H), 3.82 (s, 3H), 6.32 (s, 1H), 7.24 (s, 1H), 8.18 (br s, 1H), 8.44 (s, 2H), 8.54 (br s, 1H), 9.03 (br s, 1H), 10.35 (br s, 1H).

**Example 130: N-[2-[[6-[(2,6-dimethylpyrimidin-4-yl)amino]pyrimidin-4-yl]amino]-5-(2-methyl-1,2,4-triazol-3-yl)-3-pyridyl]-N-methyl-methanesulfonamide**

HPLC-MS: tr = 1.27 min. (97 %). LRMS (m/z): 482 (M+1).
1H NMR (DMSO, 400 MHz) δ ppm 2.34 (s, 3H), 2.54 (s, 3H), 3.22 (s, 3H), 3.30 (s, 3H), 4.04 (s, 3H), 7.25 (s, 1H), 8.06 (s, 1H), 8.38 (d, J = 2.1 Hz, 1H), 8.48 (d, J = 1 Hz, 1H), 8.65 (s, 1H), 8.72 (d, J = 2.1 Hz, 1H), 9.10 (s, 1H), 10.39 (s, 1H).

**Example 131: N-[2-[[6-[(2,6-dimethylpyrimidin-4-yl)amino]pyrimidin-4-yl]amino]-5-(1-methylpyrazol-3-yl)-3-pyridyl]-N-methyl-methanesulfonamide**

HPLC-MS: tr = 1.70 min. (99 %). LRMS (m/z): 481 (M+1).
1H NMR (DMSO, 400 MHz) δ ppm 2.33 (s, 3H), 2.53 (s, 3H), 3.20 (s, 3H), 3.28 (s, 3H), 3.91 (s, 3H), 6.81 (d, J = 2.3 Hz, 1H), 7.28 (s, 1H), 7.79 (d, J = 2 Hz, 1H), 8.30 (d, J = 2 Hz, 1H), 8.43 (d, J = 1 Hz, 1H), 8.49 (s, 1H), 8.74 (d, J = 2 Hz, 1H), 8.92 (s, 1H), 10.32 (s, 1H).

**Example 132: N4-(2,6-dimethylpyrimidin-4-yl)-N6-(3-methoxy-4-methyl-2-pyridyl)pyrimidine-4,6-diamine**

HPLC-MS: tr = 1.40 min. (99 %). LRMS (m/z): 338 (M+1).
1H NMR (CDCl3, 400 MHz) δ ppm 2.29 (s, 3H), 2.32 (s, 3H), under dmso (s, 3H), 3.76 (s, 3H), 6.94 (d, J = 5 Hz, 1H), 7.23 (s, 1H), 7.99 (d, J = 5 Hz, 1H), 8.41 (s, 1H), 8.45 (s, 1H), 8.90 (s, 1H), 10.24 (s, 1H).

**Example 133: N-[5-(1,5-dimethylpyrazol-4-yl)-2-[[6-[(2,6-dimethylpyrimidin-4-yl)amino]pyrimidin-4-yl]amino]-3-pyridyl]-N-(trideuteriomethyl)methanesulfonamide**

HPLC-MS: tr = 1.40 min. (99 %). LRMS (m/z): 338 (M+1).
1H NMR (400 MHz, DMSO-d6) δ 10.28 (s, 1H), 8.98 (s, 1H), 8.45 - 8.38 (m, 2H), 8.35 (d, J = 2.1 Hz, 1H), 8.02 (d, J = 2.2 Hz, 1H), 7.67 (s, 1H), 7.21 (s, 1H), 3.80 (s, 3H), 3.17 (s, 3H), 2.51 (s, 3H), 2.39 (s, 3H), 2.31 (s, 3H).

**Example 134: N-[2-[[6-[(2,6-dimethylpyrimidin-4-yl)amino]pyrimidin-4-yl]amino]-5-(4,5-dimethyl-1,2,4-triazol-3-yl)-3-pyridyl]-N-methyl-methanesulfonamide**

HPLC-MS: tr = 1.50 min. (99 %). LRMS (m/z): 496 (M+1)
1H NMR (400 MHz, DMSO-d6) δ 2.34 (s, 3H), 2.43 (s, 3H), 2.53 (s, 3H), 3.20 (s, 3H), 3.29 (s, 3H), 3.64 (s, 3H), 7.25 (s, 1H), 8.32 (d, J = 2.1 Hz, 1H), 8.47 (d, J = 0.9 Hz, 1H), 8.59 - 8.64 (m, 2H), 9.10 (s, 1H), 10.39 (s, 1H).

**Example 135: N-[5-(1,5-dimethylpyrazol-4-yl)-2-[[6-[(6-methylpyrimidin-4-yl)amino]pyrimidin-4-yl]amino]-3-pyridyl]-N-methyl-methanesulfonamide**

HPLC-MS: tr = 1.82 min. (97 %). LRMS (m/z): 481 (M+1).
1H NMR (400 MHz, DMSO-d6) δ 2.39 (s, 3H), 2.41 (s, 3H), 3.18 (s, 3H), 3.27 (s, 3H), 3.81 (s, 3H), 7.66 (s, 1H), 7.71 (s, 1H), 8.02 (d, J = 2.0 Hz, 1H), 8.39 (d, J = 2.1 Hz, 1H), 8.46 (d, J = 9.7 Hz, 2H), 8.61 (s, 1H), 8.69 (s, 1H), 10.38 (s, 1H).

**Example 136: N-[2-[[6-[(2,6-dimethylpyrimidin-4-yl)amino]pyrimidin-4-yl]amino]-5-(4,5-dimethyl-1,2,4-triazol-3-yl)-3-pyridyl]-N-(trideuteriomethyl)methanesulfonamide**

HPLC-MS: tr = 1.50 min. (99 %). LRMS (m/z): 499 (M+1).
1H NMR (400 MHz, DMSO-d6) δ 10.39 (s, 1H), 9.09 (s, 1H), 8.62 (s, 1H), 8.61 (d, J = 2.2 Hz, 1H), 8.47 (d, J = 1.1 Hz, 1H), 8.33 (d, J = 2.1 Hz, 1H), 7.25 (s, 1H), 3.65 (s, 3H), 3.20 (s, 3H), 2.54 (s, 3H), 2.44 (s, 3H), 2.34 (s, 3H).

**Example 137: N4-[5-(1,5-dimethylpyrazol-4-yl)-3-methylsulfonyl-2-pyridyl]-N6-(2,6-dimethylpyrimidin-4-yl)pyrimidine-4,6-diamine**

uPLC-MS: tr = 1.08 min. (100 %). LRMS (m/z): 466 (M+1).
1H NMR (400 MHz, DMSO-d6) δ 10.41 (s, 1H), 9.56 (s, 1H), 9.11 (s, 1H), 8.66 (d, J = 2.3 Hz, 1H), 8.48 (d, J = 0.9 Hz, 1H), 8.21 (d, J = 2.3 Hz, 1H), 7.75 (s, 1H), 7.25 (s, 1H), 3.82 (s, 3H), 3.46 (s, 3H), 2.54 (s, 3H), 2.42 (s, 3H), 2.34 (s, 3H).

**Example 138: N-[5-(1,5-dimethylpyrazol-4-yl)-2-[[6-[(5-fluoro-2-pyridyl)amino]pyrimidin-4-yl]amino]-3-pyridyl]-N-methyl-methanesulfonamide**

HPLC-MS: tr = 2.00 min. (99 %). LRMS (m/z): 484 (M+1).

1H NMR (DMSO, 400 MHz) δ ppm 2.41 (s, 3H), 3.18 (s, 3H), 3.27 (s, 3H), 3.81 (s, 3H), 7.65-7.73 (m, 2H), 7.79 (dd, J = 9 and 4 Hz, 1H), 8.00 (d, J = 2 Hz, 1H), 8.30-8.34 (m, 2H), 8.36 (s, 1H), 8.39 (d, J = 2 Hz, 1H), 8.54 (s, 1H), 10.1 (s, 1H).

**Example 139: N-[6-[[5-(1,5-dimethylpyrazol-4-yl)-3-[methyl(methylsulfonyl)amino]-2-pyridyl]amino]pyrimidin-4-yl]cyclopropanecarboxamide**

HPLC-MS: tr = 2.00 min. (100 %). LRMS (m/z): 457 (M+1).

1H NMR (DMSO, 400 MHz) δ ppm 0.82-0.86 (m, 4H), 2.00-2.07 (m, 1H), 2.40 (s, 3H), 3.17 (s, 3H), 3.26 (s, 3H), 3.80 (s, 3H), 7.71 (s, 1H), 8.01 (d, J = 2.1 Hz, 1H), 8.35 (d, J = 2.1 Hz, 1H), 8.44 (d, J = 1 Hz, 1H), 8.58 (s, 1H), 8.77 (d, J = 1Hz, 1H), 10.93 (s, 1H).

**Example 140: N-[5-(1,5-dimethylimidazol-2-yl)-2-[[6-[(2,6-dimethylpyrimidin-4-yl)amino]pyrimidin-4-yl]amino]-3-pyridyl]-N-methyl-methanesulfonamide**

HPLC-MS: tr = 1.33 min. (97 %). LRMS (m/z): 495 (M+1).

1H NMR (400 MHz, DMSO-d6) δ 2.25 (s, 3H), 2.33 (s, 3H), 2.53 (s, 3H), 3.19 (s, 3H), 3.28 (s, 3H), 3.63 (s, 3H), 6.82 (s, 1H), 7.23 (s, 1H), 8.23 (d, J = 2.1 Hz, 1H), 8.45 (s, 1H), 8.54 (s, 1H), 8.57 (d, J = 2.1 Hz, 1H), 9.08 (s, 1H), 10.34 (s, 1H).

**Example 141: N6-[5-(1,5-dimethylpyrazol-4-yl)-3-methoxy-4-methyl-2-pyridyl]-N4-(2,6-dimethylpyrimidin-4-yl)pyrimidine-4,6-diamine**

HPLC-MS: tr = 1.68 min. (99 %). LRMS (m/z): 432 (M+1).

1H NMR (DMSO, 400 MHz) δ ppm 2.17 (s, 3H), 2.19 (s, 3H), 2.32 (s, 3H), 2.48 (s, 3H), 3.79 (s, 3H), 3.81 (s, 1H), 7.21 (s, 1H), 7.44 (s, 1H), 7.87 (s, 1H), 8.41 (d, J = 1 Hz, 1H), 8.52 (s, 1H), 8.91 (s, 1H), 10.24 (s, 1H).

**Example 142: N-[5-(2,3-dimethylimidazol-4-yl)-2-[[6-[(2,6-dimethylpyrimidin-4-yl)amino]pyrimidin-4-yl]amino]-3-pyridyl]-N-methyl-methanesulfonamide**

HPLC-MS: tr = 1.30 min. (98 %). LRMS (m/z): 495 (M+1).

1H NMR (DMSO, 400 MHz) δ ppm 2.33 (s, 3H), 2.38 (s, 3H), 2.52 (s, 3H), 3.18 (s, 3H), 3.27 (s, 3H), 3.57 (s, 1H), 7.02 (s, 1H), 7.22 (s, 1H), 8.12 (d, J = 2.1 Hz, 1H), 8.39 (d, J = 2.1 Hz, 1H), 8.44 (s, 1H), 8.50 (s, 1H), 9.03 (s, 1H), 10.33 (s, 1H).

**Example 143: N6-(2,6-dimethylpyrimidin-4-yl)-N4-[3-methoxy-4-[5-(trifluoromethyl)-1,2,4-oxadiazol-3-yl]-2-pyridyl]pyrimidine-4,6-diamine**

HPLC-MS: tr = 2.25 min. (100 %). LRMS (m/z): 460 (M+1).

1H NMR (400 MHz, DMSO-d6) δ 2.34 (s, 3H), 2.52 (s, 3H), 3.85 (s, 3H), 7.27 (s, 1H), 7.55 (d, J = 5.1 Hz, 1H), 8.33 (d, J = 5.1 Hz, 1H), 8.48 (s, 1H), 8.94 (s, 1H), 8.98 (s, 1H), 10.33 (s, 1H).

**Example 144: N-[5-(1,5-dimethylpyrazol-4-yl)-2-[[6-[(2,6-dimethylpyrimidin-4-yl)amino]pyrimidin-4-yl]amino]-3-pyridyl]methanesulfonamide**

HPLC-MS: tr = 1.53 min. (99 %). LRMS (m/z): 481 (M+1).

1H NMR (400 MHz, DMSO-d6) δ 2.33 (s, 3H), 2.39 (s, 3H), 3.08 (s, 3H), 3.81 (s, 3H), 7.23 (s, 1H), 7.65 (s, 1H), 7.74 (d, J = 2.1 Hz, 1H), 8.30 - 8.34 (m, 1H), 8.42 (s, 1H), 8.76 (s, 1H), 9.02 (s, 1H), 9.59 (s, 1H), 10.26 (s, 1H).

**Example 145: N6-(3-dimethylphosphoryl-2-pyridyl)-N4-(2,6-dimethylpyrimidin-4-yl)pyrimidine-4,6-diamine**

uPLC-MS: tr = 0.74 min. (94 %). LRMS (m/z): 370 (M+1).

1H NMR (400 MHz, DMSO-d6) δ 11.31 (s, 1H), 10.28 (s, 1H), 9.09 (s, 1H), 8.47 (dt, J = 4.5, 2.0 Hz, 1H), 8.40 (d, J = 1.0 Hz, 1H), 8.02 (ddd, J = 13.8, 7.6, 1.9 Hz, 1H), 7.27 (s, 1H), 7.10 (ddd, J = 7.5, 4.9, 1.4 Hz, 1H), 2.53 (s, 3H),

2.33 (s, 3H), 1.84 (s, 3H), 1.81 (s, 3H).

**Example 146: N6-[5-(1,5-dimethylpyrazol-4-yl)-3-methoxy-2-pyridyl]-N4-(2,6-dimethylpyrimidin-4-yl)pyrimidine-4,6-diamine**

uPLC-MS: tr = 1.02min. (90 %). LRMS (m/z): 418 (M+1).

1H NMR (400 MHz, CD3OD) δ 8.95 (s, 1H), 8.30 (s, 1H), 7.88 (d, J = 1.7 Hz, 1H), 7.52 (s, 1H), 7.27 - 7.18 (m, 2H), 3.92 (s, 3H), 3.77 (s, 3H), 2.51 (s, 3H), 2.34 (s, 3H), 2.32 (s, 3H).

**Example 147: N-[2-[[6-[(2,6-dimethylpyrimidin-4-yl)amino]pyrimidin-4-yl]amino]-5-(1-methylimidazol-4-yl)-3-pyridyl]-N-methyl-methanesulfonamide**

HPLC-MS: tr = 1.37 min. (93%). LRMS (m/z): 481 (M+1)

1H NMR (400 MHz, DMSO-d6) δ 2.33 (s, 3H), 2.54 (s, 3H), 3.18 (s, 3H), 3.27 (s, 3H), 3.72 (s, 3H), 7.25 - 7.27 (m, 1H), 7.70 - 7.72 (m, 1H), 7.72 - 7.74 (m, 1H), 8.27 (d, J = 2.1 Hz, 1H), 8.40 - 8.44 (m, 2H), 8.72 (d, J = 2.1 Hz, 1H), 8.92 (s, 1H), 10.31 (s, 1H).

**Example 148: N4-(2,6-dimethylpyrimidin-4-yl)-N6-[4-(1,5-dimethyl-1,2,4-triazol-3-yl)-3-methoxy-2-pyridyl]pyrimidine-4,6-diamine**

HPLC-MS: tr = 1.48 min. (100%). LRMS (m/z): 419 (M+1)

1H NMR (DMSO, 400 MHz) δ ppm 2.34 (s, 3H), 2.35 (s, 3H), 3.21 (s, 3H), 3.30 (s, 3H), 3.95 (s, 3H), 7.27 (s, 1H), 8.44 (d, J = 2 Hz, 1H), 8.45 (d, J = 1 Hz, 1H), 8.58 (s, 1H), 8.60 (s, 1H), 8.92 (d, J = 2 Hz, 1H), 9.04 (s, 1H), 10.35 (s, 1H).

**Example 149: N6-[3-dimethylphosphoryl-5-(1,5-dimethylpyrazol-4-yl)-2-pyridyl]-N4-(2,6-dimethylpyrimidin-4-yl)pyrimidine-4,6-diamine**

uPLC-MS: tr = 0.92 min. (93%). LRMS (m/z): 464 (M+1)

1H NMR (400 MHz, DMSO-d6) δ 11.32 (s, 1H), 10.27 (s, 1H), 9.15 (s, 1H), 8.47 (s, 1H), 8.40 (s, 1H), 7.97 (d, J = 12.8 Hz, 1H), 7.69 (s, 1H), 7.24 (s, 1H), 3.81 (s, 3H), 2.54 (s, 3H), 2.39 (s, 3H), 2.33 (s, 3H), 1.89 (s, 3H), 1.86 (s, 3H).

**Example 150: N4-(2,6-dimethylpyrimidin-4-yl)-N6-(4-methyl-3-methylsulfonyl-2-pyridyl)pyrimidine-4,6-diamine**

HPLC-MS: tr = 1.78 min. (99%). LRMS (m/z): 386 (M+1).

NMR 1H (DMSO, 400 MHz) δ ppm 2.33 (s, 3H), under DMSO (s, 3H), 2.66 (s, 3H), under water (s, 3H), 7.10 (d, J = 5 Hz, 1H), 7.28 (s, 1H), 8.43 (d, J = 5 Hz, 1H), 8.45 (d, J = 1 Hz, 1H), 9.02 (s, 1H), 10.34 (s, 2H).

**Example 151: N-[6-[[5-(1,5-dimethylpyrazol-4-yl)-3-methylsulfonyl-2-pyridyl]amino]pyrimidin-4-yl]cyclopropanecarboxamide**

HPLC-MS: tr = 2.27 min. (99%). LRMS (m/z): 428 (M+1).

1H NMR (400 MHz, DMSO-d6) δ 0.80 - 0.87 (m, 4H), 1.98 - 2.09 (m, 1H), 2.39 (s, 3H), 3.42 (s, 3H), 3.79 (s, 3H), 7.76 (s, 1H), 8.17 (d, J = 2.3 Hz, 1H), 8.49 (d, J = 1.1 Hz, 1H), 8.64 (d, J = 2.3 Hz, 1H), 8.94 (d, J = 1.1 Hz, 1H), 9.58 (s, 1H), 11.02 (s, 1H).

**Example 152: N4-[5-(1,5-dimethylpyrazol-4-yl)-3-methylsulfonyl-2-pyridyl]-N6-(5-fluoro-2-pyridyl)pyrimidine-4,6-diamine**

HPLC-MS: tr = 2.22 min. (99%). LRMS (m/z): 455 (M+1).

1H NMR (400 MHz, DMSO-d6) δ 2.42 (s, 3H), 3.44 (s, 3H), 3.82 (s, 3H), 7.71 (td, J = 9.1, 8.7, 3.0 Hz, 1H), 7.78 (d, J = 5.3 Hz, 2H), 8.18 (d, J = 2.3 Hz, 1H), 8.36 (d, J = 3.0 Hz, 1H), 8.40-8.42 (m, 1H), 8.68 - 8.74 (m, 2H), 9.45 (s, 1H), 10.22 (s, 1H).

**Example 153: N6-[5-(1,5-dimethylpyrazol-4-yl)-4-methyl-3-methylsulfonyl-2-pyridyl]-N4-(2,6-dimethylpyrimidin-4-yl)pyrimidine-4,6-diamine**

HPLC-MS: tr = 1.90 min. (99%). LRMS (m/z): 480 (M+1).

NMR 1H (DMSO, 400 MHz) δ ppm 2.18 (s, 3H), 2.33 (s, 3H), under DMSO (s, 3H), 2.54 (s, 3H), 3.49 (s, 3H),

3.82 (s, 3H), 7.28 (s, 1H), 7.46 (s, 1H), 8.26 (s, 1H), 8.46 (s, 1H), 9.04 (s, 1H), 10.36 (s, 1H), 10.44 (s, 1H).

**Example 154: N-[5-(1,5-dimethyl-1,2,4-triazol-3-yl)-2-[[6-[(6-methylpyrimidin-4-yl)amino]pyrimidin-4-yl]amino]-3-pyridyl]-N-methyl-methanesulfonamide**

HPLC-MS: tr = 2.67 min. (98 %). LRMS (m/z): 482 (M+1).

1H NMR (400 MHz, DMSO-d6) δ 2.39 (s, 3H), 2.47 (s, 3H), 3.20 (s, 3H), 3.28 (s, 3H), 3.85 (s, 3H), 7.69 (s, 1H), 8.38 (d, J = 2.0 Hz, 1H), 8.47 - 8.49 (m, 1H), 8.60 (s, 1H), 8.64 (d, J = 0.8 Hz, 1H), 8.69 (s, 1H), 8.87 (d, J = 2.0 Hz, 1H), 10.42 (s, 1H).

**Example 155: N-[5-(2,5-dimethyl-1,2,4-triazol-3-yl)-2-[[6-[(6-methylpyrimidin-4-yl)amino]pyrimidin-4-yl]amino]-3-pyridyl]-N-methyl-methanesulfonamide**

HPLC-MS: tr = 2.58 min. (98 %). LRMS (m/z): 482 (M+1).

1H NMR (400 MHz, DMSO-d6) δ 2.30 (s, 3H), 2.39 (s, 3H), 3.20 (s, 3H), 3.29 (s, 3H), 3.96 (s, 3H), 7.63 (s, 1H), 8.33 (d, J = 2.1 Hz, 1H), 8.50 (d, J = 1.0 Hz, 1H), 8.68 (s, 1H), 8.70 (d, J = 2.1 Hz, 1H), 8.72 (d, J = 0.9 Hz, 1H), 8.77 (d, J = 0.9 Hz, 1H), 10.47 (s, 1H).

**Example 156: N6-(6-methyl-3-methylsulfonyl-2-pyridyl)-N4-(6-methylpyrimidin-4-yl)pyrimidine-4,6-diamine**

HPLC-MS: tr = 1.70 min. (99 %). LRMS (m/z): 372 (M+1).

1H NMR (DMSO, 400 MHz) δ ppm 2.39 (s, 3H), 2.85 (s, 3H), 3.24 (s, 3H), 7.48 (d, J = 8.9 Hz, 1H), 7.56 (s, 1H), 8.80 (d, J = 8.9 Hz, 1H), 8.51 (d, J = 0.8 Hz, 1H), 8.65 (s, 1H), 8.69 (d, J = 0.8Hz, 1H), 10.49 (s, 1H), 10.56 (s, 1H).

**Example 157: N-[2-[[6-[(2,6-dimethylpyrimidin-4-yl)amino]pyrimidin-4-yl]amino]-5-(oxan-4-yl)pyridin-3-yl]-N-methylmethanesulfonamide**

HPLC-MS: tr = 1.61 min. (100 %). LRMS (m/z): 485 (M+1).

1H NMR (CD3Cl, 400 MHz) δ ppm 8.67 (s, 1H), 8.51 (s, 1H), 8.26-8.25 (m, 1H), 8.14 (s, 1H), 7.68 (bs, 1H), 7.53-7.52 (m, 1H), 7.41 (s, 1H), 4.13-4.11 (m, 2H), 3.59-3.53 (m, 2H), 3.28 (s, 3H), 3.03 (s, 3H), 2.85-2.81 (m. 1H), 2.65 (s, 3H), 2.49 (s, 3H), 1.82-1.75 (m, 4H).

**Example 158: N-[5-(1,5-dimethylpyrazol-4-yl)-2-[[6-[(6-methylpyridazin-3-yl)amino]pyrimidin-4-yl]amino]-3-pyridyl]-N-methyl-methanesulfonamide**

HPLC-MS: tr = 1.78 min. (100 %). LRMS (m/z): 481 (M+1).

1H NMR (400 MHz, DMSO-d6) δ 2.41 (s, 3H), 2.53 (s, 3H), 3.18 (s, 3H), 3.27 (s, 3H), 3.81 (s, 3H), 7.48 (d, J = 9.2 Hz, 1H), 7.70 (s, 1H), 8.02 (d, J = 2.2 Hz, 1H), 8.11 (d, J = 9.2 Hz, 1H), 8.27 - 8.31 (m, 1H), 8.34 (d, J = 2.2 Hz, 1H), 8.37 (d, J = 1.0 Hz, 1H), 8.41 (s, 1H), 10.37 (s, 1H).

**Example 159: 4-N-(2,6-dimethylpyrimidin-4-yl)-6-N-[3-methoxy-4-(5-methyl-1,2,4-oxadiazol-3-yl)pyridin-2-yl]pyrimidine-4,6-diamine**

HPLC-MS: tr = 1.80 min. (100 %). LRMS (m/z): 406 (M+1).

1H NMR (400 MHz, Chloroform-d) δ 8.86 (s, 1H), 8.52 (s, 1H), 8.24 (d, J = 5.2 Hz, 1H), 8.18 (s, 1H), 7.65 (s, 1H), 7.50 (d, J = 5.2 Hz, 1H), 7.36 (d, J = 14.8 Hz, 1H), 3.93 (s, 3H), 2.73 (s, 3H), 2.67 (s, 3H), 2.49 (s, 3H).

**Example 160: (1R,2R)-N-[6-[[5-(1,5-dimethylpyrazol-4-yl)-3-[methyl(methylsulfonyl)amino]-2-pyridyl]amino]pyrimidin-4-yl]-2-fluoro-cyclopropanecarboxamide**

HPLC-MS: tr = 1.97 min. (97 %). LRMS (m/z): 475 (M+1).

1H NMR (400 MHz, Methanol-d4) δ 1.11 - 1.40 (m, 1H), 1.66 - 1.94 (m, 1H), 2.45 (s, 3H), 3.12 (s, 3H), 3.32 (s, 3H), 3.86 (s, 3H), 4.66 - 5.11 (m, 1H), 7.66 (s, 1H), 7.96 (d, J = 2.0 Hz, 1H), 8.42 (s, 1H), 8.47 (d, J = 2.1 Hz, 1H), 9.09 (s, 1H).

**Example 161: N-[6-[[3-dimethylphosphoryl-5-(1,5-dimethylpyrazol-4-yl)-2-pyridyl]amino]pyrimidin-4-yl]cyclopropanecarboxamide**

uPLC-MS: tr = 1.11 min. (100 %). LRMS (m/z): 426 (M+1).

1H NMR (400 MHz, DMSO-d6) δ 11.39 (s, 1H), 10.93 (s, 1H), 9.05 (d, J = 1.0 Hz, 1H), 8.48-8.39 (m, 2H), 7.95 (dd, J = 14.1, 2.3 Hz, 1H), 7.70 (s, 1H), 3.80 (s, 3H), 2.38 (s, 3H), 2.04 (p, J = 7.1 Hz, 1H), 1.88 (s, 3H), 1.85 (s, 3H), 0.84 (m, 4H).

**Example 162: N-methyl-N-[6-methyl-5-(1-methylpyrazol-4-yl)-2-[[6-[(6-methylpyrimidin-4-yl)amino]pyrimidin-4-yl]amino]-3-pyridyl]methanesulfonamide**

HPLC-MS: tr = 1.85 min. (98 %). LRMS: 481 (M+1)
1H NMR (400 MHz, DMSO-d6) δ 2.39 (s, 3H), 2.69 (s, 3H), 3.16 (s, 3H), 3.24 (s, 3H), 3.91 (s, 3H), 7.58 (s, 1H), 7.80 (s, 1H), 7.98 (s, 1H), 8.07 (s, 1H), 8.32 (s, 1H), 8.44 (s, 1H), 8.68 (s, 1H), 8.94 (s, 1H), 10.49 (s, 1H).

**Example 163: N-[5-(1,5-dimethylpyrazol-4-yl)-6-methyl-2-[[6-[(6-methylpyrimidin-4-yl)amino]pyrimidin-4-yl]amino]-3-pyridyl]-N-methyl-methanesulfonamide**

HPLC-MS: tr = 1.90 min. (96 %). LRMS (m/z): 495 (M+1).
1H NMR (400 MHz, DMSO-d6) δ 2.21 (s, 3H), 2.38 (s, 3H), 3.13 (s, 3H), 3.23 (s, 3H), 3.81 (s, 3H), 7.52 (s, 1H), 7.56 (s, 1H), 7.78 (s, 1H), 8.36 (s, 1H), 8.44 (s, 1H), 8.67 (s, 1H), 8.95 (s, 1H), 10.49 (s, 1H).

**Example 164: N4-(2,6-dimethylpyrimidin-4-yl)-N6-[3-(N,S-dimethylsulfonimidoyl)-2-pyridyl]pyrimidine-4,6-diamine**

uPLC-MS: tr = 0.93 min. (97 %). LRMS (m/z): 385 (M+1).
1H NMR (400 MHz, Chloroform-d) δ 10.49 (s, 1H), 8.74 (s, 1H), 8.59 - 8.53 (m, 2H), 8.23 (dd, J = 7.8, 1.9 Hz, 1H), 7.50 (s, 1H), 7.38 (s, 1H), 7.11 (dd, J = 7.8, 4.8 Hz, 1H), 3.15 (s, 3H), 2.81 (s, 3H), 2.65 (s, 3H), 2.49 (s, 3H).

**Example 165: N4-[3-methoxy-6-methyl-5-(1-methylpyrazol-4-yl)-2-pyridyl]-N6-(6-methylpyrimidin-4-yl)pyrimidine-4,6-diamine**

uPLC-MS: tr = 1.11 min. (98 %). LRMS (m/z): 404 (M+1).
1H NMR (400 MHz, Chloroform-d) δ 9.09 (s, 1H), 8.78 (s, 1H), 8.44 (d, J = 1.0 Hz, 1H), 8.06 (s, 2H), 7.62 (s, 1H), 7.49 (d, J = 9.0 Hz, 2H), 7.04 (s, 1H), 3.99 (s, 3H), 3.92 (s, 3H), 2.62 (s, 3H), 2.52 (s, 3H).

**Example 166: N4-(2,6-dimethylpyrimidin-4-yl)-N6-(3-methoxy-5-tetrahydropyran-2-yl-2-pyridyl)pyrimidine-4,6-diamine**

uPLC-MS: tr = 1.11 min. (98 %). LRMS (m/z): 404 (M+1).
1H NMR (400 MHz, Methanol-d4) δ 8.88 (s, 1H), 8.28 (s, 1H), 7.83 (d, J = 1.5 Hz, 1H), 7.24 (d, J = 1.6 Hz, 1H), 7.22 (s, 1H), 3.89 (s, 3H), 2.50 (s, 3H), 2.47 - 2.33 (m, 3H), 2.32 (s, 3H), 1.69 - 1.46 (m, 6H).

**Example 167: N-[2-[[6-[(2,6-dimethylpyrimidin-4-yl)amino]pyrimidin-4-yl]amino]-5-tetrahydropyran-2-yl-3-pyridyl]-N-methyl-methanesulfonamide**

uPLC-MS: tr = 1.12 min. (99 %). LRMS (m/z): 485 (M+1).
1H NMR (400 MHz, DMSO-d6) δ 10.30 (s, 1H), 8.89 (s, 1H), 8.41 (d, J = 0.9 Hz, 1H), 8.30 (d, J = 1.9 Hz, 1H), 7.96 (d, J = 1.9 Hz, 1H), 7.27 (s, 1H), 4.46 - 4.39 (m, 1H), 4.05 (d, J = 11.2 Hz, 1H), 3.57 (td, J = 11.2, 3.9 Hz, 1H), 3.15 (s, 3H), 2.52 (s, 3H), 2.33 (s, 3H), 1.88 (t, J = 10.0 Hz, 2H), 1.60 (tdd, J = 23.9, 13.5, 3.5 Hz, 4H).

**Example 168: N6-[5-(1,5-dimethylpyrazol-4-yl)-3-(N,S-dimethylsulfonimidoyl)-2-pyridyl]-N4-(2,6-dimethylpyrimidin-4-yl)pyrimidine-4,6-diamine**

uPLC-MS: tr = 1.09 min. (93 %). LRMS (m/z): 480 (M+1).
1H NMR (400 MHz, Chloroform-d) δ 10.43 (s, 1H), 8.75 (s, 1H), 8.58 - 8.53 (m, 2H), 8.20 (d, J = 2.3 Hz, 1H), 7.60 (s, 1H), 7.56 (s, 1H), 7.37 (s, 1H), 3.89 (s, 3H), 3.18 (s, 3H), 2.84 (s, 3H), 2.65 (s, 3H), 2.49 (s, 3H), 2.42 (s, 3H).

**Example 169: N6-(2,6-dimethylpyrimidin-4-yl)-N4-[3-methoxy-4-[1-(2-methoxyethyl)-1,2,4-triazol-3-yl]-2-pyridyl]pyrimidine-4,6-diamine**

uPLC-MS: tr = 1.03 min. (97 %). LRMS (m/z): 449 (M+1).

1H NMR (400 MHz, Chloroform-d) δ 2.49 (s, 3H), 2.67 (s, 3H), 3.37 (s, 3H), 3.77 - 3.84 (m, 2H), 3.88 (s, 3H), 4.39 - 4.49 (m, 2H), 7.42 (s, 1H), 7.58 (d, J = 5.2 Hz, 1H), 7.63 (s, 1H), 8.17 (d, J = 5.2 Hz, 1H), 8.22 (s, 1H), 8.26 (s, 1H), 8.51 (d, J = 1.0 Hz, 1H), 8.84 (s, 1H).

**Example 170: N-[2-[[6-[(2,6-dimethylpyrimidin-4-yl)amino]pyrimidin-4-yl]amino]-5-(4-methyl-1,3-oxazol-5-yl)pyridin-3-yl]-N-methylmethanesulfonamide,**

HPLC-MS: tr = 1.70 min. (100 %). LRMS (m/z): 482 (M+1).
1H NMR (400 MHz, CD3OD) δ 8.74-8.67 (m, 1H), 8.66 (s, 1H), 8.62 (d, J=2 Hz, 1H), 8.24 (s, 1H), 8.18 (d J = 5Hz, 1H), 3.36 (s, 3H), 3.17 (s, 3H), 2.75 (s, 3H), 2.61 (s, 3H), 2.45 (s, 3H).

**Example 171: 4-N-(5-cyclopropyl-3-dimethylphosphorylpyridin-2-yl)-6-N-(2,6-dimethylpyrimidin-4-yl)pyrimidine-4,6-diamine**

HPLC-MS: tr = 1.63 min. (98 %). LRMS (m/z): 410 (M+1).
1H NMR (400 MHz, CD3OD) δ 8.59 (d, J = 5 Hz, 1H), 8.52-8.43 (bs, 1H), 8.35-8.34 (m, 1H), 8.97-7.85 (bs, 1H), 7.72-7.68 (m, 1H), 2.73 (s, 3H), 2.59 (s, 3H), 1.99-1.98 (m, 1H), 1.97 (s, 3H), 1.94 (s, 3H), 1.08-1.06 (m, 2H), 0.80-0.78 (m, 2H).

**Example 172: 2-[2-[[6-[(2,6-dimethylpyrimidin-4-yl)amino]pyrimidin-4-yl]amino]pyridin-3-yl]propan-2-ol,**

HPLC-MS: tr = 1.46 min. (95 %). LRMS (m/z): 352 (M+1).
1H NMR (400 MHz, DMSO-d6) δ 11.54 (s, 1H), 10.68 (s, 1H), 9.03-8.85 (bs, 1H), 8.54 (s, 1H), 8.26 (d, J = 5 Hz, 1H), 7.70 (m, 2H), 7.06 (m, 2H), 6.54-6.19 (bs, 1H), 2.67 (s, 3H), 2.53 (s, 3H), 1.56 (s, 6H).

**Example 173: N4-[5-(1,5-dimethylpyrazol-4-yl)-3-methylsulfinyl-2-pyridyl]-N6-(2,6-dimethylpyrimidin-4-yl)pyrimidine-4,6-diamine**

uPLC-MS: tr = 0.90 min. (93 %). LRMS (m/z): 450 (M+1).
1H NMR (400 MHz, Methanol-d4) δ 8.64 (s, 1H), 8.46 (d, J = 2.3 Hz, 1H), 8.29 (d, J = 1.0 Hz, 1H), 7.92 (d, J = 2.3 Hz, 1H), 7.56 (s, 1H), 7.20 (s, 1H), 4.48 (s, 3H), 3.77 (s, 3H), 2.93 (s, 3H), 2.48 (s, 3H), 2.33 (s, 3H).

**Example 174: N4-(5-fluoro-6-methyl-2-pyridyl)-N6-[3-methoxy-4-(1-methyl-1,2,4-triazol-3-yl)-2-pyridyl]pyrimidine-4,6-diamine**

HPLC-MS: tr = 2.02 min. (98 %). LRMS (m/z): 408.
1H NMR (400 MHz, DMSO-d6) δ 2.46 (d, J = 2.9 Hz, 3H), 3.82 (s, 3H), 3.99 (s, 3H), 7.42 (dd, J = 9.0, 3.0 Hz, 1H), 7.50 (d, J = 5.2 Hz, 1H), 7.58 (t, J = 9.0 Hz, 1H), 8.16 (d, J = 5.2 Hz, 1H), 8.36 (d, J = 1.0 Hz, 1H), 8.45 (s, 1H), 8.66 (s, 1H), 8.93 (s, 1H), 10.04 (s, 1H). (M+1).

**Example 175: 6-N-(2,6-dimethylpyrimidin-4-yl)-4-N-[3-methylsulfonyl-5-(oxan-2-yl)pyridin-2-yl]pyrimidine-4,6-diamine**

HPLC-MS: tr = 1.94 min. (97 %). LRMS (m/z): 456.
1H NMR (400 MHz, Cl3CD) δ 9.51 (s, 1H), 8.61 (s, 1H), 8.55 (s, 1H), 8.52 (s, 1H), 8.27 (s, 1H), 7.62 (s, 1H), 7.43 (s, 1H), 4.43-4.40 (m, 1H), 4.18-4.15 (m, 1H), 3.65-3.64 (m, 1H), 3.14 (s, 3H), 2.65 (s, 3H), 2.50 (s, 3H), 2.01-2.00 (m, 1H), 1.90-1,87 (m,1H), 1.85-1.60 (m, 4H).

**Example 176: N6-[5-(1,5-dimethylpyrazol-4-yl)-3-methoxy-6-methyl-2-pyridyl]-N4-(6-methylpyrimidin-4-yl)pyrimidine-4,6-diamine**

uPLC-MS: tr = 1.16 min. (97 %). LRMS (m/z): 418.
1H NMR (400 MHz, Chloroform-d) δ 8.87 (d, J = 0.9 Hz, 1H), 8.78 - 8.74 (m, 1H), 8.49 (d, J = 0.8 Hz, 1H), 7.99 (s, 1H), 7.56 (d, J = 12.9 Hz, 2H), 7.41 (s, 1H), 6.89 (d, J = 5.8 Hz, 1H), 5.30 (s, 1H), 3.88 (d, J = 3.5 Hz, 6H), 2.52 (s, 3H), 2.41 (s, 3H), 2.19 (s, 3H).

## PHARMACOLOGICAL ACTIVITY

### TYK2 JH2 domain binding assay

TYK2 (JH2domain-pseudokinase) KINOMEscan assay. For the assay, TYK2 (JH2domain-pseudokinase)-tagged T7 phage strains were prepared in an E. coli host derived from the BL21 strain.E. coli were grown to log-phase and infected with T7 phage and incubated with shaking at 32°C until lysis. The lysates were centrifuged and filtered to remove cell debris. The remaining TYK2 (JH2domain-pseudokinase) were produced in HEK-293 cells and subsequently tagged with DNA for qPCR detection. Streptavidin-coated magnetic beads were treated with biotinylated small molecule ligands for 30 minutes at room temperature to generate affinity resins for kinase assays. The liganded beads were blocked with excess biotin and washed with blocking buffer (SeaBlock (Pierce), 1% BSA, 0.05% Tween 20, 1 mM DTT) to remove unbound ligand and to reduce non-specific binding. Binding reactions were assembled by combining TYK2 (JH2domain-pseudokinase), liganded affinity beads, and test compounds in 1x binding buffer (20% SeaBlock, 0.17x PBS, 0.05% Tween 20, 6 mM DTT). Test compounds were prepared as 111X stocks in 100% DMSO. Kds were determined using an 11-point 3-fold compound dilution series with three DMSO control points. All compounds for Kd measurements are distributed by acoustic transfer (non-contact dispensing) in 100% DMSO. The compounds were then diluted directly into the assays such that the final concentration of DMSO was 0.9%. All reactions performed in polypropylene 384-well plate. Each was a final volume of 0.02 ml. The assay plates were incubated at room temperature with shaking for 1 hour and the affinity beads were washed with wash buffer (1x PBS, 0.05% Tween 20). The beads were then re-suspended in elution buffer (1x PBS, 0.05% Tween 20, 0.5 μM non-biotinylated affinity ligand) and incubated at room temperature with shaking for 30 minutes. The kinase concentration in the eluates was measured by qPCR. Confidential SDZ179-01-s-00001 11/1/2019 2 of 6

Compound Handling

An 11-point 3-fold serial dilution of each test compound was prepared in 100% DMSO at 100x final test concentration and subsequently diluted to 1x in the assay (final DMSO concentration = 1%). Most Kds were determined using a compound top concentration = 30,000 nM. If the initial Kd determined was < 0.5 nM (the lowest concentration tested), the measurement was repeated with a serial dilution starting at a lower top concentration. A Kd value reported as 40,000 nM indicates that the Kd was determined to be >30,000 nM.

Binding Constants (Kds)
Binding constants (Kds) were calculated with a standard dose-response curve using the Hill equation: Response = Background + Signal - Background

1 + (KdHill Slope / DoseHill Slope)

The Hill Slope was set to -1.

Curves were fitted using a non-linear least square fit with the Levenberg-Marquardt algorithm. Affinities for Tyk2 JH2 Domain Binding Assay are presented in Table 1. Compounds denoted as 'A" have a Kd lower than 200 pM; compounds denoted as "B" have a Kd between 200 pM and 1 nM; compounds denoted as 'C" have a Kd between 1 nM and 10 nM, and compounds denoted as "D" have a Kd greater than 10 nM.

TABLE 1: Kd Affinities for Tyk2 JH2 Domain Binding Assay.

| Compound Example | TYK2 JH2 Kd |
|---|---|
| 1 | C |
| 2 | D |
| 3 | C |
| 4 | B |
| 5 | C |
| 6 | B |

(continued)

| Compound Example | TYK2 JH2 Kd |
|---|---|
| 7 | D |
| 8 | D |
| 9 | D |
| 10 | D |
| 11 | B |
| 12 | A |
| 13 | D |
| 14 | B |
| 15 | B |
| 16 | C |
| 18 | C |
| 19 | C |
| 20 | B |
| 21 | C |
| 22 | D |
| 23 | C |
| 24 | A |
| 25 | A |
| 26 | B |
| 27 | D |
| 28 | B |
| 29 | C |
| 30 | B |
| 31 | B |
| 32 | A |
| 33 | A |
| 34 | A |
| 35 | D |
| 36 | C |
| 37 | A |
| 38 | B |
| 39 | B |
| 40 | C |
| 41 | A |
| 42 | C |
| 43 | B |
| 44 | B |
| 45 | B |

(continued)

| Compound Example | TYK2 JH2 Kd |
| --- | --- |
| 46 | A |
| 47 | B |
| 48 | D |
| 49 | B |
| 50 | C |
| 51 | A |
| 52 | B |
| 53 | B |
| 55 | B |
| 61 | C |
| 62 | A |
| 63 | B |
| 64 | B |
| 65 | B |
| 66 | B |
| 67 | A |
| 69 | A |
| 70 | A |
| 71 | A |
| 72 | C |
| 73 | D |
| 75 | A |
| 76 | A |
| 77 | A |
| 78 | C |
| 80 | A |
| 81 | D |
| 82 | C |
| 83 | C |
| 84 | A |
| 85 | C |
| 86 | A |
| 87 | B |
| 88 | A |
| 89 | A |
| 90 | C |
| 91 | C |
| 92 | A |

(continued)

| Compound Example | TYK2 JH2 Kd |
|---|---|
| 93 | C |
| 94 | A |
| 95 | A |
| 96 | C |
| 97 | A |
| 99 | B |
| 100 | D |
| 101 | A |
| 102 | B |
| 103 | A |
| 104 | B |
| 105 | A |
| 106 | A |
| 107 | A |
| 108 | A |
| 109 | A |
| 110 | C |
| 111 | A |
| 112 | A |
| 113 | A |
| 114 | A |
| 115 | A |
| 116 | B |
| 117 | C |
| 119 | A |
| 120 | A |
| 121 | A |
| 122 | A |
| 123 | C |
| 124 | A |
| 125 | A |
| 126 | A |
| 127 | B |
| 128 | A |
| 129 | A |
| 130 | B |
| 131 | A |
| 132 | B |

(continued)

| Compound Example | TYK2 JH2 Kd |
|---|---|
| 133 | A |
| 134 | B |
| 135 | A |
| 136 | A |
| 137 | A |
| 138 | A |
| 139 | A |
| 140 | A |
| 141 | A |
| 142 | A |
| 143 | C |
| 144 | A |
| 145 | A |
| 146 | A |
| 147 | A |
| 148 | A |
| 149 | A |
| 150 | A |
| 151 | A |
| 152 | A |
| 153 | A |
| 154 | A |
| 155 | B |
| 156 | C |
| 157 | B |
| 158 | A |
| 159 | A |
| 160 | A |
| 161 | A |
| 162 | A |
| 163 | A |
| 164 | A |
| 165 | B |
| 166 | C |
| 167 | A |
| 168 | A |
| 169 | A |
| 170 | A |

(continued)

| Compound Example | TYK2 JH2 Kd |
|---|---|
| 171 | A |
| 172 | B |
| 176 | B |

**IFNa induced STAT phosphorylation in HEK293 cells**

Stimulation of HEK293T-sie-bla cells with interferon alpha (INFa) activates the signaling pathway of STAT1/3 through TYK2/JAK1. In this assay, measurement of the activity of the reporter gene □-lactamase expression was used as the readout.

CellSensorTM SIE-bla HEK293T cell line were purchased from Thermo Fisher Scientific (ref. #K1649B) and cultured as specified by the provider. A number of 30.000 cells per well (40 μl) were plated in a 384-well plate (Corning ref.# 3712) and cultured overnight at 37°C with 5% CO2 in a complete Optimem media (ThermoFisher ref. #11058-021) containing 0.5% (v/v) heat inactivated dialyzed FBS (Life Technologies ref. #26400044), 10 μM non-essential aminoacids NEAA (Sigma ref. #M7145), 100 μM Sodium Pyruvate (Gibco ref. #11360-039) and 100 U/mL Penicillin (Sigma ref. #P0781). Compounds were initially dissolved in 100% DMSO. Compound curves were then prepared using a 1/5 serial dilution (10 dilutions). The dilutions were directly diluted in complete Optimem media at 40-fold their final concentration in 20% DMSO. IFNa initial solution (R&D systems ref. #11100-1) was prepared at 10-fold its EC80 in complete Optimem media. Compounds and IFNa were added simultaneously to the wells with cells and incubated during 4 hours at 37°C (the final DMSO was 0.1%). The □-lactamase substrate (LifeTechnologies ref.#K1138) was prepared at 6-fold its final concentration as specified by the provider. Then 10 μL of the mixture was added to the wells to stop the stimulation. Plate was left in the dark over-night. Then a FRET reading was performed on an appropriate plate reader.

All experiments were analysed using an ExcelFit template and the four-parameter log equation. Inhibitions of IFNa induced STAT phosphorylation in HEK293 cells are presented in Table 2. Compounds denoted as 'A" have a IC50 lower than 10 nM; compounds denoted as "B" have a IC50 between 10 nM and 100 nM; compounds denoted as 'C" have a IC50 between 100 nM and 1 μM, and compounds denoted as "D" have a IC50 greater than 1 μM.

TABLE 2: IC50 Inhibitions of IFNa induced STAT phosphorylation in HEK293 cells

| Compound Example | Inhibition of IFNa induced STAT phosphorylation HEK293 cells IC50 |
|---|---|
| 1 | C |
| 2 | D |
| 3 | D |
| 4 | B |
| 5 | D |
| 6 | B |
| 7 | D |
| 8 | D |
| 9 | D |
| 10 | D |
| 11 | B |
| 12 | D |
| 13 | D |
| 14 | C |
| 15 | B |
| 16 | D |
| 17 | D |

(continued)

| Compound Example | Inhibition of IFNa induced STAT phosphorylation HEK293 cells IC50 |
|---|---|
| 18 | D |
| 19 | D |
| 20 | C |
| 21 | D |
| 22 | C |
| 23 | C |
| 24 | B |
| 25 | A |
| 26 | C |
| 27 | D |
| 28 | D |
| 29 | D |
| 30 | C |
| 31 | C |
| 32 | A |
| 33 | B |
| 34 | A |
| 3 | D |
| 36 | D |
| 37 | B |
| 38 | B |
| 39 | C |
| 40 | C |
| 41 | B |
| 42 | D |
| 43 | C |
| 44 | B |
| 45 | C |
| 46 | B |
| 47 | B |
| 48 | D |
| 49 | D |
| 50 | C |
| 51 | B |
| 52 | C |
| 53 | C |
| 54 | C |
| 55 | B |

(continued)

| Compound Example | Inhibition of IFNa induced STAT phosphorylation HEK293 cells IC50 |
|---|---|
| 56 | D |
| 57 | B |
| 58 | C |
| 59 | B |
| 60 | B |
| 61 | C |
| 62 | B |
| 63 | B |
| 64 | C |
| 65 | B |
| 66 | C |
| 67 | B |
| 69 | B |
| 70 | A |
| 71 | B |
| 72 | C |
| 73 | D |
| 75 | B |
| 76 | B |
| 77 | B |
| 78 | D |
| 79 | D |
| 80 | B |
| 81 | D |
| 83 | D |
| 84 | B |
| 85 | B |
| 86 | A |
| 87 | D |
| 88 | B |
| 89 | B |
| 90 | D |
| 91 | C |
| 92 | A |
| 93 | D |
| 94 | A |
| 95 | A |
| 96 | C |

(continued)

| Compound Example | Inhibition of IFNa induced STAT phosphorylation HEK293 cells IC50 |
|---|---|
| 97 | B |
| 98 | D |
| 99 | B |
| 100 | D |
| 101 | B |
| 102 | B |
| 103 | A |
| 104 | C |
| 105 | A |
| 106 | B |
| 107 | A |
| 108 | A |
| 109 | A |
| 110 | C |
| 111 | B |
| 112 | B |
| 113 | A |
| 114 | A |
| 115 | A |
| 116 | B |
| 117 | D |
| 118 | A |
| 119 | A |
| 120 | B |
| 121 | B |
| 122 | B |
| 123 | B |
| 124 | A |
| 125 | A |
| 126 | A |
| 127 | B |
| 128 | C |
| 129 | B |
| 130 | C |
| 131 | B |
| 132 | C |
| 133 | A |
| 134 | D |

(continued)

| Compound Example | Inhibition of IFNa induced STAT phosphorylation HEK293 cells IC50 |
|---|---|
| 135 | C |
| 136 | A |
| 137 | A |
| 138 | A |
| 139 | A |
| 140 | B |
| 141 | B |
| 142 | B |
| 143 | D |
| 144 | B |
| 145 | B |
| 146 | A |
| 147 | B |
| 148 | B |
| 149 | A |
| 150 | A |
| 151 | A |
| 152 | A |
| 153 | A |
| 154 | B |
| 155 | C |
| 156 | D |
| 157 | B |
| 158 | B |
| 159 | A |
| 160 | B |
| 161 | B |
| 162 | A |
| 163 | A |
| 164 | B |
| 165 | C |
| 166 | C |
| 167 | A |
| 168 | A |
| 169 | B |
| 170 | B |
| 171 | B |
| 172 | C |

(continued)

| Compound Example | Inhibition of IFNa induced STAT phosphorylation HEK293 cells IC50 |
|---|---|
| 173 | A |
| 174 | B |
| 175 | A |
| 176 | B |

## IL4 induced STAT phosphorylation in Ramos cells

Stimulation of RAMOS cells with interleukine IL-4 activates the signaling pathway of STAT6 through JAK1/JAK3. In this assay, measurement of the activity of the reporter gene β-lactamase expression was used as the readout.

Ramos STAT6-bla RA-1 cell line were purchased from Life Technologies (ref. #K1243) and cultured as specified by the provider. A number of 30.000 cells per well (35 μl) were plated in a 384-well plate (Corning ref.# 3712) and cultured overnight at 37°C with 5% CO2 in a complete Optimem media (Life Technologies ref. #11058-021) containing 556 μg/mL CD40 (Life Technologies ref. #PHP0025), 0.5% (v/v) heat inactivated FBS (Life Technologies ref. #10082-147), 10 μM non-essential aminoacids NEAA (Sigma ref. #M7145), 100 μM Sodium Pyruvate (Gibco ref. #11360-039) and 100 U/mL Penicillin (Sigma ref. #P0781). Compounds were initially dissolved in 100% DMSO. Compound curves were then prepared using a 1/5 serial dilution (10 dilutions). The dilutions were directly diluted in complete Optimem media without CD40 at 40-fold their final concentration in 20% DMSO. IL-4 initial solution (Prepotech ref. #200-04) was prepared at 10-fold its EC80 in complete Optimem media without CD40. Compounds and IL-4 were added simultaneously to the wells with cells and incubated during 4 hours at 37°C (the final DMSO was 0.1%). The β-lactamase substrate (LifeTechnologies ref.#K1138) was prepared at 6-fold its final concentration as specified by the provider. Then 10 μL of the mixture was added to the wells to stop the stimulation. Plate was left in the dark over-night. Then a FRET reading was performed on an appropriate plate reader.

All experiments were analysed using an ExcelFit template and the four-parameter log equation. Inhibitions of IL4 induced STAT phosphorylation in Ramos cells are presented in Table 3. As % vs compound concentration.

TABLE 3: Inhibition of IL4 induced STAT phosphorylation in Ramos cells.

| Compound Example | Compoound concentration, μM | % Inhibition of IL4 induced STAT phosphorylation Ramos cells |
|---|---|---|
| 1 | 5 | 25 |
| 4 | 5 | 33 |
| 6 | 5 | 62 |
| 12 | 5 | 46 |
| 25 | 5 | 57 |
| 32 | 5 | 70 |
| 37 | 5 | 44 |
| 70 | 5 | 78 |
| 70 | 5 | 55 |
| 80 | 5 | 47 |
| 86 | 25 | 78 |
| 103 | 5 | 49 |
| 119 | 5 | 47 |
| 124 | 5 | 50 |
| 125 | 5 | 46 |
| 126 | 25 | 54 |
| 135 | 5 | 37 |

(continued)

| Compound Example | Compoound concentration, μM | % Inhibition of IL4 induced STAT phosphorylation Ramos cells |
|---|---|---|
| 137 | 25 | 0 |
| 138 | 5 | 6 |
| 139 | 5 | 28 |

## COMBINATIONS

The pyrimidine derivatives of the present invention may also be combined with other active compounds in the treatment of a pathological condition or disease susceptible to amelioration by inhibition of Tyrosine kinase 2 (Tyk2).

The combination of the invention can optionally comprise one or more additional active substances which are known to be useful in the treatment of a dermatological disease, an inflammatory or autoimmune-mediated disease and a metabolism/endocrine function disorder; more in particular wherein the pathological condition or disease is selected from plaque psoriasis, scalp psoriasis, genital psoriasis, pustular psoriasis, psoriatic erythroderma, acrodermatitis continua, pityriasis rubra pilaris, lichen planus, hidradenitis suppurativa, bullous pemphigoid, pyoderma gangrenousm (PASH), ichthyosis, atopic dermatitis, psoriatic arthritis, ankylosing spondylitis, systemic sclerosis, primary biliary cholangitis, asthma and human multiple myeloma, such as,

a) Corticosteroids, such as prednisone, methylprednisolone or beta-methasone;
b) Immunosuppressants, such as cyclosporine, tacrolimus methotrexate, hydroxyurea, mycophenolate mofetil, mycophenolic acid, sulfasalazine, 6-thioguanine or azathioprine;
c) Fumaric acid esters, such as dimethyl fumarate or tepilamide fumarate;
d) Dihydroorotate dehydrogenase (DHODH) inhibitors such as leflunomide;
e) Retinoids, such as acitretin or isotretinoin;
f) Anti-inflammatories such as apremilast, crisaborole, celecoxib, diclofenac, aceclofenac, aspirin or naproxen;
g) Antibiotics such as gentamicin;
h) Antivirals such as aciclovir, efavirenz or ribavirin
i) Anti-cancer agents such as lenalidomide, pomalidomide, pembrolizumab, nivolumab, daratumumab, bortezomib, carfilzomib, ixazomib, bendamustine or ventoclast;
j) T-cell blockers such as alefacept or efalizumab;
k) Tumor necrosis factor-alpha (TNF-alpha) blockers such as etanercept, adalimumab, infliximab, golimumab, certolizumab pegol;
l) anti-IL4/IL13 antagonist such as dupilumab, lebrikizumab or tralokinumab;
o) IL-1b blockers such as canakinumab;
p) IL-alpha blockers such as bermekimab;
q) CD6 blockers such as itolizumab;
r) IL-36R blockers such as BI-655130 orANB019;
s) IL-6 antagonist such as tocilizumab;
t) Calcineurin inhibitors such as pimecrolimus, tacrolimus or cyclosporine;
u) Phototherapy agents commonly employed in phototherapy such as psoralen, methoxypsoralen or 5-methoxypsoralen + UVA (PUVA) or treatment with UVB (with or without tar);
v) Fixed combinations of corticosteroids and vitamin D derivatives;
w) Fixed combinations of corticosteroids and retinoids;
x) Corticosteroid tapes;
y) and other combinable agents that may include piclidenoson, LYC-30937, LEO-32731, BI-730357, PRCL-02, LNP-1955, GSK-2982772, CBP-307, KD-025, MP-1032, petesicatib, JTE-451, Hemay-005, SM-04755, EDP-1815, BI-730460, GLPG3970, SFA-002 ER, JNJ-3534, SAR-441169, BOS-172767, SCD-044, CUDC-305. ABBV-157, BAY-1834845, AUR-101, R-835, PBF-1650, IMU-935, RTA-1701, AZD-0284, CD20 antagonist, salicylic acid, coal tar, Mical-1, DUR-928, AM-001, BMX-010, TA-102, SNA-125, KP-470, pegcantratinib, pefcalcitol, ESR-114, tapinarof, NP-000888, SM-04755, BOS-475, SB-414, LEO-134310, CBS-3595, PF-06763809, XCUR-17 or BTX-1308, MGC topical cream, RIST4721 and aminopterin sodium.

## PHARMACEUTICAL COMPOSITIONS

Pharmaceutical compositions according to the present invention comprise the pyrimidine substituted derivatives of the invention in association with a pharmaceutically acceptable diluent or carrier.

As used herein, the term pharmaceutical composition refers to a mixture of one or more of the pyrimidine substituted derivatives of the invention or prodrugs thereof, with other chemical components, such as physiologically/pharmaceutically acceptable carriers and excipients. The purpose of a pharmaceutical composition is to facilitate administration of a compound to an organism.

As used herein, a physiologically/pharmaceutically acceptable diluent or carrier refers to a carrier or diluent that does not cause significant irritation to an organism and does not abrogate the biological activity and properties of the administered compound.

The invention further provides pharmaceutical compositions comprising the pyrimidine substituted derivatives of the invention in association with a pharmaceutically acceptable diluent or carrier together with one or more other therapeutic agents for use in the treatment of a pathological condition or disease susceptible to amelioration by inhibiton of Tyk2, such as the ones previously described.

The invention also encompasses the use of a pharmaceutical composition of the invention for the manufacture of a medicament for treating a pathological condition or disease susceptible to amelioration by inhibiton of Tyk2, such as the ones previously described.

The invention also provides a method of treatment of a pathological condition or disease susceptible to amelioration by inhibiton of Tyk2, such as the ones previously described, the method comprising administering a therapeutically effective amount of a pharmaceutical composition of the invention.

The present invention also provides pharmaceutical compositions which comprise, as an active ingredient, at least a pyrimidine substituted derivative of the invention in association with a pharmaceutically acceptable excipient such as a carrier or a diluent. Preferably the pharmaceutical compositions of the invention are made up in a form suitable for oral, topical, nasal, rectal, percutaneous or injectable administration.

In a preferred embodiment, the compositions are made up in a form sutiable for topical administration.

In a preferred embodiment, the compositions are made up in a form suitable for oral administration.

Pharmaceutical compositions suitable for the delivery of pyrimidine derivatives of the invention and methods for their preparation will be readily apparent to those skilled in the art. Such compositions and methods for their preparation can be found, for example, in Remington: The Science and Practice of Pharmacy, 21st Edition, Lippincott Williams & Wilkins, Philadelphia, Pa., 2001.

i) Topical Administration

The pyrimidine substituted derivatives of the invention may be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibers, bandages and microemulsions. Other means of topical administration include delivery by electroporation, iontophoresis, phonophoresis, sonophoresis and microneedle or needle-free injection.

Formulations for topical administration may be formulated to be immediate and/or modified release. Modified release formulations include delayed-, sustained-, pulsed-, controlled-, targeted and programmed release.

ii) Oral Administration

The pyrimidine substituted derivatives of the invention may be administered orally (peroral administration; *per os* (latin)). Oral administration involves swallowing, so that the compound is absorbed from the gut and delivered to the liver via the portal circulation (hepatic first pass metabolism) and finally enters the gastrointestinal (GI) tract.

Compositions for oral administration may take the form of tablets, retard tablets, sublingual tablets, capsules, inhalation aerosols, inhalation solutions, dry powder inhalation, or liquid preparations, such as mixtures, solutions, elixirs, syrups or suspensions, all containing the compound of the invention; such preparations may be made by methods well-known in the art. The active ingredient may also be presented as a bolus, electuary or paste.

iii) Oral mucosal administration

The pyrimidine substituted derivatives of the invention can also be administered via the oral mucosal. Within the oral mucosal cavity, delivery of drugs is classified into three categories: (a) sublingual delivery, which is systemic delivery of drugs through the mucosal membranes lining the floor of the mouth, (b) buccal delivery, which is drug administration through the mucosal membranes lining the cheeks (buccal mucosa), and (c) local delivery, which is drug

delivery into the oral cavity.

Pharmaceutical products to be administered via the oral mucosal can be designed using mucoadhesive, quick dissolve tablets and solid lozenge formulations, which are formulated with one or more mucoadhesive (bioadhesive) polymers and/or oral mucosal permeation enhancers.

iv) Inhaled administration

The pyrimidine substituted derivatives of the invention can also be administered by inhalation, typically in the form of a dry powder from a dry powder inhaler or as an aerosol spray from a pressurized container, pump, spray, atomizer (preferably an atomizer using electrohydrodynamics to produce a fine mist), or nebulizer, with or without the use of a suitable propellant.

v) Nasal mucosal administration

The pyrimidine substituted derivatives of the invention may also be administered via the nasal mucosal.

Typical compositions for nasal mucosa administration are typically applied by a metering, atomizing spray pump and are in the form of a solution or suspension in an inert vehicle such as water optionally in combination with conventional excipients such as buffers, antimicrobials, tonicity modifying agents and viscosity modifying agents

vi) Parenteral Administration

The pyrimidine substituted derivatives of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilization, may readily be accomplished using standard pharmaceutical techniques well known to those skilled in the art. The solubility of compounds of the invention used in the preparation of parenteral solutions may be increased by the use of appropriate formulation techniques, such as the incorporation of solubility-enhancing agents.

vii) Rectal/Intravaginal Administration

The pyrimidine substituted derivatives of the invention may be administered rectally or vaginally, for example, in the form of a suppository, pessary, or enema. Cocoa butter is a traditional suppository base, but various alternatives may be used as appropriate. Formulations for rectal/vaginal administration may be formulated to be immediate and/or modified release. Modified release formulations include delayed-, sustained-, pulsed-, controlled-, targeted and programmed release.

viii) Ocular Administration

The pyrimidine substituted derivatives of the invention may also be administered directly to the eye or ear, typically in the form of drops of a micronized suspension or solution in isotonic, pH- adjusted, sterile saline. Other formulations suitable for ocular and aural administration include ointments, biodegradable {e.g. absorbable gel sponges, collagen) and nonbiodegradable (e.g. silicone) implants, wafers, lenses and particulate or vesicular systems, such as niosomes or liposomes. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

The amount of the active pyrimidine derivative of the invention administered will be dependent on the subject being treated, the severity of the disorder or condition, the rate of administration, the disposition of the compound and the discretion of the prescribing physician. However, an effective dosage is typically in the range of 0.01-3000 mg, more preferably 0.5-1000 mg of active ingredient or the equivalent amount of a pharmaceutically acceptable salt thereof per day.

Daily dosage may be administered in one or more treatments, preferably from 1 to 4 treatments, per day.

Preferably, the pharmaceutical compositions of the invention are made up in a form suitable for oral or topical

administration.

The amount of each active which is required to achieve a therapeutic effect will, of course, vary with the particular active, the route of administration, the subject under treatment, and the particular disorder or disease being treated.

## Claims

**1.** A pyrimidine substituted derivative, which pyrimidine substituted derivative is a compound of Formula (I), or a pharmaceutically acceptable salt, or a solvate, or a N-oxide, or a tautomer, or a stereoisomer, or an isotopically-labelled derivative thereof:

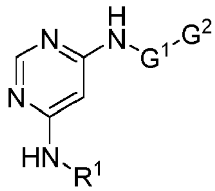

Formula (I)

wherein:

- $G^1$ represents a phenyl group or a 5- to 6-membered heteroaryl group containing one atom of N, wherein the phenyl and heteroaryl groups are unsubstituted or substituted by one or more substituents selected from a linear or branched $C_{1-4}$ alkyl group, a linear or branched $C_{1-3}$ alkenyl, a linear or branched $C_{1-4}$ alkoxy group, a halogen atom, a linear or branched $C_{1-4}$ haloalkyl group, a linear or branched $C_{1-2}$ haloalkoxy group, a CN group, a $-P(O)(CH_3)_2$ group, a linear or branched $C_{1-3}$ hydroxyalkyl group, a linear or branched $-(C_{1-4}$ alkyl)-($C_{1-4}$ alkoxy) group, a $-NR^aSO_2R^b$ group, a -(linear or branched $C_{1-3}$ alkyl group)$_{0-1}$-$SO_nCH_3$ group, a $-SO_2NR^cR^d$ group, a $-S(O)Me=NR^e$ group and a $-C(O)-R^f$ group;
- $G^2$ is absent or represents a $-(CH_2)$-phenyl group, a $C_{3-6}$ cycloalkyl group, a 5- to 6-membered heteroaryl group containing at least one heteroatom selected from N, O and S, and a 5- to 6-membered heterocyclyl group containing at least one heteroatom selected from N, O and S, wherein the cycloalkyl, heteroaryl and heterocyclyl groups are unsubstituted or substituted by one or more substituents selected from a linear or branched $C_{1-3}$ alkyl group, a linear or branched $C_{1-3}$ haloalkyl, a linear or branched $-(C_{1-4}$ alkyl)-($C_{1-4}$ alkoxy) group, a halogen atom, a $-(CH_2)_{0-1}$-4- to 6- membered heterocyclyl group containing at least one heteroatom selected from N, O and S, a $-C(O)-R^9$, and a $-(CH_2)_{0-1}-C_{3-6}$, cycloalkyl group, wherein the cycloalkyl group is unsubstituted or substituted by one or more substituents selected from halogen atom and a hydroxyl group;
- $R^1$ represents a $-C(O)-R^2$ group or a $G^3$ group;
- $R^2$ represents a monocyclic $C_{3-6}$ cycloalkyl group, wherein the $C_{3-6}$ cycloalkyl group is unsubstituted or substituted by one or more halogen atom;
- $G^3$ represents a 5- to 6- membered N-heteroaryl group, wherein the 5- to 6- membered N-heteroaryl group is substituted by one or more substituents selected from a linear or branched $C_{1-4}$ alkyl group, a fluorine atom, a CN group and a 6-membered heterocyclyl group containing at least one atom of O;
- $R^a$ represents a hydrogen atom, a linear or branched $C_{1-4}$ alkyl group or a linear or branched $C_{1-4}$ deuteroalkyl group;
- $R^b$ represents a group consisting of a linear or branched $C_{1-4}$ alkyl group, a linear or branched $C_{1-4}$ haloalkyl group or a $C_{3-6}$ cycloalkyl group;
- $R^c$ and $R^d$ together with the N atom to which they are attached form a 6-membered heterocyclyl group, which optionally contains in addition to the N-atom one additional heteroatom selected from N, O and S;
- $R^e$ represents a linear or branched $C_{1-3}$ alkyl group;
- $R^f$ represents a linear or branched $C_{1-5}$ hydroxyalkyl group, a 5-membered heterocyclyl group containing at least one heteroatom selected from N, O and S, a $-NH_2$ group or a linear or branched $C_{1-3}$ alkylamino group;
- $R^9$ represents a linear or branched $C_{1-4}$ alkoxy group or a heterocyclyl group containing at least one heteroatom selected from N, O and S; and
- n represents 1 or 2.

**2.** A pyrimidine derivative according to claim 1, wherein $G^1$ is selected from the group consisting of a phenyl group

and a pyridine group,
wherein the phenyl and pyridine groups are unsubstituted or substituted by one or more substituents selected from a linear or branched $C_{1-2}$ alkyl group, a linear or branched $C_{1-2}$ alkoxy group, a halogen atom, a linear or branched $C_{1-2}$ haloalkyl group, a CN group, a $-P(O)(CH_3)_2$ group, a $-NR^aSO_2R^b$ group, a linear or branched $C_{1-3}$ hydroxyalkyl group, a -(linear or branched $C_{1-2}$ alkyl group)$_{0-1}$-$SO_nCH_3$ group, a $-S(O)CH_3{=}NCH_3$ and a -C(O)-$R^f$ group.

**3.** A pyrimidine derivative according to claim 2, wherein the phenyl and pyridine groups are unsubstituted or substituted by one or more substituents selected from a linear or branched $C_{1-2}$ alkoxy group, a $-NR^aSO_2R^b$ group, a $SO_2CH_3$ group, a $-S(O)CH_3{=}NCH_3$ and a - $P(O)(CH_3)_2$ group.

**4.** A pyrimidine derivative according to claim 2 and 3, wherein $G^1$ represents a phenyl group, and wherein the phenyl is unsubstituted or substituted by one or more substituents selected from a methoxy group and a $NCH_3SO_2CH_3$ group.

**5.** A pyrimidine derivative according to claims 1 to 4, wherein $G^2$ is absent or is selected from the group consisting of a 5-membered heteroaryl group containing at least one heteroatom selected from N or O and a 5- to 6-membered heterocyclyl group containing at least one heteroatom selected from N or O;
wherein the heteroaryl and heterocyclyl groups are unsubstituted or substituted by one or more substituents selected from a linear or branched $C_{1-2}$ alkyl group or a linear or branched ($C_{1-3}$ alkyl) -($C_{1-3}$ alkoxy) group, a linear or branched $C_{1-2}$ haloalkyl group and a $C(O)R^g$ group.

**6.** A pyrimidine derivative according to claim 5, wherein $G^2$ represents a 5-membered heteroaryl group containing at least two atoms of N, wherein the heteroaryl is unsubstituted or substituted by one or more methyl group and a linear or branched $C_{1-2}$ haloalkyl group.

**7.** A pyrimidine derivative according to anyone of the preceding claims, wherein $R^1$ represents a -C(O)-$R^2$.

**8.** A pyrimidine derivative according to claim 7, wherein $R^2$ represents a cyclopropyl group, which is unsubstituted or substituted by one or more fluorine atoms.

**9.** A pyrimidine derivative according to claims 1 to 6, wherein $R^1$ represents a $G^3$ group.

**10.** A pyrimidine derivative according to claim 9 wherein the $G^3$ group represents a pyrimidinyl group, wherein the pyrimidinyl group is substituted by one or more substituents selected from a methyl group and a fluorine atom.

**11.** A pyrimidine derivative according to anyone of the preceding claims, wherein $R^a$ represents a methyl group.

**12.** A pyrimidine derivative according to anyone of the preceding claims, wherein $R^b$ represents a methyl group or a trifluoromethyl group.

**13.** A pyrimidine derivative according to claim 1, wherein:

- $G^1$ is selected from the group consisting of a phenyl group and a 6-membered heteroaryl group containing one atom of N,
wherein the phenyl and heteroaryl group are unsubstituted or substituted by one or more substituents selected from a linear or branched $C_{1-2}$ alkoxy group, a $-NR^aSO_2R^b$ group, a $SO_2CH_3$ group, a $-S(O)CH_3{=}NCH_3$ or a $-P(O)(CH_3)_2$ group;
- $G^2$ is selected from the group consisting of a 5-membered heteroaryl group containing at least one heteroatom selected from N or O and a 5- to 6-membered heterocyclyl group containing at least one heteroatom selected from N or O,
wherein the heteroaryl group is unsubstituted or substituted by one or more substituents selected from a linear or branched $C_{1-2}$ alkyl group or a linear or branched ($C_{1-3}$ alkyl) -($C_{1-3}$ alkoxy) group;
- $R^1$ represents a -C(O)-$R^2$;
- $R^2$ represents a cyclopropyl group, which is unsubstituted or substituted by one or more fluorine atoms;
- $R^a$ represents a methyl group; and
- $R^b$ represents a methyl group or a trifluoromethyl group.

**14.** A pyrimidine derivative according to claim 1, wherein:

- $G^1$ is selected from the group consisting of a phenyl group and a 6-membered heteroaryl group containing one atom of N, wherein the phenyl and heteroaryl group are unsubstituted or substituted by one or more substituents selected from a linear or branched $C_{1-2}$ alkyl group, a linear or branched $C_{1-2}$ alkoxy group, a halogen atom, a - $NR^aSO_2R^b$ group, a $SO_2CH_3$ group, a -$S(O)CH_3$=$NCH_3$ or a -$P(O)(CH_3)_2$ group;
- $G^2$ is selected from the group consisting of a 5-membered heteroaryl group containing at least one heteroatom selected from N or O and a 5- to 6-membered heterocyclyl group containing at least one heteroatom selected from N or O,
wherein the heteroaryl group is unsubstituted or substituted by one or more substituents selected from a linear or branched $C_{1-2}$ alkyl group, a linear or branched $C_{1-2}$ haloalkyl group or a linear or branched ($C_{1-3}$ alkyl) -($C_{1-3}$ alkoxy) group;
- $R^1$ represents a $G^3$ group;
- $G^3$ group represents a pyrimidinyl group, wherein the pyrimidinyl group is substituted by one or more substituents selected from methyl group and a fluorine atom.

**15.** A pyrimidine derivative according to claim 1, wherein:

- $G^1$ represents a phenyl group, wherein the phenyl is unsubstituted or substituted by one or more substituents selected from a methoxy group or a $NCH_3SO_2CH_3$ group;
- $G^2$ represents a 5-membered heteroaryl group containing at least two atoms of N, wherein the heteroaryl is unsubstituted or substituted by one or more methyl groups and a linear or branched $C_{1-2}$ haloalkyl groups.

**16.** A pyrimidine derivative according to claim 1, wherein:

- $G^1$ represents a phenyl group or a pyridine group, wherein the phenyl and pyridine groups are unsubstituted or substituted by one or more substituents selected from a methyl group, an allyl group, a methoxy group, an ethoxy group, a -$(CH_2)OCH_3$ group, a fluorine group, a bromine atom, a trifluoromethyl group, a trifluoromethoxy group, a -$(CH_2)_{0-2}SO_2CH_3$ group, a -$NR^aSO_2R^b$ group, a -CN group, a -S(O)Me=$NCH_3$ group, a $C(CH_3)_2OH$ group, a -$SO_2$-N-pyperidyl group, a -$PO(CH_3)_2$ group or a -$C(O)R^f$ group;
- $G^2$ is absent or selected from the group consisting of a pyrazolyl group, a pyrimidinyl group, an imidazolyl group, a triazolyl group, an oxadiazolyl group, a tetrazolyl group, a cyclopropyl group, a piperidinyl group, a $CH_2$-phenyl group, a pyrrolidinyl group, a tetrahydrofuranyl group, a tetrahydropyranyl group, a dihydropyranyl group, a pyridazinyl group and an oxazolyl group,
wherein the pyrazolyl, thea pyrimidinyl, the imidazolyl, the triazolyl, the oxadiazolyl, the tetrazolyl, the cyclopropyl, the piperidinyl, the $CH_2$-phenyl, the pyrrolidinyl, the tetrahydrofuranyl, the tetrahydropyranyl, the dihydropyranyl, the pyridazinyl and the oxazolyl groups are unsubstituted or substituted by one or more substituents selected from a methyl group, an ethyl group, a -$CF_3$ group, a -$CH_2OCH_3$ group, a -$(CH_2)_2OCH_3$ group, a fluorine atom, a -$CH_2CF_3$ group, a -$(CH_2)$-tetrahydropyranyl group, a-$(CH_2)$-morpholinyl group, $\alpha$-C(O)OtBu group, a-$(CH_2)$-tetrahydrofuranyl group, a -$(CH_2)_{0-1}$-oxetanyl group, a difluorosubstituted -$CH_2$-cyclobutyl group, a difluorosubstituted cyclopropyl group, a $CHF_2$ group and an hydroxyl substituted cyclobutyl group;
- $R^1$ represents a -C(O)-$R^2$ group or a $G^3$ group;
- $R^2$ represents a cyclopropyl group, wherein the cyclopropyl group is unsubstituted or substituted by a fluorine atom;
- $G^3$ represents a pyridyl group, a pyrimidinyl group, a pyridazinyl group, a triazolyl group or a pyrazolyl group, wherein the pyridyl, the pyrimidinyl, the pyridazinyl, the triazolyl and the pyrazolyl groups are substituted by one or more substituents selected from a methyl group, a tetrahydropyranyl group, a CN group and a fluorine atom;
- $R^a$ represents a hydrogen atom, a methyl group or a trideuteromethyl group;
- $R^b$ represents a methyl group or trifluoromethyl group; and
- $R^f$ represents a pyrrolidinyl group or a -$N(CH_3)_2$ group.

**17.** A pyrimidine derivative according to claims 1 to 16, wherein the compound of Formula (I) is one of:

N-[6-[2-methoxy-3-(1-methyl-1,2,4-triazol-3-yl)anilino]pyrimidin-4 yl]cyclopropanecarboxamide
N-[6-(2-methoxyanilino) pyrimidin-4-yl] cyclopropanecarboxamide
N-[6-(2-methoxy-3-methyl-anilino)pyrimidin-4-yl]cyclopropanecarboxamide
N6-(2,6-dimethylpyrimidin-4-yl)-N4-[2-methoxy-3-(1-methyl-1,2,4-triazol-3-yl)phenyl]pyrimidine-4,6-diamine
N6-(2,6-dimethylpyrimidin-4-yl)-N4-(3-methoxyphenyl)pyrimidine-4,6-diamine
N6-(2,6-dimethylpyrimidin-4-yl)-N4-(2-methoxy-3-methyl-phenyl)pyrimidine-4,6-diamine
N6-(2,6-dimethylpyrimidin-4-yl)-N4-(3-fluoro-2-methoxy-phenyl) pyrimidine-4,6-diamine

N4-(2,6-dimethylpyrimidin-4-yl)-N6-(3-methoxy-6-methyl-2-pyridyl)pyrimidine-4,6-diamine
N4-(2-methoxy-3-methyl-phenyl)-N6-(1-tetrahydropyran-4-ylpyrazol-4-yl)pyrimidine-4,6-diamine
N6-(2,6-dimethylpyrimidin-4-yl)-N4-[2-(trifluoromethoxy)phenyl] pyrimidine-4,6-diamine
N6-(2,6-dimethylpyrimidin-4-yl)-N4-(2-methylsulfonylphenyl)pyrimidine-4,6-diamine
N-[2-[[6-[(2,6-dimethylpyrimidin-4-yl)amino]pyrimidin-4-yl]amino]phenyl]-N-methyl-methanesulfonamide
N-methyl-N-[2-[[6-[(1-methyl-1,2,4-triazol-3-yl)amino]pyrimidin-4-yl]amino]phenyl]methanesulfonamide
N-[6-[2-[methyl(methylsulfonyl)amino]anilino]pyrimidin-4-yl]cyclopropanecarboxamide
N4-(2,6-dimethylpyrimidin-4-yl)-N6-[2-methoxy-3-(1-methylpyrazol-4-yl)phenyl]pyrimidine-4,6-diamine
3-[[6-[(2,6-dimethylpyrimidin-4-yl)amino]pyrimidin-4-yl]amino]-2-methoxy-benzonitrile
N4-(2,4-dimethoxyphenyl)-N6-(2,6-dimethylpyrimidin-4-yl)pyrimidine-4,6-diamine
N6-(2,6-dimethylpyrimidin-4-yl)-N4-[2-methoxy-3-(trifluoromethyl) phenyl] pyrimidine-4,6-diamine
N4-(3,4-difluoro-2-methoxy-phenyl)-N6-(2,6-dimethylpyrimidin-4-yl)pyrimidine-4,6-diamine
N4-(2,3-dimethoxyphenyl)-N6-(2,6-dimethylpyrimidin-4-yl)pyrimidine-4,6-diamine
N4-(2,6-dimethylpyrimidin-4-yl)-N6-(4-fluoro-2-methoxy-phenyl)pyrimidine-4,6-diamine
N6-(2,6-dimethylpyrimidin-4-yl)-N4-[2-methoxy-4-(trifluoromethyl)phenyl]pyrimidine-4,6-diamine
N6-(2,6-dimethylpyrimidin-4-yl)-N4-(2-methoxyphenyl)pyrimidine-4,6-diamine
N-[2-[[6-[(2,6-dimethylpyrimidin-4-yl)amino]pyrimidin-4-yl]amino]-5-(3-methylimidazol-4-yl)phenyl]-N-methyl-methanesulfonamide
N-[5-(1,5-dimethylpyrazol-4-yl)-2-[[6-[(2,6-dimethylpyrimidin-4-yl)amino]pyrimidin-4-yl]amino]phenyl]-N-methyl-methanesulfonamide
N4-(2,6-dimethylpyrimidin-4-yl)-N6-[4-(3-methylimidazol-4-yl)-2-methylsulfonyl-phenyl]pyrimidine-4,6-diamine
6-[[6-[(2,6-dimethylpyrimidin-4-yl)amino]pyrimidin-4-yl]amino]-5-methoxy-pyridine-3-carbonitrile
4-N-(2-dimethylphosphorylphenyl)-6-N-(2,6-dimethylpyrimidin-4-yl)pyrimidine-4,6-diamine
N6-(2,6-dimethylpyrimidin-4-yl)-N4-[2-methoxy-3-[1-methyl-5-(trifluoromethyl)-1,2,4-triazol-3-yl]phenyl]pyrimidine-4,6-diamine
N4-(2,6-dimethylpyrimidin-4-yl)-N6-[2-methoxy-3-[5-(methoxymethyl)-1,2,4-oxadiazol-3-yl]phenyl]pyrimidine-4,6-diamine
N4-(2,6-dimethylpyrimidin-4-yl)-N6-[3-(5-fluoropyrimidin-2-yl)-2-methoxy-phenyl]pyrimidine-4,6-diamine
N-[2-[[6-[(6-cyano-2-pyridyl)amino]pyrimidin-4-yl]amino]phenyl]-N-methyl-methanesulfonamide
N6-[2-methoxy-3-(1-methyl-1,2,4-triazol-3-yl)phenyl]-N4-(6-methylpyrimidin-4-yl)pyrimidine-4,6-diamine
N-[2-[[6-[(2,6-dimethylpyrimidin-4-yl)amino]pyrimidin-4-yl]amino]phenyl]-N-(trideuteriomethyl)methanesulfonamide
N-[3-[[6-[(2,6-dimethylpyrimidin-4-yl)amino]pyrimidin-4-yl]amino]pyridin-4-yl]-N-methylmethanesulfonamide
N6-(2,6-dimethylpyrimidin-4-yl)-N4-[2-methoxy-3-[2-methyl-5-(trifluoromethyl)pyrazol-3-yl]phenyl]pyrimidine-4,6-diamine
N4-(2,6-dimethylpyrimidin-4-yl)-N6-[2-methoxy-3-(5-methyl-1,2,4-oxadiazol-3-yl)phenyl]pyrimidine-4,6-diamine
N6-(2,6-dimethylpyrimidin-4-yl)-N4-[2-methoxy-3-[5-methyl-1-(2,2,2-trifluoroethyl)-1,2,4-triazol-3-yl]phenyl]pyrimidine-4,6-diamine
N6-(2,6-dimethylpyrimidin-4-yl)-N4-[3-(1,5-dimethyl-1,2,4-triazol-3-yl)-2-methoxyphenyl]pyrimidine-4,6-diamine
N6-(2,6-dimethylpyrimidin-4-yl)-N4-[3-(2,5-dimethyl-1,2,4-triazol-3-yl)-2-methoxyphenyl]pyrimidine-4,6-diamine
N4-(2,6-dimethylpyrimidin-4-yl)-N6-[2-methoxy-3-(2-methyltetrazol-5-yl)phenyl]pyrimidine-4,6-diamine
N6-(2,6-dimethylpyrimidin-4-yl)-N4-(2-methoxy-3-pyridazin-4-yl-phenyl)pyrimidine-4,6-diamine
N4-(2,6-dimethylpyrimidin-4-yl)-N6-[2-methoxy-3-[1-(oxetan-3-yl)-1,2,4-triazol-3-yl]phenyl]pyrimidine-4,6-diamine
N6-(2,6-dimethylpyrimidin-4-yl)-N4-[2-methoxy-3-[1-(2-methoxyethyl)-1,2,4-triazol-3-yl]phenyl]pyrimidine-4,6-diamine
N4-(2,6-dimethylpyrimidin-4-yl)-N6-[2-ethoxy-3-(1-methyl-1,2,4-triazol-3-yl)phenyl]pyrimidine-4,6-diamine
N4-(2,6-dimethylpyrimidin-4-yl)-N6-[3-(1-ethyl-1,2,4-triazol-3-yl)-2-methoxyphenyl]pyrimidine-4,6-diamine
N4-(2,6-dimethylpyrimidin-4-yl)-N6-[2-methoxy-3-[1-(tetrahydropyran-2-ylmethyl)-1,2,4-triazol-3-yl]phenyl]pyrimidine-4,6-diamine
N4-(2,6-dimethylpyrimidin-4-yl)-N6-[2-methoxy-3-[5-methyl-2-(oxetan-3-yl)-1,2,4-triazol-3-yl]phenyl]pyrimidine-4,6-diamine
N4-(2,6-dimethylpyrimidin-4-yl)-N6-[2-methoxy-3-[5-methyl-1-(oxetan-3-yl)-1,2,4-triazol-3-yl]phenyl]pyrimidine-4,6-diamine
N6-(3-bromo-2-methoxy-phenyl)-N4-(2,6-dimethylpyrimidin-4-yl)pyrimidine-4,6-diamine

N6-(2,6-dimethylpyrimidin-4-yl)-N4-[2-methoxy-3-[1-(oxetan-3-ylmethyl)-1,2,4-triazol-3-yl]phenyl]pyrimidine-4,6-diamine
N4-(2,6-dimethylpyrimidin-4-yl)-N6-[2-methoxy-3-[1-(tetrahydropyran-4-ylmethyl)-1,2,4-triazol-3-yl]phenyl]pyrimidine-4,6-diamine
N6-[3-[1-[(3,3-difluorocyclobutyl)methyl]-1,2,4-triazol-3-yl]-2-methoxy-phenyl]-N4-(2,6-dimethylpyrimidin-4-yl)pyrimidine-4,6-diamine
N4-[3-[1-(difluoromethyl)-1,2,4-triazol-3-yl]-2-methoxy-phenyl]-N6-(2,6-dimethylpyrimidin-4-yl)pyrimidine-4,6-diamine
N6-[3-[1-(2,2-difluorocyclopropyl)-1,2,4-triazol-3-yl]-2-methoxy-phenyl]-N4-(2,6-dimethylpyrimidin-4-yl)pyrimidine-4,6-diamine
N6-(2,6-dimethylpyrimidin-4-yl)-N4-(2-methoxy-3-tetrahydropyran-2-yl-phenyl)pyrimidine-4,6-diamine
N4-(2,6-dimethylpyrimidin-4-yl)-N6-[2-methoxy-3-[1-(tetrahydropyran-3-ylmethyl)-1,2,4-triazol-3-yl]phenyl]pyrimidine-4,6-diamine
3-[3-[3-[[6-[(2,6-dimethylpyrimidin-4-yl)amino]pyrimidin-4-yl]amino]-2-methoxy-phenyl]-1,2,4-triazol-1-yl]cyclobutanol
N4-(2,6-dimethylpyrimidin-4-yl)-N6-[2-methoxy-3-[1-(tetrahydrofuran-2-ylmethyl)-1,2,4-triazol-3-yl]phenyl]pyrimidine-4,6-diamine
N6-(2,6-dimethylpyrimidin-4-yl)-N4-[2-methoxy-3-[1-(tetrahydrofuran-3-ylmethyl)-1,2,4-triazol-3-yl]phenyl]pyrimidine-4,6-diamine
N-(2-((6-((2,6-dimethylpyrimidin-4-yl)amino)pyrimidin-4-yl)amino)-4-methylpyridin-3-yl)-N-methylmethanesulfonamide
N6-(2,6-dimethylpyrimidin-4-yl)-N4-[2-methoxy-3-[5-(morpholinomethyl)-1,2,4-oxadiazol-3-yl]phenyl]pyrimidine-4,6-diamine
6-N-(2,6-dimethylpyrimidin-4-yl)-4-N-[2-methoxy-4-methyl-3-(1-methyl-1,2,4-triazol-3-yl)phenyl]pyrimidine-4,6-diamine
N4-(2,6-dimethylpyrimidin-4-yl)-N6-[4-fluoro-2-methoxy-3-(1-methyl-1,2,4-triazol-3-yl)phenyl]pyrimidine-4,6-diamine
N4-(5-fluoro-2-pyridyl)-N6-[2-methoxy-3-(1-methyl-1,2,4-triazol-3-yl)phenyl]pyrimidine-4,6-diamine
N4-(2,6-dimethylpyrimidin-4-yl)-N6-[2-(methylsulfonylmethyl)phenyl]pyrimidine-4,6-diamine
N-[5-bromo-2-[[6-[(2,6-dimethylpyrimidin-4-yl)amino]pyrimidin-4-yl]amino]phenyl]-N-methyl-methanesulfonamide
N-[2-[[6-[(2,6-dimethylpyrimidin-4-yl)amino]pyrimidin-4-yl]amino]-5-methyl-phenyl]-N-methyl-methanesulfonamide
N-[5-cyclopropyl-2-[[6-[(2,6-dimethylpyrimidin-4-yl)amino]pyrimidin-4-yl]amino]phenyl]-N-methyl-methanesulfonamide
N-[2-[[6-[(2,6-dimethylpyrimidin-4-yl)amino]pyrimidin-4-yl]amino]phenyl]-1,1,1-trifluoro-N-methyl-methanesulfonamide
N4-(2,6-dimethylpyrimidin-4-yl)-N6-[2-(1-methylsulfonylethyl)phenyl]pyrimidine-4,6-diamine
N6-(2,6-dimethylpyrimidin-4-yl)-N4-[2-(1-piperidylsulfonyl)phenyl]pyrimidine-4,6-diamine
N-[2-[[6-[(2,6-dimethylpyrimidin-4-yl)amino]pyrimidin-4-yl]amino]-4-fluorophenyl]-N-methylmethanesulfonamide
N-[2-[[6-[(2,6-dimethylpyrimidin-4-yl)amino]pyrimidin-4-yl]amino]-6-fluorophenyl]-N-methylmethanesulfonamide
4-[[6-[(2,6-dimethylpyrimidin-4-yl)amino]pyrimidin-4-yl]amino]-N, N-dimethyl-3-[methyl(methylsulfonyl)amino]benzamide
[4-[[6-[(2,6-dimethylpyrimidin-4-yl)amino]pyrimidin-4-yl]amino]-3-methoxy-phenyl]-pyrrolidin-1-yl-methanone
N6-(2,6-dimethylpyrimidin-4-yl)-N4-[2-(1-methyl-1,2,4-triazol-3-yl)phenyl]pyrimidine-4,6-diamine
N6-[4-(1,5-dimethylpyrazol-4-yl)-2-methoxy-phenyl]-N4-(2,6-dimethylpyrimidin-4-yl)pyrimidine-4,6-diamine
N4-(2,6-dimethylpyrimidin-4-yl)-N6-[4-(5-fluoropyrimidin-2-yl)-2-methoxy-phenyl]pyrimidine-4,6-diamine
N6-(2,6-dimethylpyrimidin-4-yl)-N4-[2-methoxy-4-[1-methyl-5-(trifluoromethyl)-1,2,4-triazol-3-yl]phenyl]pyrimidine-4,6-diamine
N6-(2,6-dimethylpyrimidin-4-yl)-N4-[2-methoxy-3-methyl-4-(1-methyl-1,2,4-triazol-3-yl)phenyl]pyrimidine-4,6-diamine
N4-[4-(1,5-dimethylpyrazol-4-yl)-2-methoxy-3-methyl-phenyl]-N6-(2,6-dimethylpyrimidin-4-yl)pyrimidine-4,6-diamine
N6-(2,6-dimethylpyrimidin-4-yl)-N4-[2-methoxy-4-(1-methyl-1,2,4-triazol-3-yl)phenyl]pyrimidine-4,6-diamine
N4-[4-(1,5-dimethylpyrazol-4-yl)-2-methylsulfonyl-phenyl]-N6-(2,6-dimethylpyrimidin-4-yl)pyrimidine-4,6-diamine

N-[2-[[6-[(2,6-dimethylpyrimidin-4-yl)amino]pyrimidin-4-yl]amino]-5-[2-methyl-5-(trifluoromethyl)pyrazol-3-yl]-3-pyridyl]-N-methyl-methanesulfonamide
N-[2-[[6-[(2,6-dimethylpyrimidin-4-yl)amino]pyrimidin-4-yl]amino]-5-(1,3,5-trimethylpyrazol-4-yl)-3-pyridyl]-N-methyl-methanesulfonamide
N-[2-[[6-[(2,6-dimethylpyrimidin-4-yl)amino]pyrimidin-4-yl]amino]-5-(5-methyl-1H-pyrazol-4-yl)phenyl]-N-methyl-methanesulfonamide
N4-(2,6-dimethylpyrimidin-4-yl)-N6-[2-methoxy-4-(1-methylimidazol-4-yl)phenyl]pyrimidine-4,6-diamine
N4-(2,6-dimethylpyrimidin-4-yl)-N6-[2-methoxy-4-(1-methylimidazol-2-yl)phenyl]pyrimidine-4,6-diamine
N-[5-(3,4-dihydro-2H-pyran-6-yl)-2-[[6-[(2,6-dimethylpyrimidin-4-yl)amino]pyrimidin-4-yl]amino]phenyl]-N-methylmethanesulfonamide
N6-(2,6-dimethylpyrimidin-4-yl)-N4-[2-methoxy-4-(5-methyl-1,2,4-oxadiazol-3-yl)phenyl]pyrimidine-4,6-diamine
N-[5-(3,6-dihydro-2H-pyran-5-yl)-2-[[6-[(2,6-dimethylpyrimidin-4-yl)amino]pyrimidin-4-yl]amino]phenyl]-N-methyl-methanesulfonamide
N-[5-(3,6-dihydro-2H-pyran-4-yl)-2-[[6-[(2,6-dimethylpyrimidin-4-yl)amino]pyrimidin-4-yl]amino]phenyl]-N-methyl-methanesulfonamide
N4-[4-(1,5-dimethylpyrazol-4-yl)-2-fluoro-6-methylsulfonyl-phenyl]-N6-(2,6-dimethylpyrimidin-4-yl)pyrimidine-4,6-diamine
N-[2-[[6-[(2,6-dimethylpyrimidin-4-yl)amino]pyrimidin-4-yl]amino]-5-(1,5-dimethyl-1,2,4-triazol-3-yl)phenyl]-N-methyl-methanesulfonamide
N-[5-(1,5-dimethylpyrazol-4-yl)-2-[[6-[(2,6-dimethylpyrimidin-4-yl)amino]pyrimidin-4-yl]amino]phenyl]-N-(trideuteriomethyl)methanesulfonamide
N-[2-[[6-[(2,6-dimethylpyrimidin-4-yl)amino]pyrimidin-4-yl]amino]-5-(2,5-dimethyl-1,2,4-triazol-3-yl)phenyl]-N-methyl-methanesulfonamide
N-[2,4-dimethyl-6-[[6-[(6-methylpyrimidin-4-yl)amino]pyrimidin-4-yl]amino]phenyl]-N-methylmethanesulfonamide
N-[2-[[6-[(5-fluoro-4-methyl-2-pyridyl)amino]pyrimidin-4-yl]amino]phenyl]-N-methyl-methanesulfonamide
N-[2-[[6-[(5-cyano-2-pyridyl)amino]pyrimidin-4-yl]amino]phenyl]-N-methyl-methanesulfonamide
N-[5-(1,5-dimethylpyrazol-4-yl)-2-[[6-[(5-fluoro-4-methyl-2-pyridyl)amino]pyrimidin-4-yl]amino]-3-pyridyl]-N-methyl-methanesulfonamide
4-N-[2-methoxy-5-methyl-3-(1-methyl-1,2,4-triazol-3-yl)phenyl]-6-N-(6-methylpyrimidin-4-yl)pyrimidine-4,6-diamine
N-[2-[[6-[(2,6-dimethylpyrimidin-4-yl)amino]pyrimidin-4-yl]amino]-5-tetrahydropyran-3-yl-phenyl]-N-methyl-methanesulfonamide
N-[2-[[6-[(2,6-dimethylpyrimidin-4-yl)amino]pyrimidin-4-yl]amino]-5-tetrahydropyran-4-yl-phenyl]-N-methyl-methanesulfonamide
N-[2-[[6-[(2,6-dimethylpyrimidin-4-yl)amino]pyrimidin-4-yl]amino]-5-tetrahydropyran-2-yl-phenyl]-N-methyl-methanesulfonamide
N-[2-[[6-[(2,6-dimethylpyrimidin-4-yl)amino]pyrimidin-4-yl]amino]-5-vinyl-phenyl]-N-methyl-methanesulfonamide
N-[5-benzyl-2-[[6-[(2,6-dimethylpyrimidin-4-yl)amino]pyrimidin-4-yl]amino]phenyl]-N-methyl-methanesulfonamide
N-[2-[[6-[(2,6-dimethylpyrimidin-4-yl)amino]pyrimidin-4-yl]amino]-5-pyrrolidin-3-yl-phenyl]-N-methyl-methanesulfonamide
N-[2-[[6-[(2,6-dimethylpyrimidin-4-yl)amino]pyrimidin-4-yl]amino]-5-tetrahydrofuran-3-yl-phenyl]-N-methyl-methanesulfonamide
N6-(2,6-dimethylpyrimidin-4-yl)-N4-[2-(N,S-dimethylsulfonimidoyl)phenyl]pyrimidine-4,6-diamine
N4-[4-(1,5-dimethylpyrazol-4-yl)-2-(N,S-dimethylsulfonimidoyl)phenyl]-N6-(2,6-dimethylpyrimidin-4-yl)pyrimidine-4,6-diamine
N-[2-[[6-[(2,6-dimethylpyrimidin-4-yl)amino]pyrimidin-4-yl]amino]-5-tetrahydrofuran-2-yl-phenyl]-N-methyl-methanesulfonamide
N-[2-[[6-[(2,6-dimethylpyrimidin-4-yl)amino]pyrimidin-4-yl]amino]-5-(methoxymethyl)phenyl]-N-methyl-methanesulfonamide;
N6-(2,6-dimethylpyrimidin-4-yl)-N4-[2-(N,S-dimethylsulfonimidoyl)-3-methylphenyl]pyrimidine-4,6-diamine;
N4-(2,6-dimethylpyrimidin-4-yl)-N6-(2-methoxy-4-tetrahydrofuran-3-yl-phenyl)pyrimidine-4,6-diamine;
N4-[4-cyclopropyl-2-(N,S-dimethylsulfonimidoyl)phenyl]-N6-(2,6-dimethylpyrimidin-4-yl)pyrimidine-4,6-diamine;
N6-(2,6-dimethylpyrimidin-4-yl)-N4-[3-methoxy-4-(1-methyl-1,2,4-triazol-3-yl)-2-pyridyl]pyrimidine-4,6-diamine;
N-[2-[[6-[(2,6-dimethylpyrimidin-4-yl)amino]pyrimidin-4-yl]amino]-3-pyridyl]-N-methyl-methanesulfonamide;
N-[2-[[6-[(2,6-dimethylpyrimidin-4-yl)amino]pyrimidin-4-yl]amino]-3-pyridyl]-N-(trideuteriomethyl)methanesulfonamide;
N-[2-[[6-[(2,6-dimethylpyrimidin-4-yl)amino]pyrimidin-4-yl]amino]-5-[1-methyl-5-(trifluoromethyl)-1,2,4-triazol-3-yl]-3-pyridyl]-N-methyl-methanesulfonamide;
N-[2-[[6-[(2,6-dimethylpyrimidin-4-yl)amino]pyrimidin-4-yl]amino]-5-(1-methyl-1,2,4-triazol-3-yl)-3-pyridyl]-N-methyl-methanesulfonamide;
N-[5-(1,5-dimethylpyrazol-4-yl)-2-[[6-[(2,6-dimethylpyrimidin-4-yl)amino]pyrimidin-4-yl]amino]-3-pyridyl]-N-methyl-methanesulfonamide;
N4-(2,6-dimethylpyrimidin-4-yl)-N6-(3-methylsulfonyl-2-pyridyl)pyrimidine-4,6-diamine;
N-[2-[[6-[(2,6-dimethylpyrimidin-4-yl)amino]pyrimidin-4-yl]amino]-5-(1-methylpyrazol-4-yl)-3-pyridyl]-N-methyl-methanesulfonamide;
N-[2-[[6-[(2,6-dimethylpyrimidin-4-yl)amino]pyrimidin-4-yl]amino]-5-(2-methylpyrazol-3-yl)-3-pyridyl]-N-methyl-methanesulfonamide;
N-[2-[[6-[(2,6-dimethylpyrimidin-4-yl)amino]pyrimidin-4-yl]amino]-5-(1-methylimidazol-2-yl)-3-pyridyl]-N-methyl-methanesulfonamide;
N-[5-(2,5-dimethylpyrazol-3-yl)-2-[[6-[(2,6-dimethylpyrimidin-4-yl)amino]pyrimidin-4-yl]amino]-3-pyridyl]-N-methyl-methanesulfonamide;
N-[2-[[6-[(2,6-dimethylpyrimidin-4-yl)amino]pyrimidin-4-yl]amino]-5-(2-methyl-1,2,4-triazol-3-yl)-3-pyridyl]-N-methyl-methanesulfonamide;
N-[2-[[6-[(2,6-dimethylpyrimidin-4-yl)amino]pyrimidin-4-yl]amino]-5-(1-methylpyrazol-3-yl)-3-pyridyl]-N-methyl-methanesulfonamide;
N4-(2,6-dimethylpyrimidin-4-yl)-N6-(3-methoxy-4-methyl-2-pyridyl)pyrimidine-4,6-diamine;
N-[5-(1,5-dimethylpyrazol-4-yl)-2-[[6-[(2,6-dimethylpyrimidin-4-yl)amino]pyrimidin-4-yl]amino]-3-pyridyl]-N-(trideuteriomethyl)methanesulfonamide;
N-[2-[[6-[(2,6-dimethylpyrimidin-4-yl)amino]pyrimidin-4-yl]amino]-5-(4,5-dimethyl-1,2,4-triazol-3-yl)-3-pyridyl]-N-methyl-methanesulfonamide;
N-[5-(1,5-dimethylpyrazol-4-yl)-2-[[6-[(6-methylpyrimidin-4-yl)amino]pyrimidin-4-yl]amino]-3-pyridyl]-N-methyl-methanesulfonamide;
N-[2-[[6-[(2,6-dimethylpyrimidin-4-yl)amino]pyrimidin-4-yl]amino]-5-(4,5-dimethyl-1,2,4-triazol-3-yl)-3-pyridyl]-N-(trideuteriomethyl)methanesulfonamide;
N4-[5-(1,5-dimethylpyrazol-4-yl)-3-methylsulfonyl-2-pyridyl]-N6-(2,6-dimethylpyrimidin-4-yl)pyrimidine-4,6-diamine;
N-[5-(1,5-dimethylpyrazol-4-yl)-2-[[6-[(5-fluoro-2-pyridyl)amino]pyrimidin-4-yl]amino]-3-pyridyl]-N-methyl-methanesulfonamide;
N-[6-[[5-(1,5-dimethylpyrazol-4-yl)-3-[methyl(methylsulfonyl)amino]-2-pyridyl]amino]pyrimidin-4-yl]cyclopropanecarboxamide;
N-[5-(1,5-dimethylimidazol-2-yl)-2-[[6-[(2,6-dimethylpyrimidin-4-yl)amino]pyrimidin-4-yl]amino]-3-pyridyl]-N-methyl-methanesulfonamide;
N6-[5-(1,5-dimethylpyrazol-4-yl)-3-methoxy-4-methyl-2-pyridyl]-N4-(2,6-dimethylpyrimidin-4-yl)pyrimidine-4,6-diamine;
N-[5-(2,3-dimethylimidazol-4-yl)-2-[[6-[(2,6-dimethylpyrimidin-4-yl)amino]pyrimidin-4-yl]amino]-3-pyridyl]-N-methyl-methanesulfonamide;
N6-(2,6-dimethylpyrimidin-4-yl)-N4-[3-methoxy-4-[5-(trifluoromethyl)-1,2,4-oxadiazol-3-yl]-2-pyridyl]pyrimidine-4,6-diamine;
N-[5-(1,5-dimethylpyrazol-4-yl)-2-[[6-[(2,6-dimethylpyrimidin-4-yl)amino]pyrimidin-4-yl]amino]-3-pyridyl]methanesulfonamide;
N6-(3-dimethylphosphoryl-2-pyridyl)-N4-(2,6-dimethylpyrimidin-4-yl)pyrimidine-4,6-diamine;
N6-[5-(1,5-dimethylpyrazol-4-yl)-3-methoxy-2-pyridyl]-N4-(2,6-dimethylpyrimidin-4-yl)pyrimidine-4,6-diamine;
N-[2-[[6-[(2,6-dimethylpyrimidin-4-yl)amino]pyrimidin-4-yl]amino]-5-(1-methylimidazol-4-yl)-3-pyridyl]-N-methyl-methanesulfonamide;
N4-(2,6-dimethylpyrimidin-4-yl)-N6-[4-(1,5-dimethyl-1,2,4-triazol-3-yl)-3-methoxy-2-pyridyl]pyrimidine-4,6-diamine;
N6-[3-dimethylphosphoryl-5-(1,5-dimethylpyrazol-4-yl)-2-pyridyl]-N4-(2,6-dimethylpyrimidin-4-yl)pyrimidine-4,6-diamine;
N4-(2,6-dimethylpyrimidin-4-yl)-N6-(4-methyl-3-methylsulfonyl-2-pyridyl)pyrimidine-4,6-diamine;
N-[6-[[5-(1,5-dimethylpyrazol-4-yl)-3-methylsulfonyl-2-pyridyl]amino]pyrimidin-4-yl]cyclopropanecarboxamide;

N4-[5-(1,5-dimethylpyrazol-4-yl)-3-methylsulfonyl-2-pyridyl]-N6-(5-fluoro-2-pyridyl)pyrimidine-4,6-diamine;
N6-[5-(1,5-dimethylpyrazol-4-yl)-4-methyl-3-methylsulfonyl-2-pyridyl]-N4-(2,6-dimethylpyrimidin-4-yl)pyrimidine-4,6-diamine;
N-[5-(1,5-dimethyl-1,2,4-triazol-3-yl)-2-[[6-[(6-methylpyrimidin-4-yl)amino]pyrimidin-4-yl]amino]-3-pyridyl]-N-methyl-methanesulfonamide;
N-[5-(2,5-dimethyl-1,2,4-triazol-3-yl)-2-[[6-[(6-methylpyrimidin-4-yl)amino]pyrimidin-4-yl]amino]-3-pyridyl]-N-methyl-methanesulfonamide;
N6-(6-methyl-3-methylsulfonyl-2-pyridyl)-N4-(6-methylpyrimidin-4-yl)pyrimidine-4,6-diamine;
N-[2-[[6-[(2,6-dimethylpyrimidin-4-yl)amino]pyrimidin-4-yl]amino]-5-(oxan-4-yl)pyridin-3-yl]-N-methylmethanesulfonamide;
N-[5-(1,5-dimethylpyrazol-4-yl)-2-[[6-[(6-methylpyridazin-3-yl)amino]pyrimidin-4-yl]amino]-3-pyridyl]-N-methyl-methanesulfonamide;
4-N-(2,6-dimethylpyrimidin-4-yl)-6-N-[3-methoxy-4-(5-methyl-1,2,4-oxadiazol-3-yl)pyridin-2-yl]pyrimidine-4,6-diamine;
(1R,2R)-N-[6-[[5-(1,5-dimethylpyrazol-4-yl)-3-[methyl(methylsulfonyl)amino]-2-pyridyl]amino]pyrimidin-4-yl]-2-fluoro-cyclopropanecarboxamide;
N-[6-[[3-dimethylphosphoryl-5-(1,5-dimethylpyrazol-4-yl)-2-pyridyl]amino]pyrimidin-4-yl]cyclopropanecarboxamide;
N-methyl-N-[6-methyl-5-(1-methylpyrazol-4-yl)-2-[[6-[(6-methylpyrimidin-4-yl)amino]pyrimidin-4-yl]amino]-3-pyridyl]methanesulfonamide;
N-[5-(1,5-dimethylpyrazol-4-yl)-6-methyl-2-[[6-[(6-methylpyrimidin-4-yl)amino]pyrimidin-4-yl]amino]-3-pyridyl]-N-methyl-methanesulfonamide;
N4-(2,6-dimethylpyrimidin-4-yl)-N6-[3-(N,S-dimethylsulfonimidoyl)-2-pyridyl]pyrimidine-4,6-diamine;
N4-[3-methoxy-6-methyl-5-(1-methylpyrazol-4-yl)-2-pyridyl]-N6-(6-methylpyrimidin-4-yl)pyrimidine-4,6-diamine;
N4-(2,6-dimethylpyrimidin-4-yl)-N6-(3-methoxy-5-tetrahydropyran-2-yl-2-pyridyl)pyrimidine-4,6-diamine;
N-[2-[[6-[(2,6-dimethylpyrimidin-4-yl)amino]pyrimidin-4-yl]amino]-5-tetrahydropyran-2-yl-3-pyridyl]-N-methyl-methanesulfonamide;
N6-[5-(1,5-dimethylpyrazol-4-yl)-3-(N,S-dimethylsulfonimidoyl)-2-pyridyl]-N4-(2,6-dimethylpyrimidin-4-yl)pyrimidine-4,6-diamine;
N6-(2,6-dimethylpyrimidin-4-yl)-N4-[3-methoxy-4-[1-(2-methoxyethyl)-1,2,4-triazol-3-yl]-2-pyridyl]pyrimidine-4,6-diamine;
N-[2-[[6-[(2,6-dimethylpyrimidin-4-yl)amino]pyrimidin-4-yl]amino]-5-(4-methyl-1,3-oxazol-5-yl)pyridin-3-yl]-N-methylmethanesulfonamide;
4-N-(5-cyclopropyl-3-dimethylphosphorylpyridin-2-yl)-6-N-(2,6-dimethylpyrimidin-4-yl)pyrimidine-4,6-diamine;
2-[2-[[6-[(2,6-dimethylpyrimidin-4-yl)amino]pyrimidin-4-yl]amino]pyridin-3-yl]propan-2-ol;
N4-[5-(1,5-dimethylpyrazol-4-yl)-3-methylsulfinyl-2-pyridyl]-N6-(2,6-dimethylpyrimidin-4-yl)pyrimidine-4,6-diamine;
N4-(5-fluoro-6-methyl-2-pyridyl)-N6-[3-methoxy-4-(1-methyl-1,2,4-triazol-3-yl)-2-pyridyl]pyrimidine-4,6-diamine;
6-N-(2,6-dimethylpyrimidin-4-yl)-4-N-[3-methylsulfonyl-5-(oxan-2-yl)pyridin-2-yl]pyrimidine-4,6-diamine;
N6-[5-(1,5-dimethylpyrazol-4-yl)-3-methoxy-6-methyl-2-pyridyl]-N4-(6-methylpyrimidin-4-yl)pyrimidine-4,6-diamine;
or a pharmaceutically acceptable salt, or solvate, or N-oxide, or tautomer, or stereoisomer, or isotopically labelled derivative thereof.

**18.** A pyrimidine substituted compound as defined in any one of claims 1 to 17, for use in the treatment of the human or animal body by therapy.

**19.** A pyrimidine substituted compound as defined in any one of claims 1 to 17, for use in the treatment of a pathological condition or disease susceptible to amelioration by inhibition of Tyk2.

**20.** A pyrimidine substituted compound according to any one of claims 1 to 17, for use according to claim 19, wherein the treatment is of a pathological condition or disease selected plaque psoriasis, scalp psoriasis, genital psoriasis, pustular psoriasis, psoriatic erythroderma, acrodermatitis continua, pityriasis rubra pilaris, lichen planus, hidradenitis suppurativa, bullous pemphigoid, pyoderma gangrenousm (PASH), ichthyosis, atopic dermatitis, psoriatic arthritis, ankylosing spondylitis, systemic sclerosis, primary biliary cholangitis, asthma and human multiple myeloma.

**21.** A pharmaceutical composition comprising a pyrimidine substituted compound as defined in any one of claims 1 to 17 in association with a pharmaceutically acceptable diluent or carrier.

**22.** Use of a pyrimidine substituted compound derivative as defined in any one of claims 1 to 17, for the manufacture of a medicament for the treatment of a pathological condition or disease as defined in claim 19 and 20.

**23.** A method for treating a subject afflicted with a pathological condition or disease as defined in claims 19 and 20, which comprises administering to said subject a therapeutically effective amount of a pyrimidine substituted compound as defined in any one of claims 1 to 17, or a pharmaceutical composition as defined in claim 21.

**24.** A combination product comprising (i) at least one pyrimidine substituted as defined in any one of claims 1 to 17, and (ii) one or more active ingredients selected from:

a) Corticosteroids, such as prednisone, methylprednisolone or beta-methasone;
b) Immunosuppressants, such as cyclosporine, tacrolimus methotrexate, hydroxyurea, mycophenolate mofetil, mycophenolic acid, sulfasalazine, 6-thioguanine or azathioprine;
c) Fumaric acid esters, such as dimethyl fumarate or tepilamide fumarate;
d) Dihydroorotate dehydrogenase (DHODH) inhibitors such as leflunomide;
e) Retinoids, such as acitretin or isotretinoin;
f) Anti-inflammatories such as apremilast, crisaborole, celecoxib, diclofenac, aceclofenac, aspirin or naproxen;
g) Antibiotics such as gentamicin;
h) Antivirals such as aciclovir, efavirenz or ribavirin
i) Anti-cancer agents such as lenalidomide, pomalidomide, pembrolizumab, nivolumab, daratumumab, bortezomib, carfilzomib, ixazomib, bendamustine or ventoclast;
j) T-cell blockers such as alefacept or efalizumab;
k) Tumor necrosis factor-alpha (TNF-alpha) blockers such as etanercept, adalimumab, infliximab, golimumab, certolizumab pegol;
l) anti-IL4/IL13 antagonist such as dupilumab, lebrikizumab or tralokinumab;
o) IL-1b blockers such as canakinumab;
p) IL-alpha blockers such as bermekimab;
q) CD6 blockers such as itolizumab;
r) IL-36R blockers such as BI-655130 orANB019;
s) IL-6 antagonist such as tocilizumab;
t) Calcineurin inhibitors such as pimecrolimus, tacrolimus or cyclosporine;
u) Phototherapy agents commonly employed in phototherapy such as psoralen, methoxypsoralen or 5-methoxypsoralen + UVA (PUVA) or treatment with UVB (with or without tar);
v) Fixed combinations of corticosteroids and vitamin D derivatives;
w) Fixed combinations of corticosteroids and retinoids;
x) Corticosteroid tapes;
y) Other combinable agents may include piclidenoson, LYC-30937, LEO-32731, BI-730357, PRCL-02, LNP-1955, GSK-2982772, CBP-307, KD-025, MP-1032, petesicatib, JTE-451, Hemay-005, SM-04755, EDP-1815, BI-730460, GLPG3970, SFA-002 ER, JNJ-3534, SAR-441169, BOS-172767, SCD-044, CUDC-305. ABBV-157, BAY-1834845, AUR-101, R-835, PBF-1650, IMU-935, RTA-1701, AZD-0284, CD20 antagonist, salicylic acid, coal tar, Mical-1, DUR-928, AM-001, BMX-010, TA-102, SNA-125, KP-470, pegcantratinib, pefcalcitol, ESR-114, tapinarof, NP-000888, SM-04755, BOS-475, SB-414, LEO-134310, CBS-3595, PF-06763809, XCUR-17 or BTX-1308, MGC topical cream, RIST4721, aminopterin Na.

Europäisches Patentamt
European Patent Office
Office européen des brevets

# EUROPEAN SEARCH REPORT

Application Number
EP 21 38 3231

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 111 777 592 A (UNIV WENZHOU MEDICAL) 16 October 2020 (2020-10-16) * claims 1, 5; compound X20 * | 1-5,9, 18,21 | INV. C07D403/14 C07D239/48 C07D401/12 C07D401/14 A61P17/06 C07D403/12 C07D405/14 C07D413/14 C07F9/53 A61K31/506 |
| X | WO 2011/088027 A1 (GLAXOSMITHKLINE LLC [US]; KALLANDER LARA S [US] ET AL.) 21 July 2011 (2011-07-21) * claims 1, 13; example 270 * | 1,5,9, 18,21 | |
| A | WO 2013/174895 A1 (CELLZOME LTD [GB]; HARRISON RICHARD JOHN [GB] ET AL.) 28 November 2013 (2013-11-28) * claims 1, 17 * | 1-24 | |
| A | WO 2012/062704 A1 (CELLZOME LTD [GB]; ELLARD KATIE [GB] ET AL.) 18 May 2012 (2012-05-18) * claims 1, 22 * | 1-24 | |

TECHNICAL FIELDS SEARCHED (IPC)

C07D
C07F
A61P
A61K

The present search report has been drawn up for all claims

1

| Place of search | Date of completion of the search | Examiner |
|---|---|---|
| Munich | 18 May 2022 | Johnson, Claire |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 21 38 3231

This annex lists the patent family members relating to the patent documents cited in the above-mentioned European search report. The members are as contained in the European Patent Office EDP file on
The European Patent Office is in no way liable for these particulars which are merely given for the purpose of information.

18-05-2022

| Patent document cited in search report | Publication date | Patent family member(s) | Publication date |
|---|---|---|---|
| CN 111777592 A | 16-10-2020 | NONE | |
| WO 2011088027 A1 | 21-07-2011 | AU 2011205485 A1 | 02-08-2012 |
| | | CA 2786999 A1 | 21-07-2011 |
| | | CN 102791131 A | 21-11-2012 |
| | | EA 201290642 A1 | 30-05-2013 |
| | | EP 2523559 A1 | 21-11-2012 |
| | | JP 2013517273 A | 16-05-2013 |
| | | KR 20120114355 A | 16-10-2012 |
| | | SG 182351 A1 | 30-08-2012 |
| | | US 2012329784 A1 | 27-12-2012 |
| | | WO 2011088027 A1 | 21-07-2011 |
| WO 2013174895 A1 | 28-11-2013 | AU 2013265307 A1 | 13-11-2014 |
| | | BR 112014029310 A2 | 26-06-2018 |
| | | CA 2875990 A1 | 28-11-2013 |
| | | CN 104507929 A | 08-04-2015 |
| | | EP 2855451 A1 | 08-04-2015 |
| | | ES 2651150 T3 | 24-01-2018 |
| | | JP 6197031 B2 | 13-09-2017 |
| | | JP 2015517553 A | 22-06-2015 |
| | | KR 20150013554 A | 05-02-2015 |
| | | RU 2014152257 A | 20-07-2016 |
| | | US 2015166513 A1 | 18-06-2015 |
| | | US 2016184307 A1 | 30-06-2016 |
| | | WO 2013174895 A1 | 28-11-2013 |
| WO 2012062704 A1 | 18-05-2012 | AU 2011328237 A1 | 23-05-2013 |
| | | CA 2815330 A1 | 18-05-2012 |
| | | CN 103298794 A | 11-09-2013 |
| | | EP 2638018 A1 | 18-09-2013 |
| | | JP 2014500254 A | 09-01-2014 |
| | | WO 2012062704 A1 | 18-05-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018075937 A
- WO 2013037755 A
- WO 2014074661 A
- WO 2019183186 A


### Non-patent literature cited in the description


- **ERNEST L. ELIEL.** Stereochemistry of Organic Compounds. Wiley, 1994
- **T. HIGUCHI ; W. STELLA.** Novel Delivery Systems. *ACS Symposium Series,* vol. 14
- Bioreversible Carriers in Drug Design. Pergamon Press, 1987
- **H. BUNDGAARD.** Design of Prodrugs. Elsevier, 1985
- **T. W. GREENE ; G. M. WUTS.** Protecting Groups in Organic Synthesis. Wiley, 1999
- *CHEMICAL ABSTRACTS,* 870987-63-6
- Remington: The Science and Practice of Pharmacy. Lippincott Williams & Wilkins, 2001